(19) **Europäisches Patentamt European Patent Office Office européen des brevets**

(11) **EP 4 542 203 A1**

(12) **EUROPEAN PATENT APPLICATION**
published in accordance with Art. 153(4) EPC

(43) Date of publication:
**23.04.2025 Bulletin 2025/17**

(21) Application number: **23845887.1**

(22) Date of filing: **27.02.2023**

(51) International Patent Classification (IPC):
*G01N 21/41* [(2006.01)]    *C12M 1/00* [(2006.01)]
*C12M 3/00* [(2006.01)]    *C12Q 1/02* [(2006.01)]
*G01N 21/17* [(2006.01)]    *G01N 33/48* [(2006.01)]
*G01N 33/483* [(2006.01)]

(52) Cooperative Patent Classification (CPC):
**C12M 1/00; C12M 3/00; C12Q 1/02; G01N 21/17;
G01N 21/41; G01N 33/48; G01N 33/483**

(86) International application number:
**PCT/JP2023/007107**

(87) International publication number:
**WO 2024/024147 (01.02.2024 Gazette 2024/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.07.2022 JP 2022119270**

(71) Applicant: **Hamamatsu Photonics K.K.
Hamamatsu-shi, Shizuoka 435-8558 (JP)**

(72) Inventors:
• **TAKEUCHI Kozo
  Hamamatsu-shi, Shizuoka 435-8558 (JP)**
• **YASUHIKO Osamu
  Hamamatsu-shi, Shizuoka 435-8558 (JP)**

(74) Representative: **Hoffmann Eitle
Patent- und Rechtsanwälte PartmbB
Arabellastraße 30
81925 München (DE)**

(54) **METHOD, DEVICE, AND PROGRAM FOR IDENTIFYING OR EVALUATING BILE CANALICULUS REGIONS**

(57)    Disclosed are: a method for identifying, by using refractive index distribution data of an observation object containing liver cells, bile canaliculus regions included in the observation object; and a method for evaluating the bile canaliculus regions on the basis of bile canaliculus parameters obtained from the refractive index distribution data of the observation object containing the liver cells.

**Fig.44**

Z=6.4μm

BILE CANALICULUS 1
Z=6.4μm    Z=7.2μm    Z=8.8μm

BILE CANALICULUS 2
Z=3.2μm    Z=4.0μm    Z=6.4μm

EP 4 542 203 A1

## Description

### Technical Field

The present disclosure relates to a method, a device, and a program for identifying or evaluating bile canaliculus regions.

### Background Art

A bile canaliculus (also notated as bile canaliculi or BC) is a canaliculus formed between the adjacent hepatocytes. The lumen of the bile canaliculus is sealed by a tight junction between the hepatocytes, and a substance is transferred via a transporter on the cell membrane of the hepatocyte. In the bile canaliculus, a bile acid synthesized in the hepatocyte is secreted, and a drug or the like metabolized in the hepatocyte is excreted. On the surface of the hepatocyte facing the bile canaliculus, there are a plurality of fine projections referred to as a microvillus (also notated as microvilli or MV) with a length of approximately submicrometers to several micrometers.

A drug-induced liver injury (DILI) is an all-inclusive term for liver disease caused by a drug administered to a patient and/or a metabolite thereof, and is one of main reasons for the withdrawal of a pre-approved drug from the market and a restriction on use thereof, and the disapproval of a new drug due to a safety issue (Non Patent Literature 1).

Regarding the bile canaliculus and the drug, in Patent Literature 1 according to a hepatocyte cultivation method, the area and the number of bile canaliculi are used as an index for evaluating the cultivated hepatocyte. In Patent Literature 2, a device for evaluating a candidate compound susceptible to excretion into bile or blood by a hepatocyte and a liver metabolite of the candidate compound is disclosed in which the accumulation and the excretion of the liver metabolite into a bile canalicular structure are accelerated. In Patent Literature 3, an in-vitro method for screening a candidate compound causing a functional disorder of a bile canaliculus is disclosed in which a morphological change in the space of a bile duct, and the maximum amount of small molecules or large molecules that can be accumulated in a canaliculus and a canalicular lumen are used as an evaluation index.

### Citation List

### Patent Literature

Patent Literature 1: WO 2011/024592
Patent Literature 2: Japanese Unexamined Patent Publication No. 2020-028305
Patent Literature 3: Japanese Patent No. 6644786

### Non Patent Literature

Non Patent Literature 1: Watkins, "Drug safety sciences and the bottleneck in drug development.", Clin. Pharmacol. Ther., 89, 788-790 (2011).
Non Patent Literature 2: Gissen et al., "Structural and functional hepatocyte polarity and liver disease", Journal of Hepatology, 63, 1023-1037 (2015).

### Summary of Invention

### Technical Problem

The bile canaliculus is useful as an evaluation index for a cluster of hepatocytes and a liver tissue, and an analysis target for drug metabolism. However, it has been reported that it is difficult to identify the bile canaliculus region by hematoxylin and eosin stain (HE stain) that is a representative tissue staining method (Non Patent Literature 2). In addition, a method for identifying a bile canaliculus region and a method for evaluating a bile canaliculus region, which are currently used, are electron microscopy and fluorescence microscopy, but the electron microscopy is not suitable to be used in combination with drug evaluation since it is difficult to perform living cell observation and chronological observation, and the fluorescence microscopy requires fluorescent labeling.

Therefore, an object of one aspect of the present disclosure is to provide a method, a device, and a program for non-invasively identifying or evaluating a bile canaliculus region.

**Solution to Problem**

The present inventors have found that when using refractive index distribution data of an observation object, it is possible to identify a bile canaliculus region included in the observation object, and evaluate the identified bile canaliculus region.

That is, a first aspect of the present disclosure is a method for identifying a bile canaliculus region included in an observation object by using refractive index distribution data of the observation object containing a hepatocyte.

A second aspect of the present disclosure is a method for evaluating a bile canaliculus region, on the basis of a parameter of a bile canaliculus obtained from refractive index distribution data of an observation object containing a hepatocyte.

A third aspect of the present disclosure is a method for evaluating an observation object containing a hepatocyte, on the basis of the number of bile canaliculus regions identified by the method according to the first aspect of the present disclosure (the method for identifying the bile canaliculus region included in the observation object by using the refractive index distribution data of the observation object containing the hepatocyte) and/or the evaluation of the bile canaliculus region obtained by the method according to the second aspect of the present disclosure (the method for evaluating the bile canaliculus region, on the basis of the parameter of the bile canaliculus obtained from the refractive index distribution data of the observation object containing the hepatocyte).

A fourth aspect of the present disclosure is a method for evaluating a hepatocyte cultivation method, comprising: a step of cultivating a hepatocyte; and a step of evaluating an observation object obtained by the cultivation, using the method according to the third aspect of the present disclosure (the method for evaluating the observation object containing the hepatocyte, on the basis of the number of bile canaliculus regions identified by the method according to the first aspect of the present disclosure (the method for identifying the bile canaliculus region included in the observation object by using the refractive index distribution data) and/or the evaluation of the bile canaliculus region obtained by the method according to the second aspect of the present disclosure (the method for evaluating the bile canaliculus region by the parameter of the bile canaliculus obtained from the refractive index distribution data)).

A fifth aspect of the present disclosure is a drug screening method, comprising: a step of adding a drug to an observation object containing a hepatocyte; and a step of evaluating the observation object to which the drug is added, using the method according to the third aspect of the present disclosure (the method for evaluating the observation object containing the hepatocyte, on the basis of the number of bile canaliculus regions identified by the method according to the first aspect of the present disclosure (the method for identifying the bile canaliculus region included in the observation object by using the refractive index distribution data) and/or the evaluation of the bile canaliculus region obtained by the method according to the second aspect of the present disclosure (the method for evaluating the bile canaliculus region by the parameter of the bile canaliculus obtained from the refractive index distribution data)).

Another aspect is a device for identifying a bile canaliculus region, comprising: a data acquisition unit acquiring refractive index distribution data of an observation object containing a hepatocyte; and an identification unit identifying the bile canaliculus region included in the observation object by using the refractive index distribution data of the observation object.

Another aspect is a device for evaluating a bile canaliculus region, comprising: a data acquisition unit acquiring refractive index distribution data of an observation object containing a hepatocyte; an identification unit identifying the bile canaliculus region included in the observation object by using the refractive index distribution data of the observation object; and an evaluation unit evaluating the bile canaliculus region, on the basis of a parameter of a bile canaliculus obtained from the refractive index distribution data.

Another aspect is a program for causing a computer to execute: a data acquisition step of acquiring refractive index distribution data of an observation object containing a hepatocyte; and an identification step of identifying a bile canaliculus region included in the observation object by using the refractive index distribution data of the observation object.

Another aspect is a program for causing a computer to execute: a data acquisition step of acquiring refractive index distribution data of an observation object containing a hepatocyte; an identification step of identifying a bile canaliculus region included in the observation object by using the refractive index distribution data of the observation object; and an evaluation step of evaluating the bile canaliculus region, on the basis of a parameter of a bile canaliculus obtained from the refractive index distribution data.

In more detail, the present disclosure relates to [1] to [20] described below.

[1] A method for identifying a bile canaliculus region included in an observation object by using refractive index distribution data of the observation object containing a hepatocyte.

[2] The method according to [1], in which the bile canaliculus region is identified on the basis that the region in the observation object has a feature of a bile canaliculus.

[3] The method according to [2], in which the feature of the bile canaliculus includes one or more features selected from the group consisting of a feature that the region is an approximately circular or approximately cylindrical region existing between hepatocytes, a feature that a refractive index of the region is lower than a refractive index of hepatocyte, and a feature that a refractive index of a circumferential edge portion of the region is higher than the refractive index of the hepatocyte.

[4] The method according to [2], in which the feature of the bile canaliculus includes a feature that the region is an approximately circular or approximately cylindrical region existing between the hepatocytes, a feature that a refractive index of the region is lower than a refractive index of the hepatocyte, and a feature that a refractive index of a circumferential edge portion of the region is higher than the refractive index of the hepatocyte.

[5] The method according to [3] or [4], in which the feature of the bile canaliculus further includes a feature of comprising a region that is derived from a microvillus and has a refractive index higher than a refractive index of the entire region.

[6] The according to any one of [1] to [5], in which the refractive index distribution data is refractive index tomography data in a predetermined direction.

[7] A method for evaluating a bile canaliculus region, on the basis of a parameter of a bile canaliculus obtained from refractive index distribution data of an observation object containing a hepatocyte.

[8] The method according to [7], in which the parameter of the bile canaliculus comprises one or more parameters selected from the group consisting of an area of a cross section perpendicular to an axis of a tubular flow or a cross section seen from a tomographic plane, a refractive index, the number of microvilli, and an average length of microvilli.

[9] The method according to [7] or [8], in which the method comprises a step of identifying the bile canaliculus region included in the observation object by the method according to any one of [1] to [6], and the bile canaliculus region is evaluated on the basis of the parameter of the bile canaliculus in the identified bile canaliculus region.

[10] A method for evaluating an observation object containing a hepatocyte, on the basis of the number of bile canaliculus regions identified by the method according to any one of [1] to [6] and/or the evaluation of the bile canaliculus region obtained by the method according to any one of [7] to [9].

[11] A method for evaluating a hepatocyte cultivation method, comprising: a step of cultivating a hepatocyte; and a step of evaluating an observation object obtained by the cultivation, using the method according to [10].

[12] A drug screening method, comprising: a step of adding a drug to an observation object containing a hepatocyte; and a step of evaluating the observation object to which the drug is added, using the method according to [10].

[13] The method according to any one of [1] to [12], in which the observation object containing the hepatocyte is selected from the group consisting of a two-dimensional liver culture, a cluster of hepatocytes, and a liver tissue sample.

[14] The method according to any one of [1] to [12], in which the observation object containing the hepatocyte is selected from the group consisting of a cluster of hepatocytes and a liver tissue sample.

[15] The method according to any one of [1] to [12], in which the observation object containing the hepatocyte is a cluster of hepatocytes.

[16] A method for diagnosing liver disease, a method for assisting diagnosis of liver disease, or a method for collecting data for diagnosing liver disease, comprising: a step of acquiring refractive index distribution data of a clinical specimen of a liver; and a step of evaluating a bile canaliculus region included in the clinical specimen or the clinical specimen by using the method according to any one of [7] to [10].

[17] A device for identifying a bile canaliculus region, comprising: a data acquisition unit acquiring refractive index distribution data of an observation object containing a hepatocyte; and an identification unit identifying the bile canaliculus region included in the observation object by the method according to any one of [1] to [6].

[18] A device for evaluating a bile canaliculus region, comprising: a data acquisition unit acquiring refractive index distribution data of an observation object containing a hepatocyte; an identification unit identifying the bile canaliculus region included in the observation object by the method according to any one of [1] to [6]; and an evaluation unit evaluating the bile canaliculus region by the method according to any one of [7] to [9].

[19] A program for causing a computer to execute: a data acquisition step of acquiring refractive index distribution data of an observation object containing a hepatocyte; and an identification step of identifying a bile canaliculus region included in the observation object by the method according to any one of [1] to [6].

[20] A program for causing a computer to execute: a data acquisition step of acquiring refractive index distribution data of an observation object containing a hepatocyte; an identification step of identifying a bile canaliculus region included in the observation object by the method according to any one of [1] to [6]; and an evaluation step of evaluating the bile canaliculus region by the method according to any one of [7] to [9].

**Advantageous Effects of Invention**

According to the method, the device, or the program of the present disclosure, it is possible to non-invasively identify and evaluate the bile canaliculus region in the observation object by using the refractive index distribution data. In addition, according to the method of the present disclosure, it is possible to evaluate a method for cultivating a cluster of hepatocytes or performing a screening of a drug by using the refractive index distribution data.

**Brief Description of Drawings**

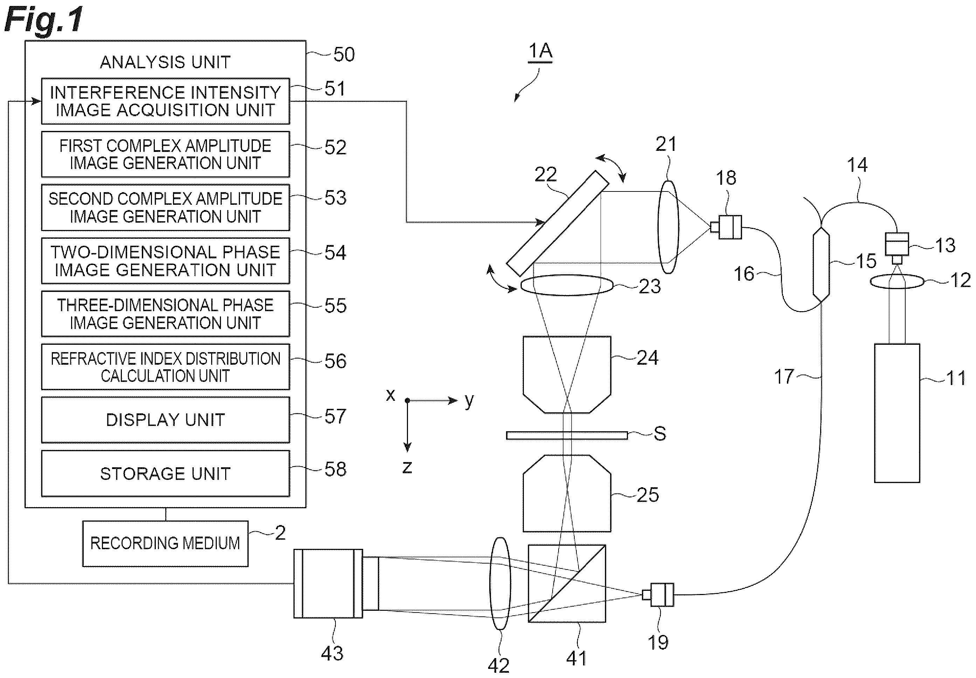

[FIG. 1] FIG. 1 (hereinafter also referred to as "FIG. A01") is a diagram illustrating a configuration of an observation apparatus 1A.

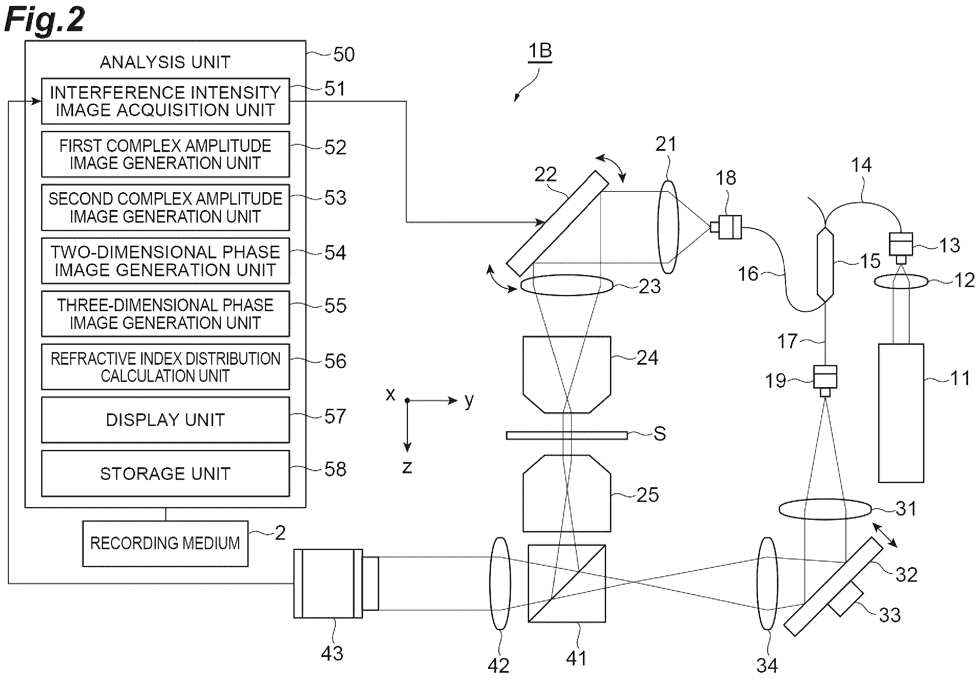

[FIG. 2] FIG. 2 (hereinafter also referred to as "FIG. A02") is a diagram illustrating a configuration of an observation apparatus 1B.

[FIG. 3] FIG. 3 (hereinafter also referred to as "FIG. A03") is a diagram illustrating a configuration of an observation apparatus 1C.

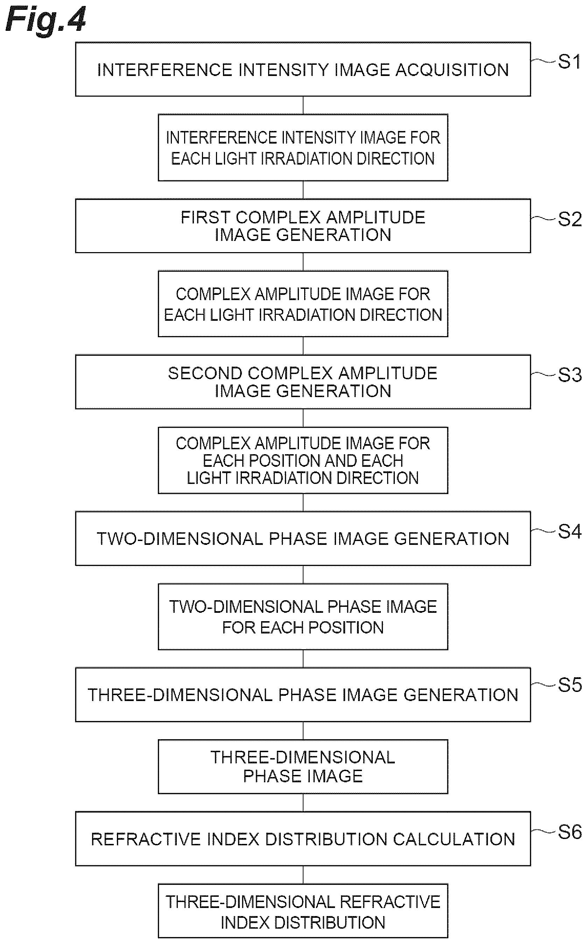

[FIG. 4] FIG. 4 (hereinafter also referred to as "FIG. A04") is a flowchart of a refractive index distribution measuring method A.

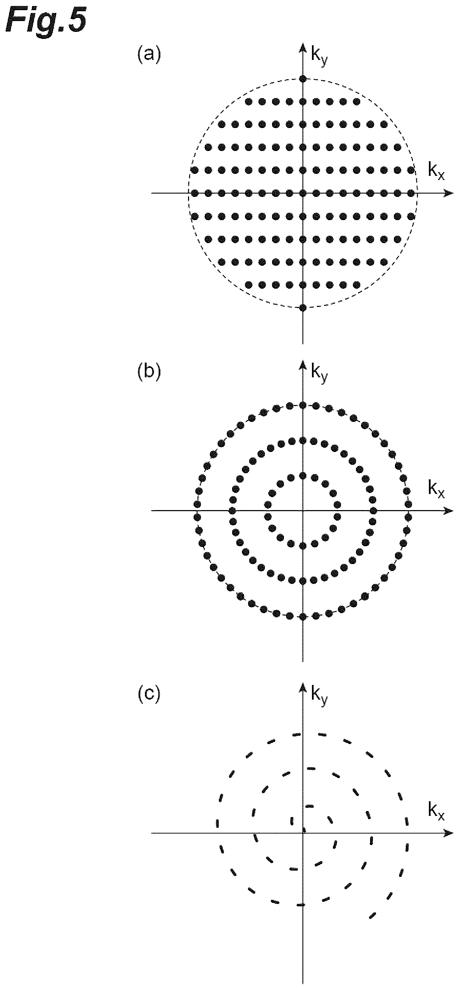

[FIG. 5] FIG. 5 (hereinafter also referred to as "FIG. A05") includes (a) - (c) diagrams illustrating examples of scanning of a light irradiation direction on an observation object S in an interference intensity image acquisition step S1.

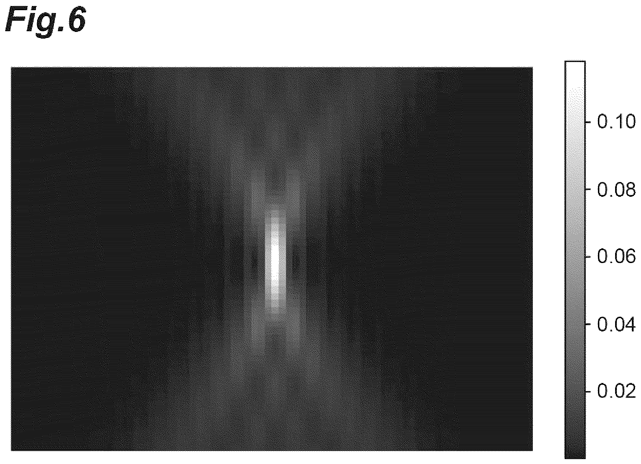

[FIG. 6] FIG. 6 (hereinafter also referred to as "FIG. A06") is a diagram showing a kernel function g.

[FIG. 7] FIG. 7 (hereinafter also referred to as "FIG. A07") includes (a) - (b) diagrams illustrating examples of scanning of a light irradiation direction on an observation object S in an interference intensity image acquisition step S1.

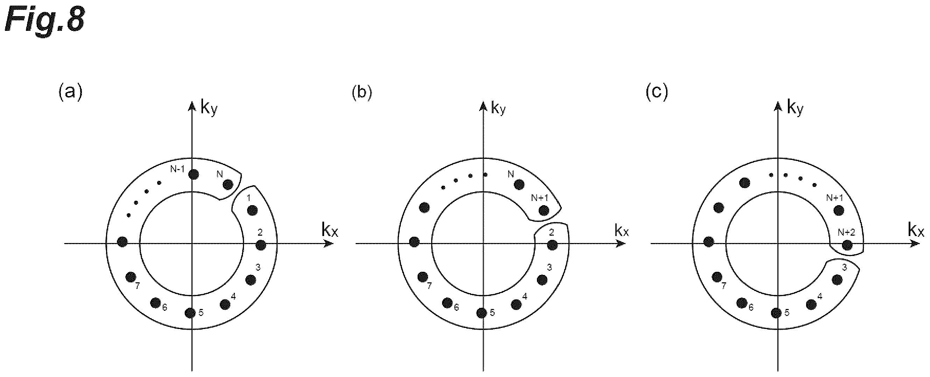

[FIG. 8] FIG. 8 (hereinafter also referred to as "FIG. A08") includes (a) - (c) diagrams illustrating examples of scanning of a light irradiation direction on an observation object S in an interference intensity image acquisition step S1.

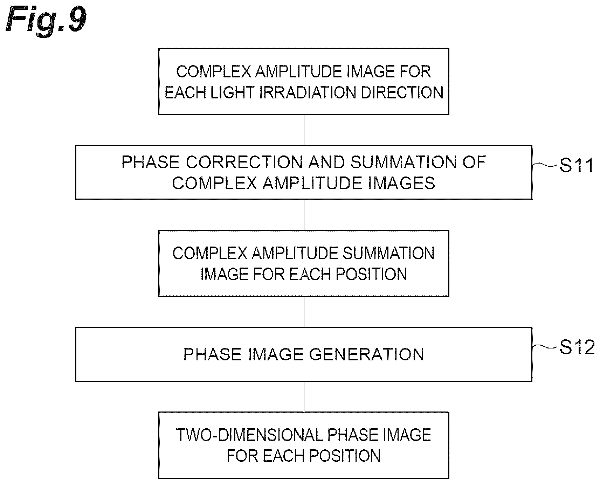

[FIG. 9] FIG. 9 (hereinafter also referred to as "FIG. A09") is a flowchart of a two-dimensional phase image generation step S4 in a refractive index distribution measuring method A1.

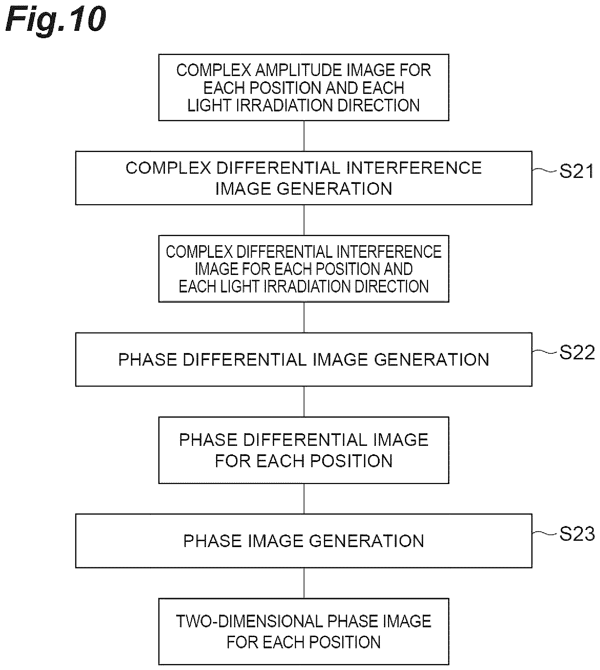

[FIG. 10] FIG. 10 (hereinafter also referred to as "FIG. A10") is a flowchart of a two-dimensional phase image generation step S4 in a refractive index distribution measuring method A2.

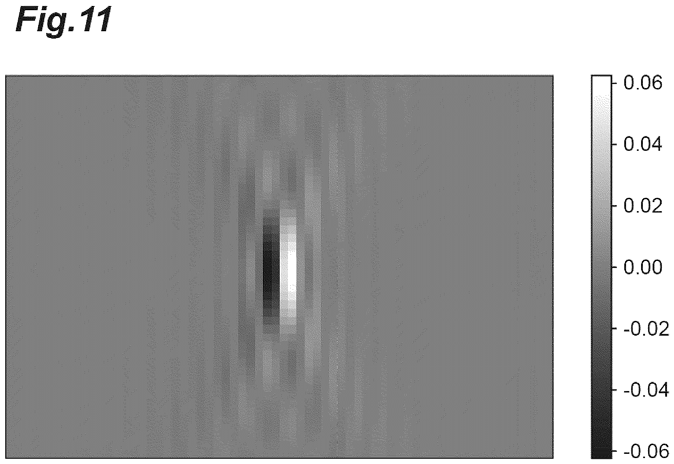

[FIG. 11] FIG. 11 (hereinafter also referred to as "FIG. A11") is a diagram showing the kernel function.

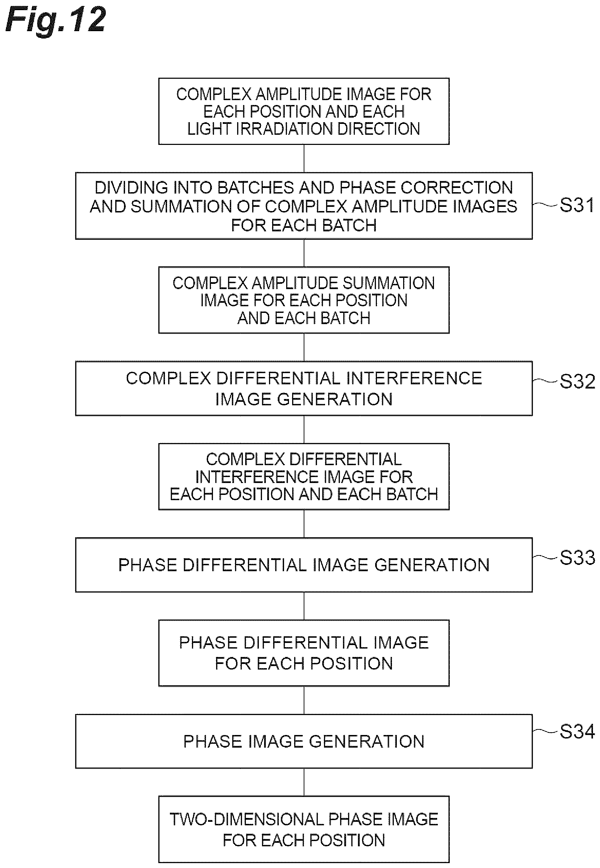

[FIG. 12] FIG. 12 (hereinafter also referred to as "FIG. A12") is a flowchart of a two-dimensional phase image generation step S4 in a refractive index distribution measuring method A3.

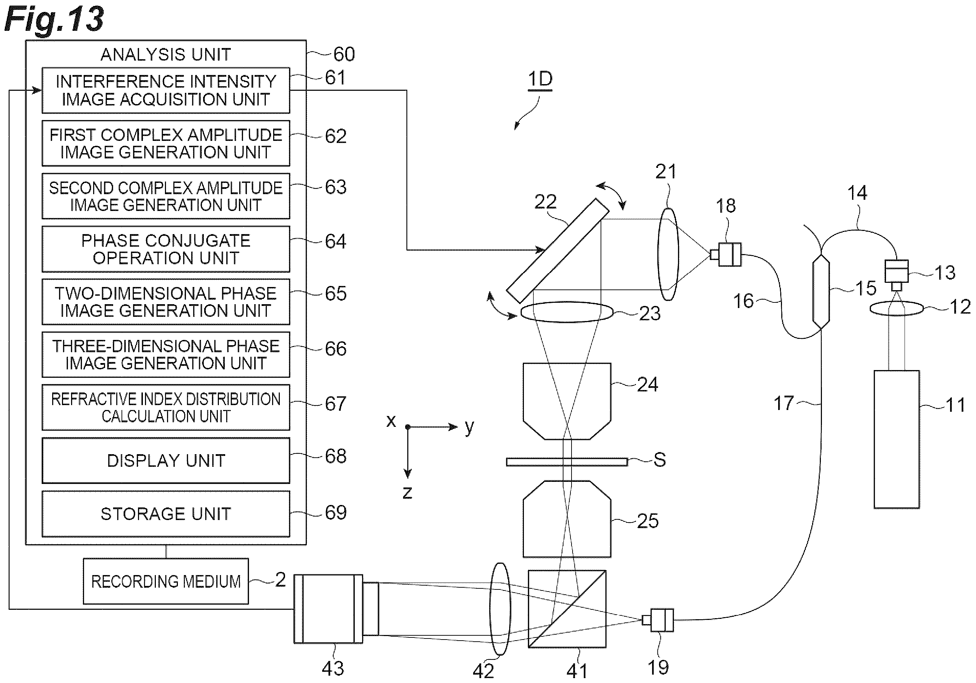

[FIG. 13] FIG. 13 (hereinafter also referred to as "FIG. B01") is a diagram illustrating a configuration of an observation apparatus 1D.

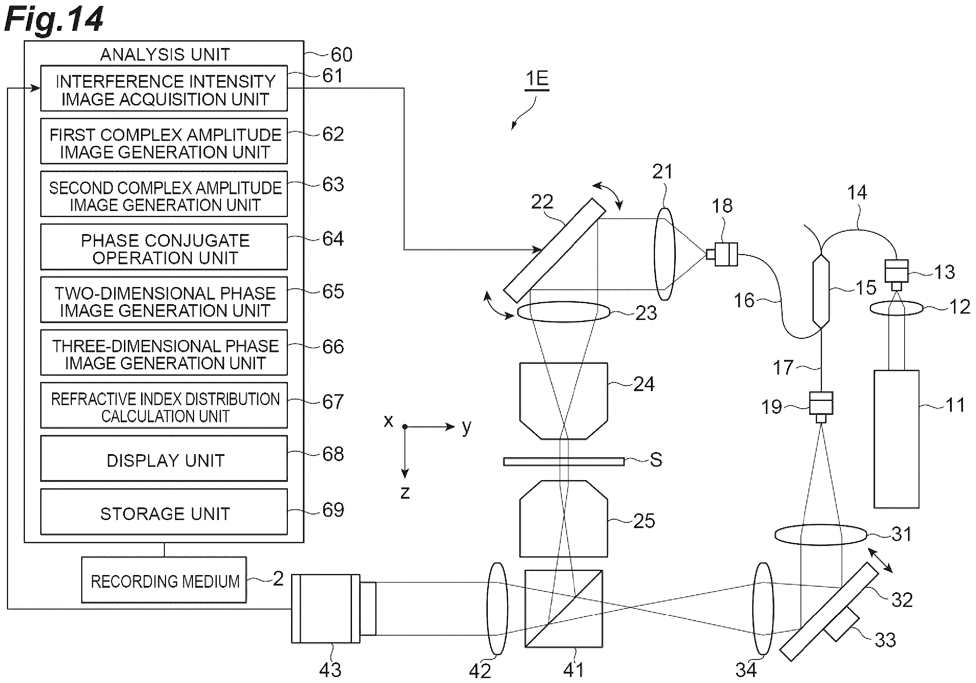

[FIG. 14] FIG. 14 (hereinafter also referred to as "FIG. B02") is a diagram illustrating a configuration of an observation apparatus 1E.

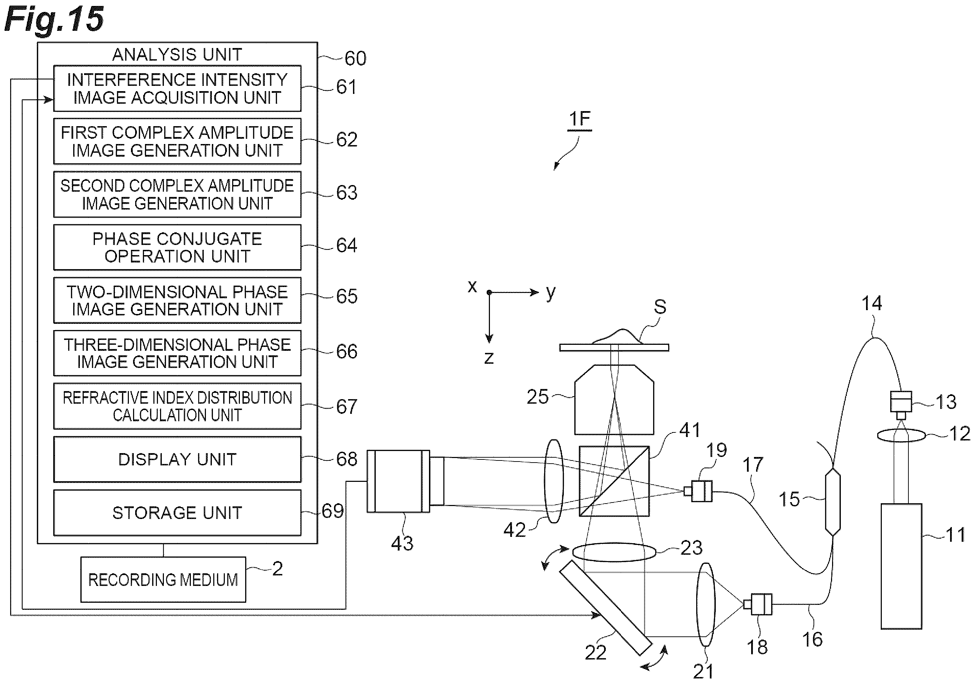

[FIG. 15] FIG. 15 (hereinafter also referred to as "FIG. B03") is a diagram illustrating a configuration of an observation apparatus 1F.

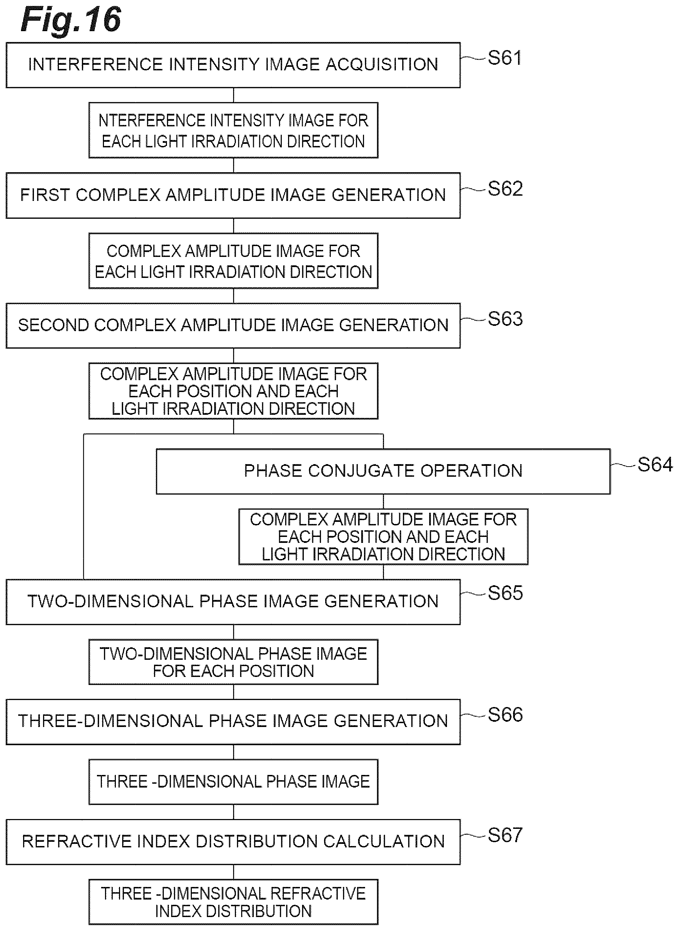

[FIG. 16] FIG. 16 (hereinafter also referred to as "FIG. B04") is a flowchart of a refractive index distribution measuring method B.

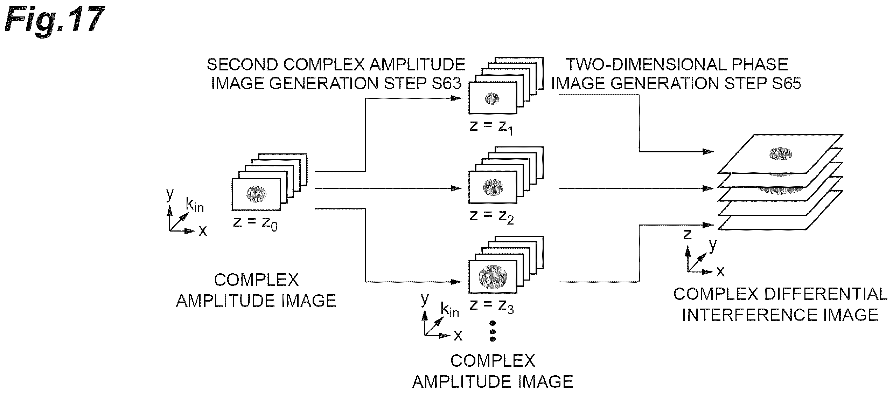

[FIG. 17] FIG. 17 (hereinafter also referred to as "FIG. B05") is a diagram illustrating images and an order of processing steps of a second complex amplitude image generation step S63 and a two-dimensional phase image generation step S65.

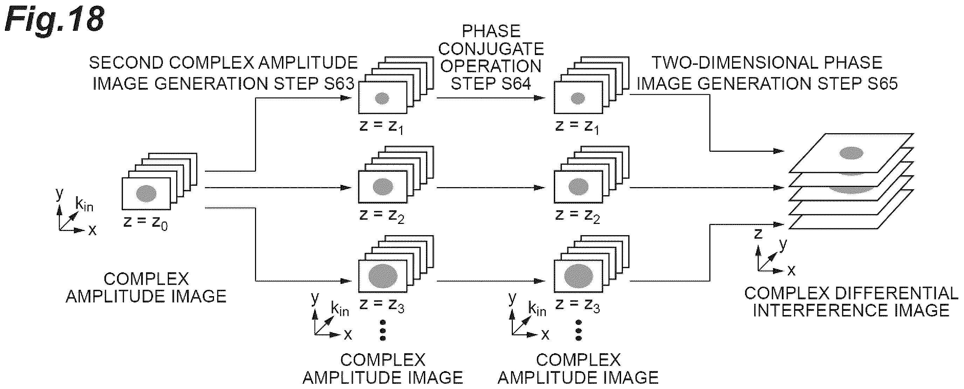

[FIG. 18] FIG. 18 (hereinafter also referred to as "FIG. B06") is a diagram illustrating images and an order of processing steps of the second complex amplitude image generation step S63, a phase conjugate operation step S64, and the two-dimensional phase image generation step S65.

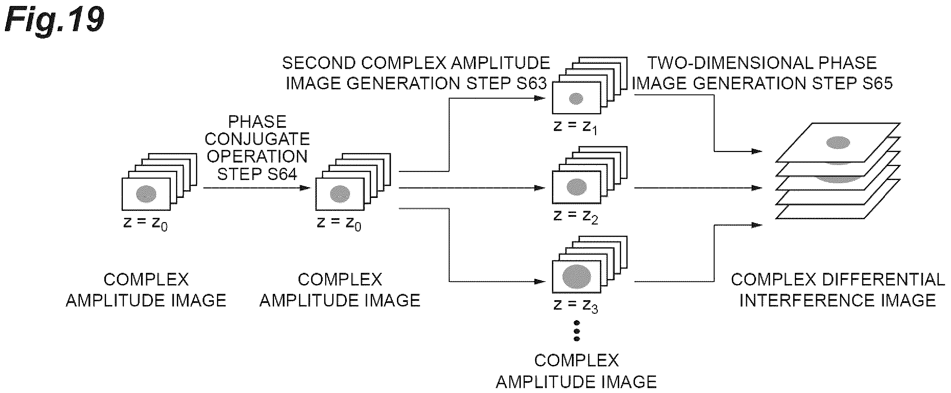

[FIG. 19] FIG. 19 (hereinafter also referred to as "FIG. B07") is a diagram illustrating images and an order of processing steps of the second complex amplitude image generation step S63, the phase conjugate operation step S64, and the two-dimensional phase image generation step S65.

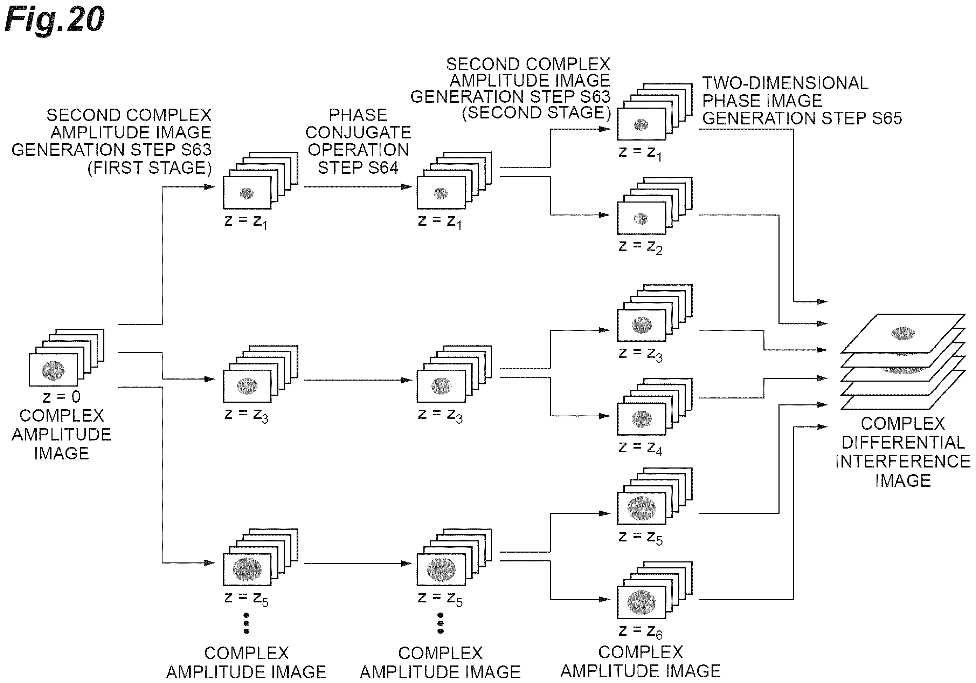

[FIG. 20] FIG. 20 (hereinafter also referred to as "FIG. B08") is a diagram illustrating images and an order of processing steps of the second complex amplitude image generation step S63, the phase conjugate operation step S64, and the two-dimensional phase image generation step S65.

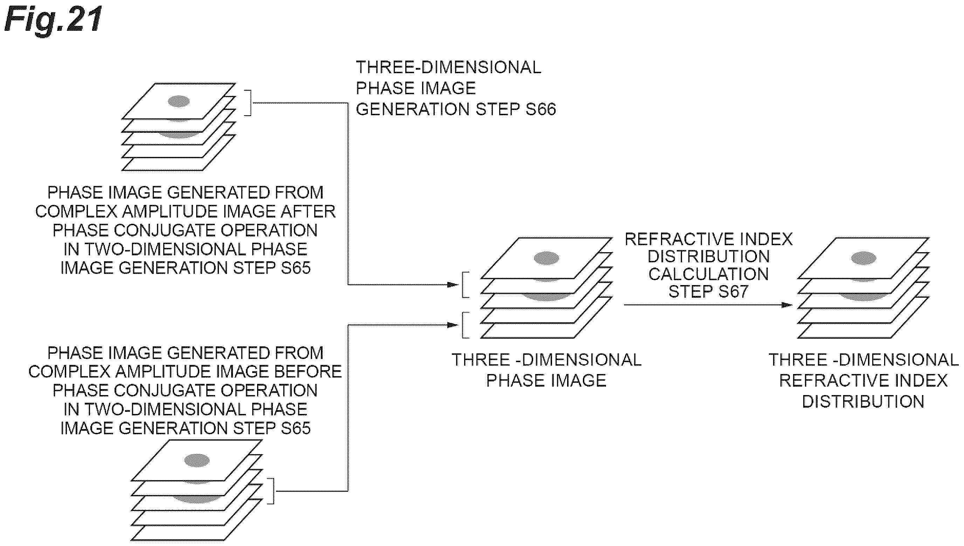

[FIG. 21] FIG. 21 (hereinafter also referred to as "FIG. B09") is a diagram illustrating images and an order of processing steps of a three-dimensional phase image generation step S66 and a refractive index distribution calculation step S67.

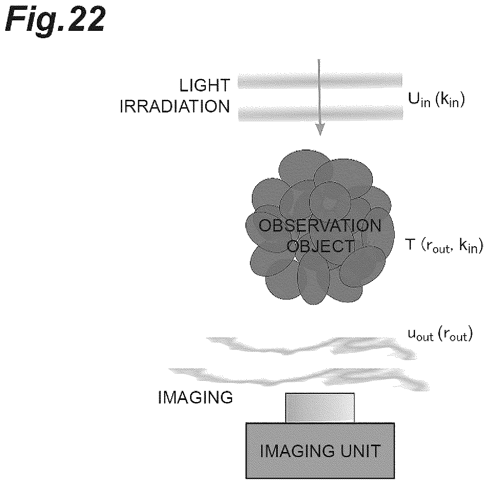

[FIG. 22] FIG. 22 (hereinafter also referred to as "FIG. B10") is a diagram for describing an outline of a phase conjugate operation, and is the diagram illustrating input light and output light when an interference intensity image is imaged by an imaging unit.

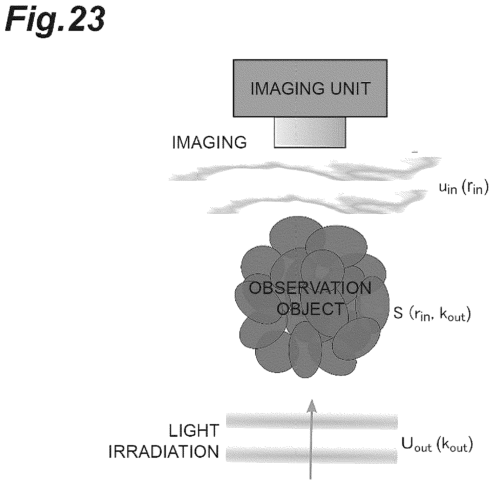

[FIG. 23] FIG. 23 (hereinafter also referred to as "FIG. B11") is a diagram for describing the outline of the phase conjugate operation, and is the diagram illustrating input light and output light in a case in which a relationship between light irradiation and imaging is reversed.

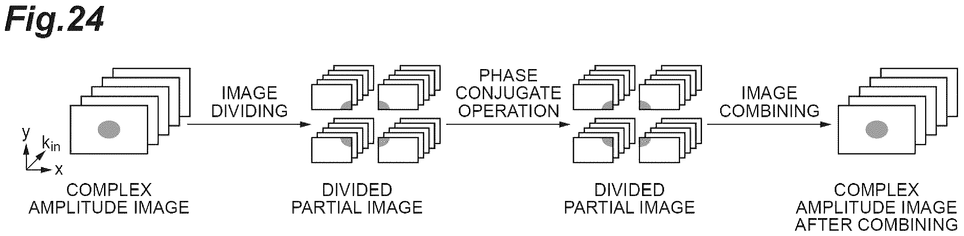

[FIG. 24] FIG. 24 (hereinafter also referred to as "FIG. B12") is a diagram illustrating image dividing, the phase conjugate operation, and image combining in the phase conjugate operation step S64.

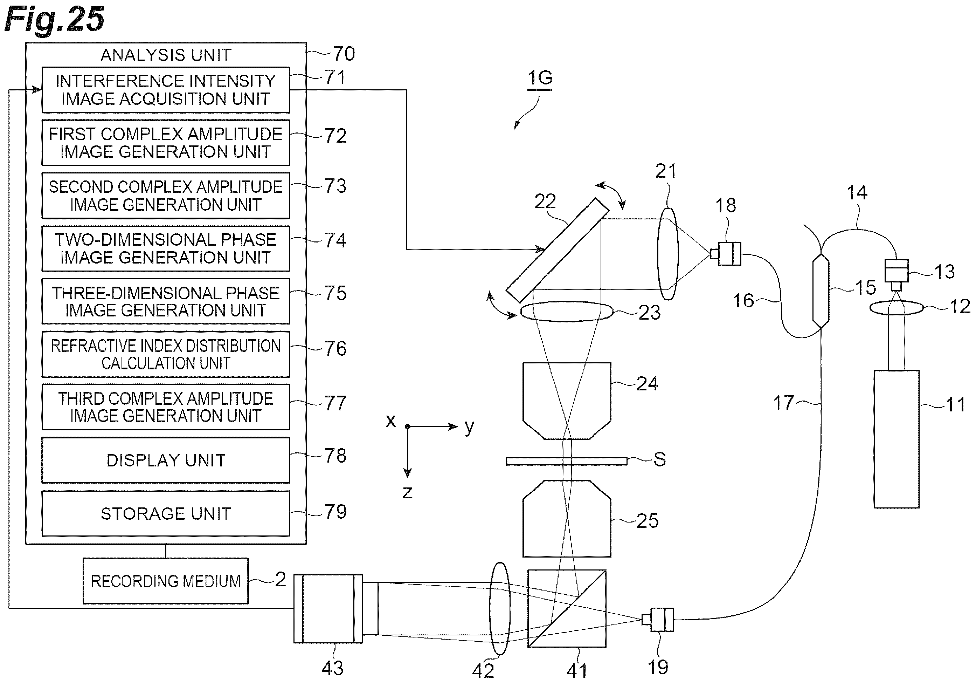

[FIG. 25] FIG. 25 (hereinafter also referred to as "FIG. C01") is a diagram illustrating a configuration of an observation apparatus 1G.

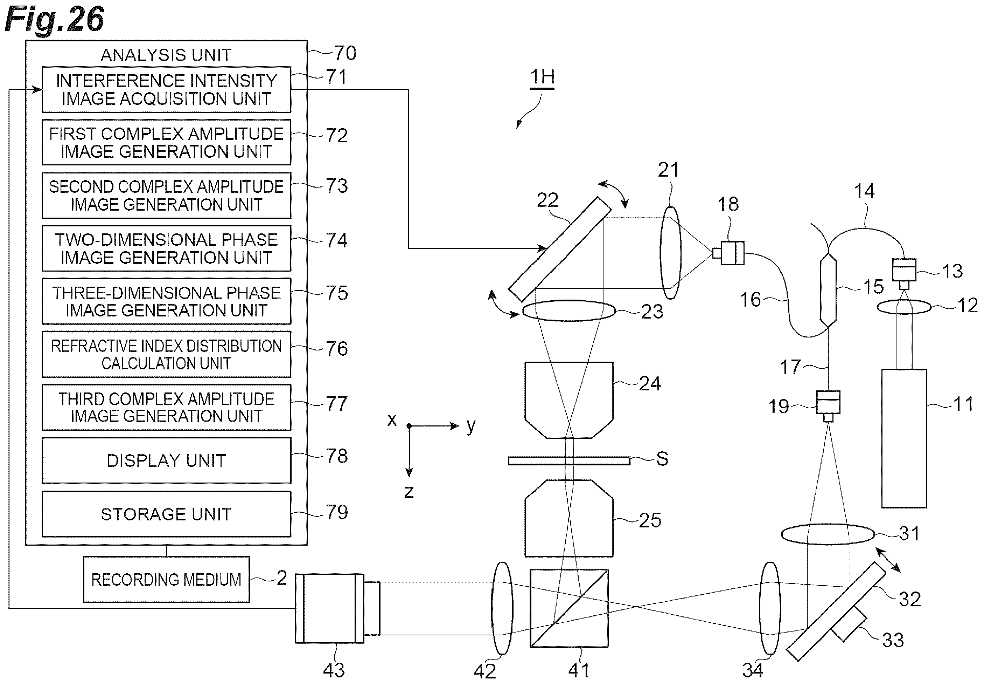

[FIG. 26] FIG. 26 (hereinafter also referred to as "FIG. C02") is a diagram illustrating a configuration of an observation apparatus 1H.

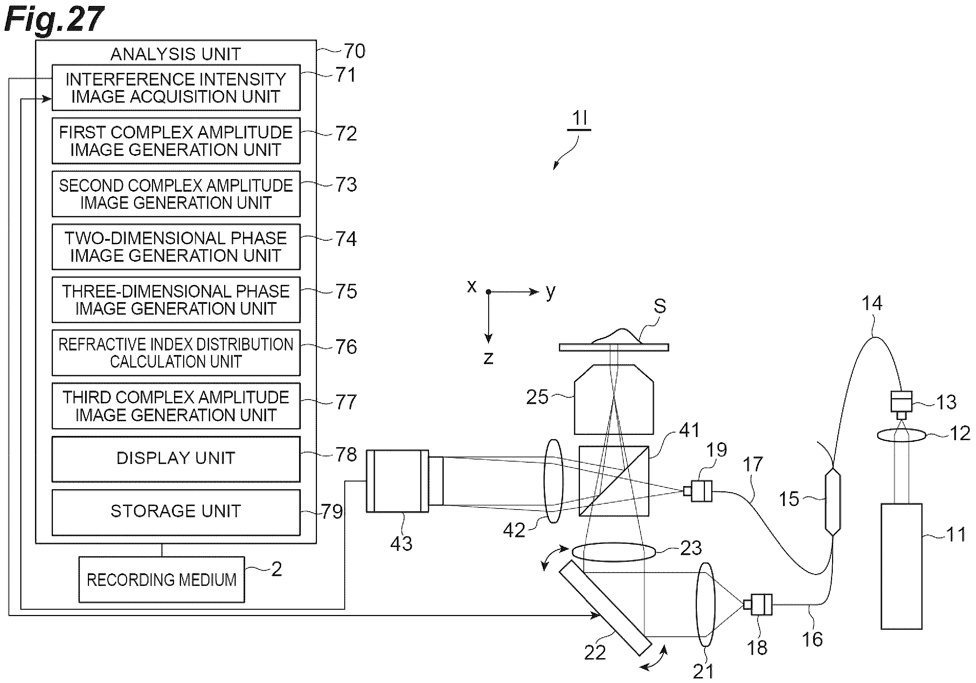

[FIG. 27] FIG. 27 (hereinafter also referred to as "FIG. C03") is a diagram illustrating a configuration of an observation apparatus 1I.

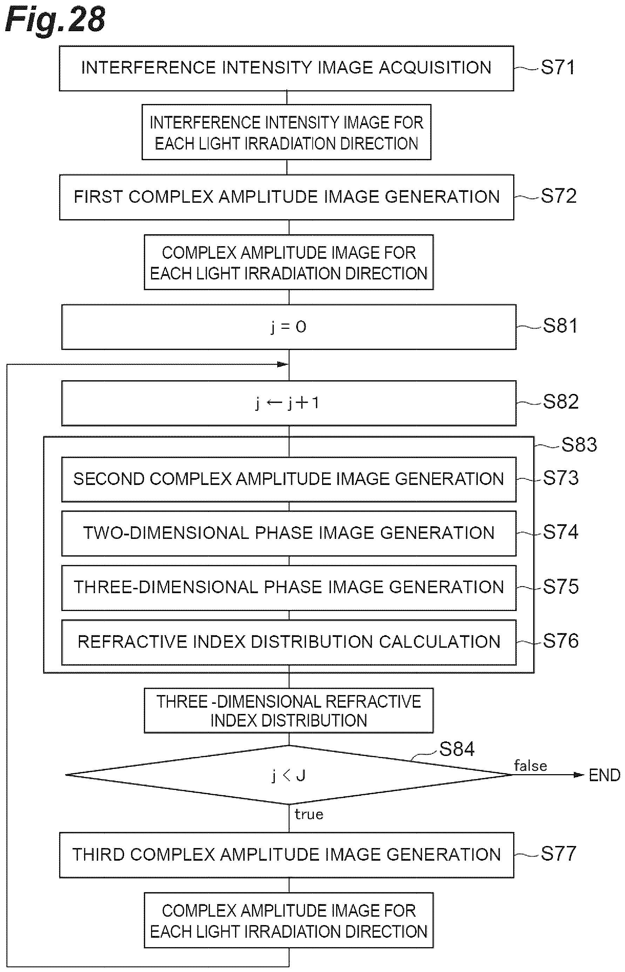

[FIG. 28] FIG. 28 (hereinafter also referred to as "FIG. C04") is a flowchart of a refractive index distribution measuring method C.

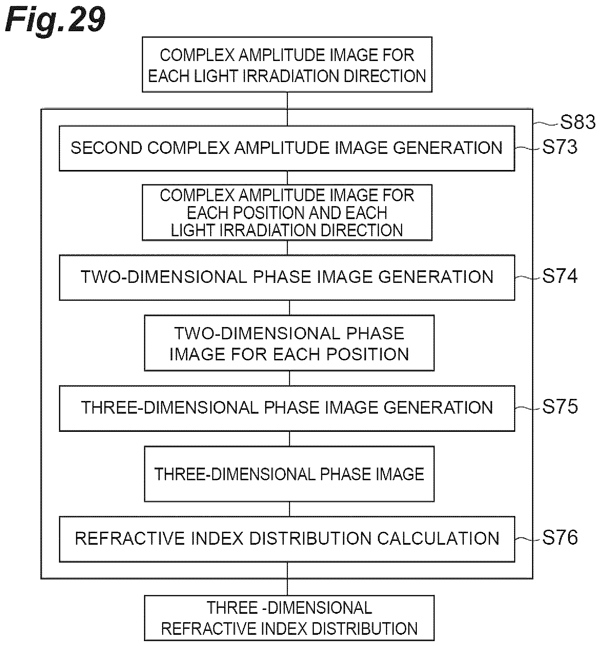

[FIG. 29] FIG. 29 (hereinafter also referred to as "FIG. C05") is a flowchart of a refractive index distribution measuring method C.

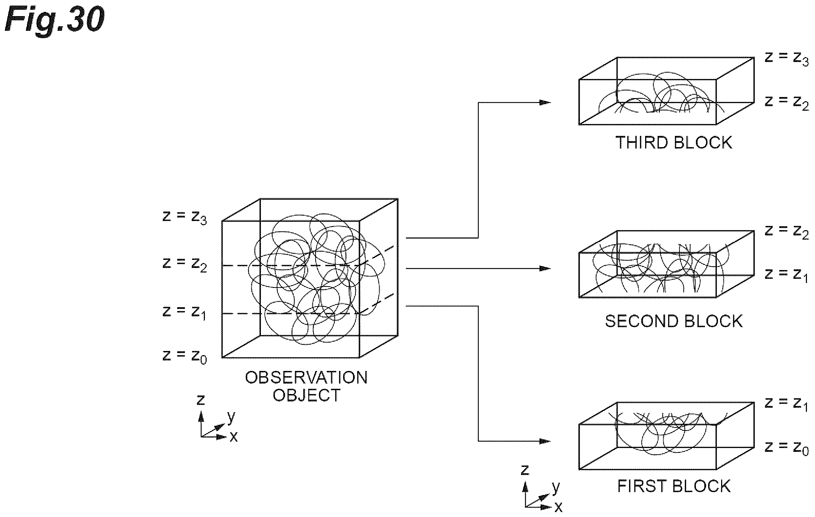

[FIG. 30] FIG. 30 (hereinafter also referred to as "FIG. C06") is a diagram illustrating a relationship between a region including an observation object and first to J-th blocks.

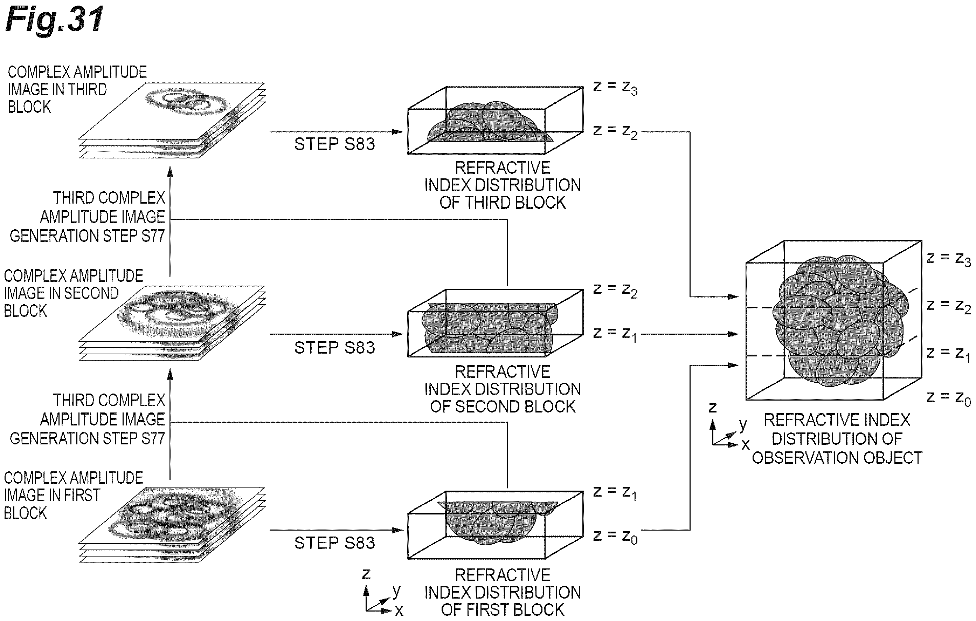

[FIG. 31] FIG. 31 (hereinafter also referred to as "FIG. C07") is a diagram illustrating a processing procedure for the first to J-th blocks.

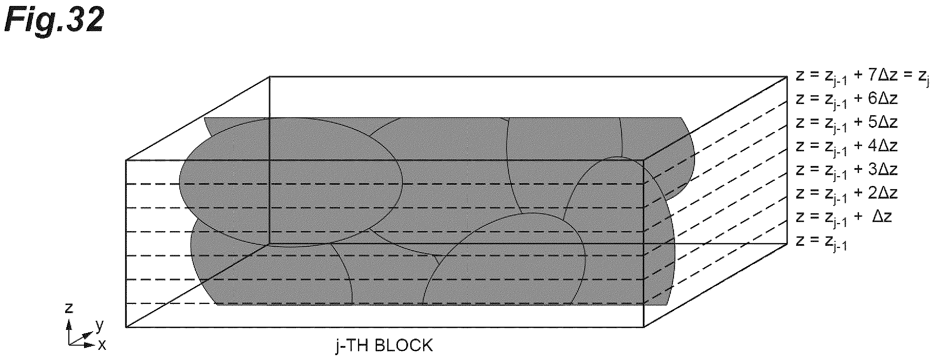

[FIG. 32] FIG. 32 (hereinafter also referred to as "FIG. C08") is a diagram illustrating processing contents of a BPM.

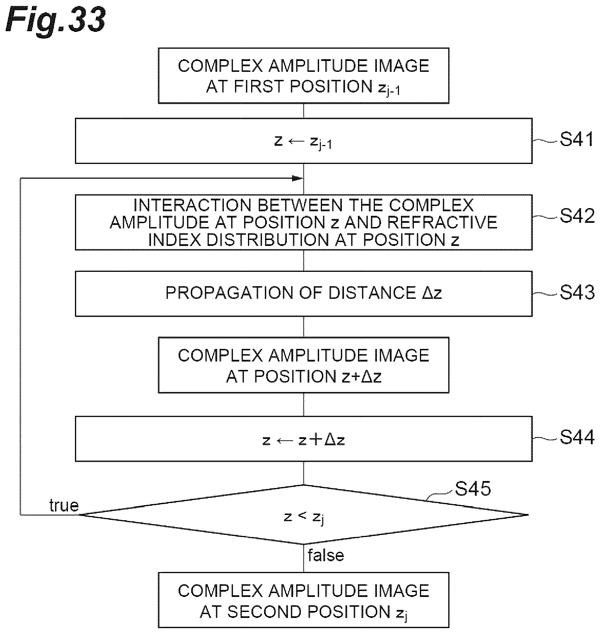

[FIG. 33] FIG. 33 (hereinafter also referred to as "FIG. C09") is a flowchart of a third complex amplitude image generation step S77.

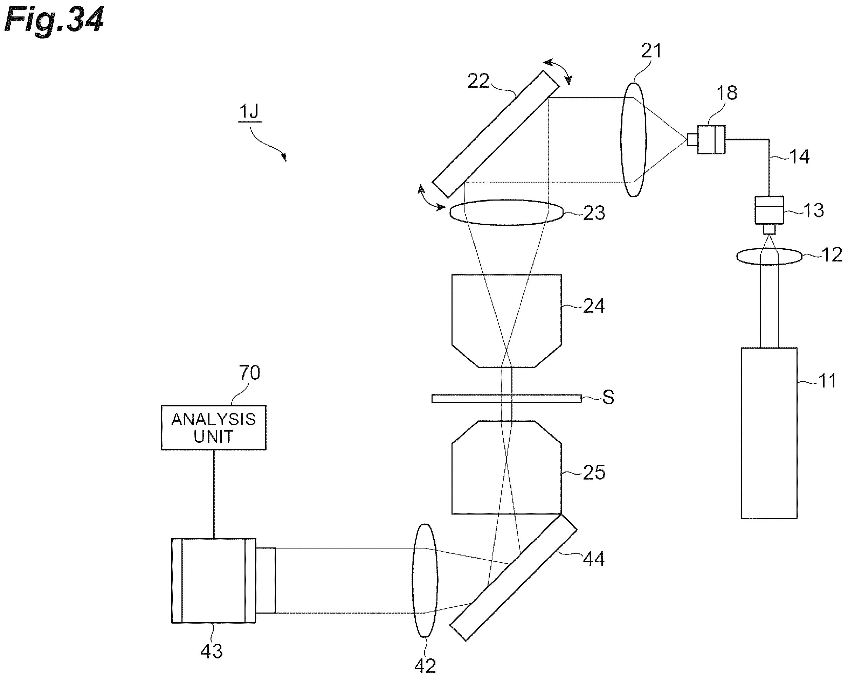

[FIG. 34] FIG. 34 (hereinafter also referred to as " FIG. C10") is a diagram illustrating a configuration of an observation apparatus 1J.

[FIG. 35] FIG. 35 is a drawing illustrating an example of a cluster of hepatocytes evaluated by a method for evaluating a cluster of hepatocytes according to a third aspect of the present disclosure.

[FIG. 36] FIG. 36 is a drawing illustrating an example of a method for evaluating a method for cultivating a cluster of hepatocytes according to a fourth aspect of the present disclosure, in which evaluation is performed on the basis of the number of bile canaliculus regions depending on the number of cultivation days of the cluster of hepatocytes.

[FIG. 37] FIG. 37 is a drawing illustrating an example of a drug screening method according to a fifth aspect of the present disclosure, in which a drug that may cause bile stasis-type DILI is screened on the basis of an area of a cross section of a bile canaliculus in a cluster of hepatocytes.

[FIG. 38] FIG. 38 is a drawing illustrating an example of the drug screening method according to the fifth aspect of the present disclosure, in which the drug that may cause the bile stasis-type DILI is screened on the basis of a length of a microvillus and/or the number of microvilli included in a bile canaliculus region in the cluster of hepatocytes.

[FIG. 39] FIG. 39 is a drawing illustrating an example of the drug screening method according to the fifth aspect of the present disclosure, in which the drug that may cause the bile stasis-type DILI is screened on the basis of a chronological change in the length of the microvillus after the drug is added.

[FIG. 40] FIG. 40 is a drawing illustrating an example of evaluating a bile canaliculus network formed by adding a taurocholic acid to a cluster of hepatocytes, in accordance with the method for identifying the bile canaliculus region according to the first aspect of the present disclosure.

[FIG. 41] FIG. 41 is a drawing illustrating an example of inserting a drain into a bile canaliculus by using as a guide the method for identifying the bile canaliculus region according to the first aspect of the present disclosure, to recover contents of a bile canaliculus in a cluster of hepatocytes.

[FIG. 42] FIG. 42 is a drawing illustrating an example of assisting diagnosis of liver disease in a clinical specimen of a liver by using the number of microvilli and/or a length of the microvillus as an index, in accordance with the method for evaluating the bile canaliculus region according to the second aspect of the present disclosure.

[FIG. 43] FIG. 43 is a drawing illustrating a result of identifying a bile canaliculus region of a two-dimensional liver culture using a phase contrast or and a fluorescence microscope in Example 1.

[FIG. 44] FIG. 44 is a drawing illustrating an outline of an evaluation result of a bile canaliculus region of a two-dimensional liver culture using optical diffraction tomography (ODT) in Example 2.

[FIG. 45] FIG. 45 is a drawing illustrating a detailed evaluation result of the bile canaliculus region of the two-dimensional liver culture using the optical diffraction tomography (ODT) in Example 2.

[FIG. 46] FIG. 46 is a drawing illustrating an outline of an evaluation result of a bile canaliculus region of a cluster 1 of hepatocytes using optical diffraction tomography (ODT) in Example 3.

[FIG. 47] FIG. 47 is a drawing illustrating a detailed evaluation result of the bile canaliculus region of the cluster 1 of hepatocytes using the optical diffraction tomography (ODT) in Example 3.

[FIG. 48] FIG. 48 is a drawing illustrating an evaluation result of a bile canaliculus region of a cluster 2 of hepatocytes using the optical diffraction tomography (ODT) in Example 3.

**Description of Embodiments**

Hereinafter, embodiments will be described in detail with reference to the drawings. Note that in the description of the drawings, the same reference numerals will be applied to the same constituents, and the repeated description will be omitted. The actual form is not limited to such an example.

An observation object according to one embodiment contains a hepatocyte. The hepatocyte indicates a cell that is derived from a liver and is capable of forming a bile canaliculus by a tight junction between the adjacent hepatocytes. The observation object according to one embodiment may contain both of a hepatocyte that forms the bile canaliculus and a hepatocyte that does not form the bile canaliculus. The hepatocyte may be a cultivated cell (including a cell line and a first cultivated cell) derived from a liver, or may be a hepatocyte included in an isolated liver tissue, and an animal species from which the hepatocyte is derived is not particularly limited. In a case where the observation object is a cell culture, the culture may be composed of one kind of hepatocytes, or may include two or more types of cells including at least one kind of hepatocyte.

The observation object according to one embodiment is an object mainly configured by cells, but may contain a component that is possibly contained in a tissue sample, such as an extracellular matrix and neutral fat, in addition to the cells. The observation object according to one embodiment may be a two-dimensional liver culture, a cluster of hepatocytes, or a liver tissue sample, from the viewpoint of the hepatocytes forming a tight junction. In addition, the observation object may be the two-dimensional liver culture, the cluster of hepatocytes, or the liver tissue sample, from the same viewpoint and from the viewpoint of ensuring the quantity and the quality of the bile canaliculus to be a determination and evaluation target. Further, the observation object according to one embodiment may be the cluster of hepatocytes, from the viewpoint of making an environment close to a physiological environment, high reproducibility, and ease of sample preparation compatible. The two-dimensional liver culture is a single-layer colony formed by the hepatocyte two-dimensionally cultivated on a flat dish or the like. The cluster of hepatocytes is a cluster of cells formed by the hepatocyte three-dimensionally cultivated. The liver tissue sample is a biological sample created by a liver tissue isolated from an animal including a human, and examples thereof include a tissue slide, a tissue block, a tissue panel, a tissue array, and the like. In a case where the observation object is the cluster of hepatocytes, the maximum diameter thereof may be 100 to 200 $\mu$m.

Refractive index distribution data is data indicating a three-dimensional distribution of a refractive index for each voxel in a space including the observation object, or data indicating a two-dimensional distribution of a refractive index for each pixel in a tomographic plane of the space including the observation object in a predetermined direction. In addition, refractive index tomography data is the data indicating the two-dimensional distribution of the refractive index for each of the pixels in the tomographic plane of the space including the observation object in the predetermined direction, in the refractive index distribution data.

A method for acquiring the refractive index distribution data according to one embodiment (hereinafter, also referred to as a "refractive index distribution measurement method") is not particularly limited, and a method as an example will be described below in detail. As a method for measuring the refractive index distribution of the observation object in a non-staining and non-invasive manner, optical diffraction tomography (ODT) is known. ODT is a developed technology enabling three-dimensional imaging of quantitative phase imaging (QPI), and is capable of attaining the three-dimensional refractive index tomography of the observation object.

Further, refractive index distribution measurement methods A to C described below are used. Embodiments of the refractive index distribution measurement method A include refractive index distribution measurement methods A1 to A3. The refractive index distribution measurement method A is an all-inclusive term for the refractive index distribution measurement methods A1 to A3. In such refractive index distribution measurement methods A to C, even in a case where the observation object is a multiple scatterer, it is possible to attain the three-dimensional refractive index tomography on which the influence of multiple scattering light is reduced.

Optical Coherence Tomography (OCT) is also known as another staining and non-invasive imaging technique. However, the resolution of OCT is about 10 $\mu$m, whereas the resolution of ODT and refractive index distribution measuring methods A to C is about 1 $\mu$m. In addition, OCT does not obtain a refractive index distribution, and it is difficult to biologically interpret a signal obtained by imaging. In these respects, ODT and the refractive index distribution measuring methods A to C are superior to OCT.

First, the refractive index distribution measuring method A (A1 to A3) will be described. FIG. A01 to A03 are diagrams showing respective configurations of observation apparatuses 1A to 1C that can be used when measuring the refractive index distribution by the refractive index distribution measuring method A.

FIG. A01 is a diagram illustrating a configuration of an observation apparatus 1A. The observation apparatus 1A includes a light source 11, a lens 12, a lens 21, a mirror 22, a lens 23, a condenser lens 24, an objective lens 25, a beam splitter 41, a lens 42, an imaging unit 43, and an analysis unit 50.

The light source 11 outputs spatially and temporally coherent light, and is preferably a laser light source. The lens 12 is optically coupled to the light source 11, focuses the light output from the light source 11 on a light input end 13 of an optical fiber 14, and inputs the light to the light input end 13. The optical fiber 14 guides the light input to the light input end 13 by the lens 12 to a fiber coupler 15. The fiber coupler 15 couples the light between the optical fiber 14 and optical fibers 16 and 17, splits the light guided by and arriving from the optical fiber 14 into two light beams, guides one split light by the optical fiber 16, and guides the other split light by the optical fiber 17. The light guided by the optical fiber 16 is output as diverging light from a light output end 18. The light guided by the optical fiber 17 is output as diverging light from a light output end 19.

The lens 21 is optically coupled to the light output end 18, and collimates the light output as the diverging light from the light output end 18. The mirror 22 is optically coupled to the lens 21, and reflects the light arriving from the lens 21 to the lens 23. An orientation of a reflection surface of the mirror 22 is changeable. The lens 23 is optically coupled to the mirror 22. The condenser lens 24 is optically coupled to the lens 23. The lens 23 and the condenser lens 24 preferably constitute a 4f optical system. The lens 23 and the condenser lens 24 irradiate an observation object S with the light from a light irradiation direction according to the orientation of the reflection surface of the mirror 22. The objective lens 25 is optically coupled to the condenser lens 24. The observation object S is disposed between the objective lens 25 and the condenser lens 24. The objective lens 25 inputs the light (object light) output from the condenser lens 24 and passed through the observation object S, and outputs the light to the beam splitter 41.

The beam splitter 41 is optically coupled to the objective lens 25, and further, is optically coupled also to the light output end 19. The beam splitter 41 combines the light (object light) output and arriving from the objective lens 25 and the light (reference light) output and arriving from the light output end 19, and outputs the light to the lens 42. The lens 42 is optically coupled to the beam splitter 41, collimates the object light and the reference light arriving from the beam splitter 41, and outputs the light to the imaging unit 43. The imaging unit 43 is optically coupled to the lens 42, and images an interference fringe image (interference intensity image) generated by interference between the object light and the reference light arriving from the lens 42. An incident direction of the reference light is inclined with respect to an incident direction of the object light on an imaging plane of the imaging unit 43. A position at which the object light and the reference light are combined by the beam splitter 41 may be in the subsequent stage of the imaging lens, and in addition, in consideration of the influence of aberration, it is desirable that the position is set between the objective lens 25 and the lens 42 as illustrated in the diagram.

The analysis unit 50 is electrically connected to the imaging unit 43, and inputs the interference intensity image captured by the imaging unit 43. The analysis unit 50 calculates a three-dimensional refractive index distribution of the observation object S by processing the input interference intensity image. The analysis unit 50 may be a computer. The analysis unit 50 includes an interference intensity image acquisition unit 51, a first complex amplitude image generation unit 52, a second complex amplitude image generation unit 53, a two-dimensional phase image generation unit 54, a three-dimensional phase image generation unit 55, a refractive index distribution calculation unit 56, a display unit 57, and a storage unit 58.

The interference intensity image acquisition unit 51 irradiates the observation object S with the light along each of a plurality of light irradiation directions by changing the orientation of the reflection surface of the mirror 22. Further, the interference intensity image acquisition unit 51 acquires the interference intensity image at a reference position for each of the plurality of light irradiation directions from the imaging unit 43. The interference intensity image acquisition unit 51 includes a CPU, has an output port for outputting a control signal for changing the orientation of the reflection surface of the mirror 22, and has an input port for inputting the interference intensity image from the imaging unit 43. It is not necessary to move the objective lens 25 in an optical axis direction. The reference position is an image plane position having a conjugate relationship with respect to the imaging plane of the imaging unit 43.

The first complex amplitude image generation unit 52, the second complex amplitude image generation unit 53, the two-dimensional phase image generation unit 54, the three-dimensional phase image generation unit 55, and the refractive index distribution calculation unit 56 perform processing based on the interference intensity images, and include a processing device such as a CPU, a GPU, a DSP, or an FPGA. The display unit 57 displays an image to be processed, an image in the middle of the processing, an image after the processing, and the like, and includes, for example, a liquid crystal display. The storage unit 58 stores data of various images, and includes a hard disk drive, a flash memory, a RAM, a ROM, and the like. The first complex amplitude image generation unit 52, the second complex amplitude image generation unit 53, the two-dimensional phase image generation unit 54, the three-dimensional phase image generation unit 55, the refractive index distribution calculation unit 56, and the storage unit 58 may be constituted by a cloud computing.

The storage unit 58 also stores a program for causing the interference intensity image acquisition unit 51, the first complex amplitude image generation unit 52, the second complex amplitude image generation unit 53, the two-dimensional phase image generation unit 54, the three-dimensional phase image generation unit 55, and the refractive index distribution calculation unit 56, to execute respective steps of the processing. The program may be stored in the storage unit 58 at the time of manufacture or shipment of the observation apparatus 1A, may be acquired via a communication line after shipment and then stored in the storage unit 58, or may be recorded in a computer readable recording medium 2 and then stored in the storage unit 58. The recording medium 2 may be an arbitrary medium such as a flexible disk, a CD-ROM, a DVD-ROM, a BD-ROM, a USB memory, or the like.

The details of the processing step of each of the interference intensity image acquisition unit 51, the first complex amplitude image generation unit 52, the second complex amplitude image generation unit 53, the two-dimensional phase image generation unit 54, the three-dimensional phase image generation unit 55, and the refractive index distribution calculation unit 56 will be described later.

FIG. A02 is a diagram illustrating a configuration of an observation apparatus 1B. The observation apparatus 1B illustrated in FIG. A02 includes a lens 31, a mirror 32, and a lens 34 in addition to the configuration of the observation apparatus 1A illustrated in FIG. A01.

The lens 31 is optically coupled to the light output end 19, and collimates the light (reference light) output as diverging light from the light output end 19. The mirror 32 is optically coupled to the lens 31, and reflects the light arriving from the lens 31 to the lens 34. The lens 34 is optically coupled to the mirror 32, and outputs the light arriving from the mirror 32 to the beam splitter 41. The light output from the lens 34 is once focused before the beam splitter 41, and then input to the beam splitter 41 as diverging light. The beam splitter 41 combines the light (object light) output and arriving from the objective lens 25 and the light (reference light) output and arriving from the lens 34, and outputs the light to the lens 42 in a coaxial manner. The imaging unit 43 images the interference fringe image (interference intensity image) generated by interference between the object light and the reference light arriving from the lens 42. The incident direction of the reference light is parallel to the incident direction of the object light on the imaging plane of the imaging unit 43.

A drive unit 33 moves the mirror 32 in a direction perpendicular to a reflection surface of the mirror 32. The drive unit 33 is, for example, a piezoelectric actuator. The movement of the mirror 32 changes an optical path difference (phase difference) of the object light and the reference light from light splitting by the fiber coupler 15 to combining by the beam splitter 41. When the optical path difference is different, the interference intensity image captured by the imaging unit 43 is also different.

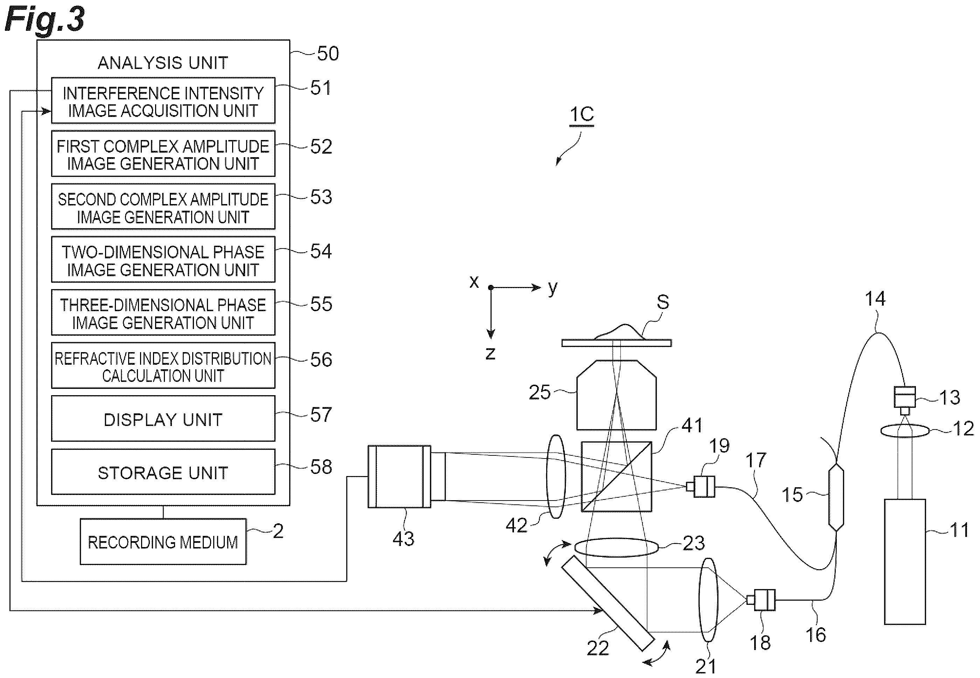

The observation apparatus is not limited to the configuration examples illustrated in FIG. A01 and FIG. A02, and various modifications are possible. In the configuration of the observation apparatus 1A (FIG. A01) and the observation apparatus 1B (FIG. A02), the object light transmitted through the observation object S is observed, and the object light reflected by the observation object S may be observed as in a configuration of an observation apparatus 1C (FIG. 3) described below.

FIG. A03 is a diagram illustrating a configuration of an observation apparatus 1C. The observation apparatus 1C includes the light source 11, the lens 12, the lens 21, the mirror 22, the lens 23, the objective lens 25, the beam splitter 41, the lens 42, the imaging unit 43, and the analysis unit 50. Hereinafter, differences from the observation apparatus 1A (FIG. A01) will be mainly described.

The lens 21 is optically coupled to the light output end 18 of the optical fiber 16, and collimates the light output as diverging light from the light output end 18. The mirror 22 is optically coupled to the lens 21, and reflects the light arriving from the lens 21 to the lens 23. The orientation of the reflection surface of the mirror 22 is changeable. The lens 23 is optically coupled to the mirror 22. The objective lens 25 is optically coupled to the lens 23. The beam splitter 41 is disposed between the lens 23 and the objective lens 25. The lens 23 and the objective lens 25 preferably constitute a 4f optical system. The lens 23 and the objective lens 25 irradiate the observation object S with the light from the light irradiation direction according to the orientation of the reflection surface of the mirror 22. The objective lens 25 inputs the light (object light) reflected from the observation object S, and outputs the light to the beam splitter 41.

The beam splitter 41 is optically coupled to the objective lens 25, and further, is optically coupled also to the light output end 19 of the optical fiber 17. The beam splitter 41 combines the light (object light) output and arriving from the objective lens 25 and the light (reference light) output and arriving from the light output end 19, and outputs the light to the lens 42. The lens 42 is optically coupled to the beam splitter 41, collimates the object light and the reference light arriving from the beam splitter 41, and outputs the light to the imaging unit 43. The imaging unit 43 is optically coupled to the lens 42, and images the interference fringe image (interference intensity image) generated by interference between the object light and the reference light arriving from the lens 42. The incident direction of the reference light is inclined with respect to the incident direction of the object light on the imaging plane of the imaging unit 43. The position at which the object light and the reference light are combined by the beam splitter 41 may be in the subsequent stage of the imaging lens, and in addition, in consideration of the influence of aberration, it is desirable that the position is set between the objective lens 25 and the lens 42 as illustrated in the diagram.

In the configuration of the observation apparatus 1C (FIG. A03), as in the observation apparatus 1B (FIG. A02), the mechanism (the lens 31, the mirror 32, the drive unit 33, and the lens 34 in FIG. A02) for changing the optical path length of the reference light may be provided for changing the optical path difference (phase difference) of the object light and the reference light from light splitting by the fiber coupler 15 to combining by the beam splitter 41. In this case, the incident direction of the reference light may be parallel to the incident direction of the object light on the imaging plane of the imaging unit 43.

FIG. A04 is a flowchart of a refractive index distribution measuring method A. The refractive index distribution measuring method A can be applied to each of the observation apparatus 1A to 1C. The refractive index distribution measuring method A includes an interference intensity image acquisition step S1, a first complex amplitude image generation step S2, a second complex amplitude image generation step S3, a two-dimensional phase image generation step S4, a three-dimensional phase image generation step S5, and a refractive index distribution calculation step S6.

The processing step of the interference intensity image acquisition step S1 is performed by the interference intensity image acquisition unit 51. The processing step of the first complex amplitude image generation step S2 is performed by the first complex amplitude image generation unit 52. The processing step of the second complex amplitude image generation step S3 is performed by the second complex amplitude image generation unit 53. The processing step of the two-dimensional phase image generation step S4 is performed by the two-dimensional phase image generation unit 54. The processing step of the three-dimensional phase image generation step S5 is performed by the three-dimensional phase image generation unit 55. The processing step of the refractive index distribution calculation step S6 is performed by the refractive index distribution calculation unit 56.

In the interference intensity image acquisition step S1, the interference intensity image acquisition unit 51 irradiates the observation object S with the light along each of the plurality of light irradiation directions by changing the orientation of the reflection surface of the mirror 22. Further, the interference intensity image acquisition unit 51 acquires the interference intensity image at the reference position for each of the plurality of light irradiation directions from the imaging unit 43.

In each of FIG. A01 to FIG. A03, an xyz orthogonal coordinate system is illustrated for convenience of explanation. The z axis is parallel to the optical axis of the objective lens 25. The reference position is the image plane position having a conjugate relationship with respect to the imaging plane of the imaging unit 43. This position is set to $z = 0$. The light irradiation direction on the observation object S can be represented by $k_x$ and $k_y$ in a wavenumber vector ($k_x$, $k_y$, $k_z$) of the irradiation light.

(a) to (c) in FIG. A05 are diagrams illustrating examples of scanning of the light irradiation direction on the observation object S in the interference intensity image acquisition step S1. In the diagram, a position of each circular point represents the light irradiation direction in the $k_x k_y$ plane in which the horizontal axis is set to $k_x$ and the vertical axis is set to $k_y$. The scanning of the light irradiation direction may be arranged in a rectangular lattice shape in the $k_x k_y$ plane as illustrated in (a) in FIG. A05, may be arranged on a circumference of each of a plurality of concentric circles in the $k_x k_y$ plane as illustrated in (b) in FIG. A05, or may be arranged in a spiral shape in the $k_x k_y$ plane as illustrated in (c) in FIG. A05. In any of the cases, the light irradiation direction can be scanned as far as it is allowed by Numerical Aperture (NA) of the condenser lens 24. Raster scan or random scan may be used. In the case of the raster scan, return scan may be performed or may not be performed.

In the first complex amplitude image generation step S2, the first complex amplitude image generation unit 52 generates, for each of the plurality of light irradiation directions, a complex amplitude image based on the interference intensity image acquired by the interference intensity image acquisition unit 51. In the case of the observation apparatus 1A (FIG. A01) or the observation apparatus 1C (FIG. A03), the first complex amplitude image generation unit 52 can generate the complex amplitude image based on one interference intensity image by a Fourier fringe analysis method. In the case of the observation apparatus 1B (FIG. A02), the first complex amplitude image generation unit 52 can generate the complex amplitude image based on three or more interference intensity images having different optical path differences (phase differences) between the object light and the reference light by a phase shift method.

In the second complex amplitude image generation step S3, the second complex amplitude image generation unit 53 generates, for each of the plurality of light irradiation directions, a complex amplitude image at each of a plurality of z direction positions based on the complex amplitude image at a reference position ($z = 0$) generated by the first complex amplitude image generation unit 52. Assuming that a two-dimensional Fourier transform of the complex amplitude image $u(x, y, 0)$ at the reference position is $U(k_x, k_y, 0)$, the complex amplitude image $u(x, y, d)$ at the position of $z = d$ and the two-dimensional Fourier transform $U(k_x, k_y, d)$ of the complex amplitude image $u(x, y, d)$ are represented by the following Formulas. i is an imaginary unit, and $k_0$ is a wavenumber of the light in the observation object.

[Formula 1]

$$U(k_x, k_y, d) = U(k_x, k_y, 0) \exp(i\sqrt{k_0^2 - k_x^2 - k_y^2}\, d) \qquad (1)$$

[Formula 2]

$$u(x, y, d) = \int U(k_x, k_y, d) \exp(-ik_x x - ik_y y)\, dk_x dk_y \qquad (2)$$

In the two-dimensional phase image generation step S4, the two-dimensional phase image generation unit 54 generates, for each of the plurality of positions, a two-dimensional phase image based on the complex amplitude image of each of the plurality of light irradiation directions generated by the second complex amplitude image generation unit 53. The two-dimensional phase image generated in this step corresponds to a phase image centered on the focused z direction position. The details of the two-dimensional phase image generation step S4 will be described below.

In addition, the two-dimensional phase image generation step S4 and the subsequent steps may be performed after all the complex amplitude images at the plurality of positions are generated for each of the plurality of light irradiation directions in the second complex amplitude image generation step S3. Further, processes of generating the complex amplitude image at one certain z direction position for each of the plurality of light irradiation directions in the second complex amplitude image generation step S3 and generating the two-dimensional phase image at the position in the two-dimensional phase image generation step S4 may be set as a unit, and the unit process may be repeatedly performed while scanning the z direction position. The latter case is preferable in that a capacity of image data to be stored in the storage unit 58 can be reduced.

In the three-dimensional phase image generation step S5, the three-dimensional phase image generation unit 55 generates a three-dimensional phase image based on the two-dimensional phase image at each of the plurality of positions generated by the two-dimensional phase image generation unit 54. The three-dimensional phase image generated in this step is an image in which the positions x and y in the two-dimensional phase image and the position z of the two-dimensional phase image are variables.

In the refractive index distribution calculation step S6, the refractive index distribution calculation unit 56 obtains a three-dimensional refractive index distribution of the observation object by deconvolution based on the three-dimensional phase image generated by the three-dimensional phase image generation unit 55. Assuming that the refractive index distribution of the observation object is n(x, y, z), an electric susceptibility distribution is f(x, y, z), and a refractive index of a background medium is $n_m$, there is a relationship of the following Formula (3) between them. The three-dimensional phase image $\Phi(x, y, z)$ generated by the three-dimensional phase image generation unit 55 is represented by convolution of a kernel function g(x, y, z) and the electric susceptibility distribution f(x, y, z) as shown in the following Formula (4). Therefore, the three-dimensional refractive index distribution n(x, y, z) of the observation object can be obtained by deconvolution based on the three-dimensional phase image $\Phi(x, y, z)$.

[Formula 3]

$$f(x, y, z) = k_0^2 \left[ \left( n(x, y, z)/n_m \right)^2 - 1 \right] \qquad (3)$$

[Formula 4]

$$\Phi(x, y, z) = \int g(x - x', y - y', z - z') f(x', y', z') dx' dy' dz' \qquad (4)$$

In addition, the kernel function g is a function based on a Green function corresponding to a solution of a wave equation. FIG. A06 is a diagram showing the kernel function g. In this diagram, a center position having the largest value of the kernel function g is the origin, the vertical direction is the z axis, and the horizontal direction is the direction perpendicular to the z axis.

Each of the processing steps of the first complex amplitude image generation step S2, the second complex amplitude image generation step S3, the two-dimensional phase image generation step S4, the three-dimensional phase image generation step S5, and the refractive index distribution calculation step S6 may be performed each time the interference intensity image of each of a predetermined number of light irradiation directions is acquired in the interference intensity image acquisition step S1 (FIG. A07), or may be performed each time the interference intensity image of one light irradiation direction is acquired in the interference intensity image acquisition step S1 (FIG. A08).

FIG. A07 and FIG. A08 are diagrams illustrating examples of scanning of the light irradiation direction on the observation object S in the interference intensity image acquisition step S1. In these diagrams, a position of each circular point represents the light irradiation direction in the $k_x k_y$ plane in which the horizontal axis is set to $k_x$ and the vertical axis is set to $k_y$. In the examples of scanning of the light irradiation direction illustrated in these diagrams, the light irradiation direction is sequentially changed, and the light irradiation direction at the time of acquisition of the (N+n)-th interference intensity image is made to coincide with the light irradiation direction at the time of acquisition of the n-th interference intensity image. n is a positive integer, and N is an integer of 2 or more.

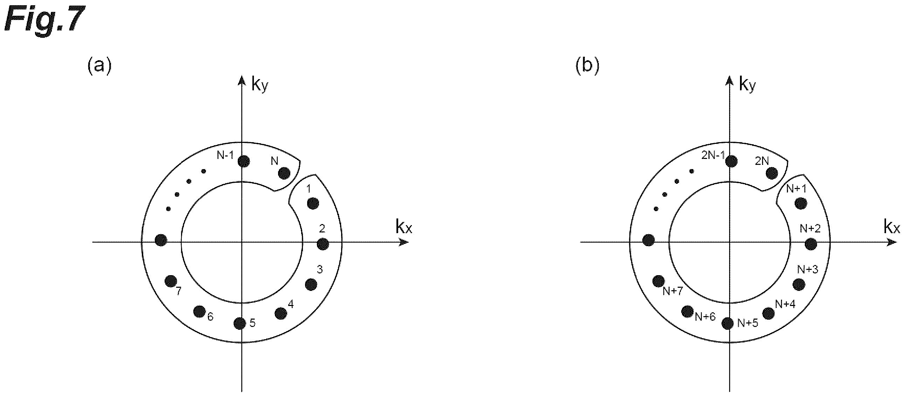

In the example illustrated in FIG. A07, when the first to N-th interference intensity images are acquired in the interference intensity image acquisition step S61, the respective processing steps of the steps S2 to S6 are performed based on the first to N-th interference intensity images ((a) in FIG. 7). Next, when the (N+1)-th to 2N-th interference intensity images are acquired in the interference intensity image acquisition step S1, the respective processing steps of the steps S2 to S6 are performed based on the (N+1)-th to 2N-th interference intensity images ((b) in FIG. A07). Next, when the (2N+1)-th to 3N-th interference intensity images are acquired in the interference intensity image acquisition step S1, the respective processing steps of the steps S2 to S6 are performed based on the (2N+1)-th to 3N-th interference intensity images. The same applies thereafter.

In the example illustrated in FIG. A08, when the first to N-th interference intensity images are acquired in the interference intensity image acquisition step S1, the respective processing steps of the steps S2 to S6 are performed based on the first to N-th interference intensity images ((a) in FIG. A08). Next, when the (N+1)-th interference intensity image is acquired in the interference intensity image acquisition step S61, the respective processing steps of the steps S2 to S6 are performed based on the latest N interference intensity images (the second to (N+1)-th interference intensity images) including the (N+1)-th interference intensity image ((b) in FIG. A08). Next, when the (N+2)-th interference intensity image is acquired in the interference intensity image acquisition step S1, the respective processing steps of the steps S2 to S6 are performed based on the latest N interference intensity images (the third to (N+2)-th interference intensity images) including the (N+2)-th interference intensity image ((c) in FIG. A08). The same applies thereafter, and when the (N+n)-th interference intensity image is acquired in the interference intensity image acquisition step S1, the respective processing steps of the steps S2 to S6 are performed based on the latest N interference intensity images (the (1+n)-th to (N+n)-th interference intensity images) including the (N+n)-th interference intensity image.

Compared with the example illustrated in FIG. A07, in the example illustrated in FIG. A08, each time the interference intensity image of one light irradiation direction is acquired in the interference intensity image acquisition step S1, the respective processing steps of the steps S2 to S6 are performed based on the plurality of latest interference intensity images including the acquired interference intensity image, and thus, the number of images obtained per unit time by the respective processing steps of the steps S2 to S6 is large.

Next, the details of the two-dimensional phase image generation step S4 in the refractive index distribution measuring method A will be described. In the two-dimensional phase image generation step S4, the two-dimensional phase image generation unit 54 generates, for each of the plurality of positions, the two-dimensional phase image based on the complex amplitude image of each of the plurality of light irradiation directions generated by the second complex amplitude image generation unit 53. The two-dimensional phase image generation step S4 depends on the refractive index distribution measuring method A1 to A3.

FIG. A09 is a flowchart of the two-dimensional phase image generation step S4 in the refractive index distribution measuring method A1. In the refractive index distribution measuring method A1, for each of the plurality of positions, the two-dimensional phase image generation step S4, in a step S11, corrects the phase of the complex amplitude image of each of the plurality of light irradiation directions based on the light irradiation direction, and then generates a complex amplitude summation image representing a summation of the complex amplitude images after the correction, and in a step S12, generates the two-dimensional phase image based on the complex amplitude summation image.

The processing of the step S11 is based on a CASS (Collective Accumulation of Single Scattering; Sungsam Kang, et al, "Imaging deep within a scattering medium using collective accumulation of single-scattered waves," NATURE PHOTONICS, Vol.9, pp.253-258 (2015)) technique. In the light with which the object is irradiated along a certain light irradiation direction and passed through the object, a spatial frequency distribution of the single scattered light which interacts with the object only once is shifted according to the light irradiation direction, whereas a spatial frequency distribution of the multiple scattered light which interacts with the object a plurality of times randomly changes according to the light irradiation direction. The CASS technique uses the above difference between the light irradiation direction dependencies of the spatial frequency distributions of the single scattered light and the multiple scattered light.

That is, in the step S11, the phase of the complex amplitude image of each of the plurality of light irradiation directions is corrected based on the light irradiation direction (that is, the spatial frequency distribution of the complex amplitude image is shifted in parallel according to the light irradiation direction in the spatial frequency domain), so that the spatial frequency distribution of the single scattered light component in the complex amplitude image has a shape and arrangement independent of the light irradiation direction, while the spatial frequency distribution of the multiple scattered light component in the complex amplitude image has a random shape and arrangement. Further, in the step S11, the complex amplitude summation image representing the summation of the plurality of complex amplitude images after the above correction is generated (that is, synthetic aperture processing is performed) to coherently sum the single scattered light components in the complex amplitude images, while the multiple scattered light components in the complex amplitude images cancel each other out.

Therefore, the influence of the multiple scattered light is reduced in the complex amplitude summation image generated in the step S11. Further, the three-dimensional refractive index distribution obtained finally in the refractive index distribution calculation step S6 also reduces the influence of the multiple scattered light, suppresses the speckles, and improves the Single-scattering to Multi-scattering Ratio (SMR).

FIG. A10 is a flowchart of the two-dimensional phase image generation step S4 in the refractive index distribution measuring method A2. In the refractive index distribution measuring method A2, for each of the plurality of positions, the two-dimensional phase image generation step S4, in a step S21, generates a complex differential interference image of each of the plurality of light irradiation directions based on the complex amplitude image of each of the plurality of light irradiation directions. In a step S22, the step generates a phase differential image based on a summation of the complex differential interference images of the plurality of light irradiation directions. In a step S23, the step generates the two-dimensional phase image based on the phase differential image.

Assuming that the complex amplitude image at the position of z = d is u(x, y, d), the complex differential interference image q(x, y, d) generated in the step S21 is represented by the following Formula (5). At least one of $\delta x$ and $\delta y$ is non-zero. When $\delta x = 0$ and $\delta y = 0$, the complex differential interference image q in which the x direction is a shear direction is obtained. When $\delta x = 0$ and $\delta y \neq 0$, the complex differential interference image q in which the y direction is the shear direction is obtained. When $\delta x \neq 0$ and $\delta y \neq 0$, the complex differential interference image q with the shear direction different from both of the x direction and the y direction is obtained. In addition, the complex differential interference image q(x, y, d) may be obtained by Formula (5) after transforming the complex amplitude image u(x, y, d) as in the following Formula (6).

[Formula 5]

$$q(x, y, d) = u^*(x + \delta x, y + \delta y, d) \cdot u(x, y, d) \qquad (5)$$

[Formula 6]

$$u(x, y, d) \exp(-ik_x x - ik_y y) \qquad (6)$$

Assuming that the summation of the complex differential interference images q of the plurality of light irradiation directions is $q_{sum}(x, y, d)$, the phase differential image $\phi(x, y, z)$ generated in the step S22 is represented by the following Formula (7) as the phase of $q_{sum}(x, y, d)$. In the step S23, the two-dimensional phase image can be generated by performing integration or deconvolution of the phase differential image $\phi(x, y, z)$.

[Formula 7]

$$\phi(x, y, d) = \angle q_{sum}(x, y, d) \qquad (7)$$

In addition, in the step S21, the complex differential interference image may be generated for each of a plurality of shear directions different from each other on the complex amplitude image. In this case, for each of the plurality of positions, the two-dimensional phase image generation step S4, in the step S21, generates the complex differential interference image of each of the plurality of light irradiation directions for each of the plurality of shear directions on the image different from each other based on the complex amplitude image of each of the plurality of light irradiation directions. In the step S22, the step generates the phase differential image based on the summation of the complex differential interference images of the plurality of light irradiation directions for each of the plurality of shear directions. In the step S23, the step generates the two-dimensional phase image based on the phase differential image of each of the plurality of shear directions.

The influence of the multiple scattered light is reduced in the phase differential image generated based on the summation of the complex differential interference image of each of the plurality of light irradiation directions in the step S22. Further, the three-dimensional refractive index distribution obtained finally in the refractive index distribution calculation step S6 also reduces the influence of the multiple scattered light, and suppresses the speckles. Further, when the complex differential interference image is generated for each of the plurality of shear directions different from each other on the complex amplitude image in the step S21, it is possible to suppress the appearance of linear noises in the two-dimensional phase image obtained in the step S23.

In the above description, the case in which the two-dimensional phase image is generated by performing integration or deconvolution of the phase differential image in the step S23 is described. However, the phase differential image may also be treated as the two-dimensional phase image. In this case, the three-dimensional refractive index distribution of the observation object can be obtained from the phase differential image (two-dimensional phase image) generated in the step S22 by using a kernel (FIG. A11) including a kernel used in deconvolution of the step S23, in deconvolution of the refractive index distribution calculation step S67, without performing the step S23. The kernel shown in FIG. A11 is obtained by convolution integration of the kernel shown in FIG. A06 and the kernel used in deconvolution of the step S23.

FIG. A12 is a flowchart of the two-dimensional phase image generation step S4 in the refractive index distribution measuring method A3. In the refractive index distribution measuring method A3, for each of the plurality of positions, the two-dimensional phase image generation step S4, in a step S31, divides the complex amplitude image of each of the plurality of light irradiation directions into a plurality of batches, corrects the phase of the complex amplitude image included in the batch based on the light irradiation direction for each of the plurality of batches, and then generates the complex amplitude summation image representing the summation of the complex amplitude images after the correction, in a step S32, generates the complex differential interference image of each of the plurality of batches based on the complex amplitude summation image of each of the plurality of batches, in a step S33, generates the phase differential image based on the summation of the complex differential interference images of the plurality of batches, and in a step S34, generates the two-dimensional phase image based on the phase differential image.

The processing of the step S31 in the refractive index distribution measuring method A3 corresponds to dividing the complex amplitude image of each of the plurality of light irradiation directions into the plurality of batches, and then performing the processing of the step S11 in the refractive index distribution measuring method A1 for each of the plurality of batches. The processing of the steps S32 and S33 in the refractive index distribution measuring method A3 corresponds to performing the processing of the steps S21 and S22 in the refractive index distribution measuring method A2 for each of the plurality of batches. The processing of the step S34 in the refractive index distribution measuring method A3 corresponds to performing the processing of the step S23 in the refractive index distribution measuring method A2.

In addition, in the step S32, the complex differential interference image may be generated for each of the plurality of shear directions different from each other on the complex amplitude image. In this case, the two-dimensional phase image generation step S4, in the step S32, generates the complex differential interference image of each of the plurality of batches for each of the plurality of shear directions on the image different from each other based on the complex amplitude summation image of each of the plurality of batches, in the step S33, generates the phase differential image based on the summation of the complex differential interference images of the plurality of batches for each of the plurality of shear directions, and in the step S34, generates the two-dimensional phase image based on the phase differential image of each of the plurality of shear directions.

The suppression of the speckles in the refractive index distribution measuring method A3 is comparable with the refractive index distribution measuring method A1 and the refractive index distribution measuring method A2. The improvement of the SMR in the refractive index distribution measuring method A3 is an intermediate degree between the refractive index distribution measuring method A1 and the refractive index distribution measuring method A2.

In the above description also, the case in which the two-dimensional phase image is generated by performing integration or deconvolution of the phase differential image in the step S34 is described. However, the phase differential image may also be treated as the two-dimensional phase image. In this case, the three-dimensional refractive index distribution of the observation object can be obtained from the phase differential image (two-dimensional phase image) generated in the step S33 by using the kernel including the kernel used in deconvolution of the step S34, in deconvolution of the refractive index distribution calculation step S6, without performing the step S34.

Next, the refractive index distribution measuring method B will be will be described. FIG. B01 to B03 are diagrams showing respective configurations of observation apparatuses 1D to 1F that can be used when measuring the refractive index distribution by the refractive index distribution measuring method B. The observation apparatus 1D illustrated in FIG. B01, as compared with the configuration of the observation apparatus 1A illustrated in FIG. A01, has the common configuration for the optical system from the light source 11 to the imaging unit 43, and further, is different in that an analysis unit 60 is provided instead of the analysis unit 50. The observation apparatus 1E illustrated in FIG. B02, as compared with the configuration of the observation apparatus 1B illustrated in FIG. A02, has the common configuration for the optical system from the light source 11 to the imaging unit 43, and further, is different in that the analysis unit 60 is provided instead of the analysis unit 50. The observation apparatus 1F illustrated in FIG. B03, as compared with the configuration of the observation apparatus 1C illustrated in FIG. A03, has the common configuration for the optical system from the light source 11 to the imaging unit 43, and further, is different in that the analysis unit 60 is provided instead of the analysis unit 50.

The analysis unit 60 is electrically connected to the imaging unit 43, and inputs the interference intensity image output from the imaging unit 43. The analysis unit 60 calculates a three-dimensional refractive index distribution of the observation object S by processing the input interference intensity image. The analysis unit 60 may be a computer. The analysis unit 60 includes an interference intensity image acquisition unit 61, a first complex amplitude image generation unit 62, a second complex amplitude image generation unit 63, a phase conjugate operation unit 64, a two-dimensional phase image generation unit 65, a three-dimensional phase image generation unit 66, a refractive index distribution calculation unit 67, a display unit 68, and a storage unit 69.

The interference intensity image acquisition unit 61 irradiates the observation object S with the light along each of a plurality of light irradiation directions by changing the orientation of the reflection surface of the mirror 22. Further, the interference intensity image acquisition unit 61 acquires the interference intensity image at a reference position for each of the plurality of light irradiation directions from the imaging unit 43. The interference intensity image acquisition unit 61 includes a CPU, has an output port for outputting a control signal for changing the orientation of the reflection surface of the mirror 22, and has an input port for inputting the interference intensity image from the imaging unit 43. It is not necessary to move the objective lens 25 in an optical axis direction. The reference position is an image plane position having a conjugate relationship with respect to the imaging plane of the imaging unit 43.

The first complex amplitude image generation unit 62, the second complex amplitude image generation unit 63, the phase conjugate operation unit 64, the two-dimensional phase image generation unit 65, the three-dimensional phase image generation unit 66, and the refractive index distribution calculation unit 67 perform processing based on the interference intensity images, and include a processing device such as a CPU, a GPU, a DSP, or an FPGA. The display unit 68 displays an image to be processed, an image in the middle of the processing, an image after the processing, and the like, and includes, for example, a liquid crystal display. The storage unit 69 stores data of various images, and includes a hard disk drive, a flash memory, a RAM, a ROM, and the like. The first complex amplitude image generation unit 62, the second complex amplitude image generation unit 63, the phase conjugate operation unit 64, the two-dimensional phase image generation unit 65, the three-dimensional phase image generation unit 66, the refractive index distribution calculation unit 67, and the storage unit 68 may be constituted by a cloud computing.

The storage unit 69 also stores a program for causing the interference intensity image acquisition unit 61, the first complex amplitude image generation unit 62, the second complex amplitude image generation unit 63, the phase conjugate operation unit 64, the two-dimensional phase image generation unit 65, the three-dimensional phase image generation unit 66, and the refractive index distribution calculation unit 67, to execute respective steps of the processing. The program may be stored in the storage unit 69 at the time of manufacture or shipment of the observation apparatus 1D to 1F, may be acquired via a communication line after shipment and then stored in the storage unit 69, or may be recorded in a computer readable recording medium 2 and then stored in the storage unit 69. The recording medium 2 may be an arbitrary medium such as a flexible disk, a CD-ROM, a DVD-ROM, a BD-ROM, a USB memory, or the like.

The details of the processing step of each of the interference intensity image acquisition unit 61, the first complex amplitude image generation unit 62, the second complex amplitude image generation unit 63, the phase conjugate operation unit 64, the two-dimensional phase image generation unit 65, the three-dimensional phase image generation unit 66, and the refractive index distribution calculation unit 67 will be described later.

FIG. B04 is a flowchart of the refractive index distribution measuring method B. The refractive index distribution measuring method B can be applied to each of the observation apparatus 1D to 1F. The refractive index distribution measuring method B includes an interference intensity image acquisition step S61, a first complex amplitude image generation step S62, a second complex amplitude image generation step S63, a phase conjugate operation step S64, a two-dimensional phase image generation step S65, a three-dimensional phase image generation step S66, and a refractive index distribution calculation step S67.

The processing step of the interference intensity image acquisition step S61 is performed by the interference intensity image acquisition unit 61. The processing step of the first complex amplitude image generation step S62 is performed by the first complex amplitude image generation unit 62. The processing step of the second complex amplitude image generation step S63 is performed by the second complex amplitude image generation unit 63. The processing step of the phase conjugate operation step S64 is performed by the phase conjugate operation unit 64. The processing step of the two-dimensional phase image generation step S65 is performed by the two-dimensional phase image generation unit 65. The processing step of the three-dimensional phase image generation step S66 is performed by the three-dimensional phase image generation unit 66. The processing step of the refractive index distribution calculation step S67 is performed by the refractive index distribution calculation unit 67.

In the interference intensity image acquisition step S61, the interference intensity image acquisition unit 61 irradiates the observation object S with the light along each of the plurality of light irradiation directions by changing the orientation of the reflection surface of the mirror 22. Further, the interference intensity image acquisition unit 61 acquires the interference intensity image at the reference position for each of the plurality of light irradiation directions from the imaging unit 43.

In the first complex amplitude image generation step S62, the first complex amplitude image generation unit 62 generates, for each of the plurality of light irradiation directions, a complex amplitude image based on the interference intensity image of the reference position acquired by the interference intensity image acquisition unit 61. In the case of the observation apparatus 1D (FIG. B01) or the observation apparatus 1F (FIG. B03), the first complex amplitude image generation unit 62 can generate the complex amplitude image based on one interference intensity image by a Fourier fringe analysis method. In the case of the observation apparatus 1E (FIG. B02), the first complex amplitude image generation unit 62 can generate the complex amplitude image based on three or more interference intensity images having different optical path differences (phase differences) between the object light and the reference light by a phase shift method.

In the second complex amplitude image generation step S63, the second complex amplitude image generation unit 63 generates, for each of the plurality of light irradiation directions, a complex amplitude image at each of a plurality of z direction positions based on the complex amplitude image at the reference position ($z = 0$) generated by the first complex amplitude image generation unit 62.

The interference intensity image acquisition step S61, the first complex amplitude image generation step S62, and the second complex amplitude image generation step S63 in the refractive index distribution measuring method B respectively perform the same processing steps as the interference intensity image acquisition step S1, the first complex amplitude image generation step S2, and the second complex amplitude image generation step S3 in the refractive index distribution measuring method A.

The phase conjugate operation step S64 is performed after the processing step of the second complex amplitude image generation step S63. The phase conjugate operation step S64 may be performed before the processing step of the second complex amplitude image generation step S63 (which will be described later). Further, when the second complex amplitude image generation step S63 generates the complex amplitude image at a certain z position through a plurality of stages from the complex amplitude image at the reference position, the phase conjugate operation step S64 may be performed between a certain stage and a next stage in the plurality of stages (which will be described later). In the phase conjugate operation step S64, the phase conjugate operation unit 64 performs a phase conjugate operation on the complex amplitude image of each of the plurality of light irradiation directions to generate a complex amplitude image of each of the plurality of light irradiation directions when the relationship between the light irradiation and the imaging for the observation object is reversed.

In addition, the phase conjugate operation is an operation for the complex amplitude image based on a phase conjugate method, and is an operation of calculating a transmission matrix representing the relationship between the light irradiation and the light output for the object, and including an inverse matrix calculation thereof and coordinate conversion. The phase conjugate method may be referred to as a phase conjugation, a time reversal method, a time reversal, a digital phase conjugation, a digital phase conjugate method, or the like. The details will be described later.

In the two-dimensional phase image generation step S65, the two-dimensional phase image generation unit 65 generates, for each of the plurality of positions, a two-dimensional phase image based on the complex amplitude image of each of the plurality of light irradiation directions generated by the second complex amplitude image generation unit 63 or the phase conjugate operation unit 64. The two-dimensional phase image generated in this step corresponds to a phase image centered on the focused z direction position.

In the two-dimensional phase image generation step S65, when a phase image generated based on the complex amplitude image before performing the processing step of the phase conjugate operation step S64 is set as a first phase image, and a phase image generated based on the complex amplitude image obtained by performing the processing step of the phase conjugate operation step S64 is set as a second phase image, for the plurality of positions, the two-dimensional phase image is generated mainly based on the first phase image at a position relatively close to the imaging unit, and the two-dimensional phase image is generated mainly based on the second phase image at a position relatively far from the imaging unit.

In addition, the phase conjugate operation unit 64 and the subsequent processing steps may be performed after all the complex amplitude images at the plurality of positions are generated for each of the plurality of light irradiation directions in the second complex amplitude image generation step S63. Further, processes of generating the complex amplitude image at one certain z direction position for each of the plurality of light irradiation directions in the second complex amplitude image generation step S63 and generating the two-dimensional phase image at the position in the two-dimensional phase image generation step S65 may be set as a unit, and the unit process may be repeatedly performed while scanning the z direction position. The latter case is preferable in that a capacity of image data to be stored in the storage unit 69 can be reduced.

In the three-dimensional phase image generation step S66, the three-dimensional phase image generation unit 66 generates a three-dimensional phase image based on the two-dimensional phase image at each of the plurality of positions generated by the two-dimensional phase image generation unit 65. The three-dimensional phase image generated in this step is an image in which the positions x and y in the two-dimensional phase image and the position z of the two-dimensional phase image are variables.

In the refractive index distribution calculation step S67, the refractive index distribution calculation unit 67 obtains a three-dimensional refractive index distribution of the observation object by deconvolution based on the three-dimensional phase image generated by the three-dimensional phase image generation unit 66.

The two-dimensional phase image generation step S65, the three-dimensional phase image generation step S66, and the refractive index distribution calculation step S67 in the refractive index distribution measuring method B respectively perform the same processing steps as the two-dimensional phase image generation step S4, the three-dimensional phase image generation step S5, and the refractive index distribution calculation step S6 in the refractive index distribution measuring method A.

FIG. B05 is a diagram illustrating the images and the order of the respective processing steps of the second complex amplitude image generation step S63 and the two-dimensional phase image generation step S65. This diagram illustrates a configuration in which the processing step of the phase conjugate operation step S64 is not performed. In this configuration, in the second complex amplitude image generation step S63, for each of the plurality of light irradiation directions, the complex amplitude image at each of the plurality of z direction positions ($z = z_1, z_2, z_3$ in this diagram) is generated based on the complex amplitude image at the reference position ($z = 0$) generated in the first complex amplitude image generation step S62 by the above Formulas (1) and (2) of the formulas of the free propagation. Further, in the two-dimensional phase image generation step S65, for each of the plurality of positions, the complex differential interference image is generated based on the complex amplitude image of each of the plurality of light irradiation directions generated in the second complex amplitude image generation step S63, and in addition, the phase differential image is generated.

Each of FIG. B06 to FIG. B08 is a diagram illustrating the images and the order of the respective processing steps of the second complex amplitude image generation step S63, the phase conjugate operation step S64, and the two-dimensional phase image generation step S65. Each of these diagrams illustrates a configuration in which the processing step of the phase conjugate operation step S64 is performed before, during, or after the processing step of the second complex amplitude image generation step S63.

A first configuration illustrated in FIG. B06 corresponds to the flowchart illustrated in FIG. B04. In the first configuration, the phase conjugate operation step S64 is performed after the processing step of the second complex amplitude image generation step S63. In the second complex amplitude image generation step S63, for each of the plurality of light irradiation directions, the complex amplitude image at each of the plurality of $z$ direction positions ($z = z_1, z_2, z_3$ in this diagram) is generated based on the complex amplitude image at the reference position ($z = 0$) generated in the first complex amplitude image generation step S62 by the above Formulas (1) and (2) of the formulas of the free propagation.

In the first configuration, subsequently, in the phase conjugate operation step S64, for each of the plurality of positions, the phase conjugate operation is performed on the complex amplitude image of each of the plurality of light irradiation directions, and the complex amplitude image of each of the plurality of light irradiation directions in the case in which the relationship between the light irradiation and the imaging for the observation object is reversed is generated. Further, in the two-dimensional phase image generation step S65, for each of the plurality of positions, the complex differential interference image is generated based on the complex amplitude image of each of the plurality of light irradiation directions generated in the phase conjugate operation step S64, and in addition, the phase differential image is generated.

In a second configuration illustrated in FIG. B07, the phase conjugate operation step S64 is performed before the processing step of the second complex amplitude image generation step S63. In the phase conjugate operation step S64, for each of the plurality of light irradiation directions, the phase conjugate operation is performed on the complex amplitude image at the reference position ($z = 0$) generated in the first complex amplitude image generation step S62, and the complex amplitude image of each of the plurality of light irradiation directions in the case in which the relationship between the light irradiation and the imaging for the observation object is reversed is generated.

In the second configuration, subsequently, in the second complex amplitude image generation step S63, for each of the plurality of light irradiation directions, the complex amplitude image at each of the plurality of $z$ direction positions ($z = z_1, z_2, z_3$ in this diagram) is generated based on the complex amplitude image at the reference position ($z = 0$) generated in the phase conjugate operation step S64 by the above Formulas (1) and (2) of the formulas of the free propagation. Further, in the two-dimensional phase image generation step S65, for each of the plurality of positions, the complex differential interference image is generated based on the complex amplitude image of each of the plurality of light irradiation directions generated in the second complex amplitude image generation step S63, and in addition, the phase differential image is generated.

In a third configuration illustrated in FIG. B08, in the case in which the second complex amplitude image generation step S63 generates the complex amplitude image at each of the plurality of positions from the complex amplitude image at the reference position through two stages, the phase conjugate operation step S64 is performed between a first stage and a second stage in the two stages.

In the third configuration, in the first stage of the second complex amplitude image generation step S63, for each of the plurality of light irradiation directions, the complex amplitude image at each of the plurality of $z$ direction positions ($z = z_1, z_3, z_5$ in this diagram) is generated based on the complex amplitude image at the reference position ($z = 0$) generated in the first complex amplitude image generation step S62 by the above Formulas (1) and (2) of the formulas of the free propagation. Subsequently, in the phase conjugate operation step S64, the phase conjugate operation is performed on the complex amplitude image of each of the plurality of light irradiation directions, and the complex amplitude image of each of the plurality of light irradiation directions in the case in which the relationship between the light irradiation and the imaging for the observation object is reversed is generated.

In the third configuration, further subsequently, in the second stage of the second complex amplitude image generation step S63, for each of the plurality of light irradiation directions, the complex amplitude image at each of the $z$ direction positions ($z = z_2, z_4, z_6$) is generated based on the complex amplitude images at the $z$ direction positions ($z = z_1, z_3, z_5$) generated in the phase conjugate operation step S64 by the above Formulas (1) and (2) of the formulas of the free propagation. Further, in the two-dimensional phase image generation step S65, for each of the plurality of positions, the complex differential interference image is generated based on the complex amplitude image of each of the plurality of light irradiation directions generated in the second complex amplitude image generation step S63, and in addition, the phase differential image is generated.

In the first configuration, the second configuration, and the third configuration described above, the number of times of the phase conjugate operation on the complex amplitude image in the phase conjugate operation step S64 is different. The overall processing time of the phase conjugate operation step S64 is shorter in the third configuration than in the first configuration, and is even shorter in the second configuration.

FIG. B09 is a diagram illustrating the images and the order of the respective processing steps of the three-dimensional phase image generation step S66 and the refractive index distribution calculation step S67. In the three-dimensional phase image generation step S66, the three-dimensional phase image is generated based on the two-dimensional phase image of each of the plurality of positions generated in the two-dimensional phase image generation step S65. In this case, for the position which is relatively close to the imaging unit, the two-dimensional phase image generated based on the complex amplitude image before performing the processing step of the phase conjugate operation step S64 (the two-dimensional phase image generated in the configuration illustrated in FIG. B05) is mainly used. On the other hand, for the position which is relatively far from the imaging unit, the two-dimensional phase image generated based on the complex amplitude image after performing the processing step of the phase conjugate operation step S64 (the two-dimensional phase image generated in any one of the configurations illustrated in FIG. B06 to FIG. B08) is mainly used. Subsequently, in the refractive index distribution calculation step S67, the three-dimensional refractive index distribution of the observation object is obtained by deconvolution based on the three-dimensional phase image generated in the three-dimensional phase image generation step S66. Each refractive index distribution data constituting the three-dimensional refractive index distribution (for example, a two-dimensional refractive index distribution data constituting the three-dimensional refractive index distribution in FIG. B09) can be a refractive index cross sectional data.

The generation of the two-dimensional phase image at each position in the z direction includes the following three configurations. The phase image generated based on the complex amplitude image before performing the processing step of the phase conjugate operation step S64 (the phase image generated in the configuration illustrated in FIG. B05) is set as the first phase image $\phi_1$. The phase image generated based on the complex amplitude image after performing the processing step of the phase conjugate operation step S64 (the phase image generated in any one of the configurations illustrated in FIG. B06 to FIG. B08) is set as the second phase image $\phi_2$. A weight function $\alpha$ having a differential coefficient of 0 or less with respect to the variable z representing the distance from the imaging unit along the light propagation path is used. The value of the weight function is 0 or more and 1 or less.

In the first configuration, it is assumed that the weight function $\alpha$ has a positive value (for example, 1) in a range in which z is threshold value $z_{th}$ or less, and has a value of 0 in a range other than the above range. That is, the two-dimensional phase image is represented by the following Formula (8).

[Formula 8]

$$\phi(x, y, z) = \begin{cases} \phi_1(x, y, z) & z \leq z_{th} \\ \phi_2(x, y, z) & z > z_{th} \end{cases} \tag{8}$$

In the second configuration, it is assumed that the weight function $\alpha$ is a function having a value which continuously changes in at least a partial range in the z direction. That is, the two-dimensional phase image is represented by the following Formula (9).

[Formula 9]

$$\phi(x, y, z) = \alpha(z) \cdot \phi_1(x, y, z) + \left[1 - \alpha(z)\right] \phi_2(x, y, z) \tag{9}$$

In the third configuration, it is assumed that the weight function $\alpha$ has a value according to the position (x, y) on the plane perpendicular to the optical axis (the z direction). That is, the two-dimensional phase image is represented by the following Formula (10).

[Formula 10]

$$\phi(x, y, z) = \alpha(x, y, z) \cdot \phi_1(x, y, z) + \left[1 - \alpha(x, y, z)\right] \phi_2(x, y, z) \tag{10}$$

Next, the contents of the phase conjugate operation by the phase conjugate operation step S64 will be described with reference to FIG. B10 and FIG. B11.

FIG. B10 is a diagram illustrating input light $U_{in}(k_{in})$ and output light $u_{out}(r_{out})$ when the interference intensity image is imaged by the imaging unit. $U_{in}(k_{in})$ represents a complex amplitude of a wavenumber $k_{in}$ of the light with which the observation object is irradiated. $u_{out}(r_{out})$ represents a complex amplitude of a position $r_{out}$ of the light output from the observation object. The relationship between $U_{in}(k_{in})$ and $u_{out}(r_{out})$ is represented by the following Formula (11). An n-th element $U_{in}(k_{in}^n)$ of a column vector $U_{in}$ represents a complex amplitude of a plane wave of a wavenumber of $k_{in}^n$. An n-th element $u_{out}(r_{out}^n)$ of a column vector $u_{out}$ represents a complex amplitude of the light observed at a position $r_{out}^n$. A matrix

$T(r_{out}, k_{in})$ of N rows and N columns represents a linear relationship between $U_{in}(k_{in})$ and $u_{out}(r_{out})$, and is referred to as a transmission matrix. A scattering process of the light in the observation object can be represented by the transmission matrix described above. An element $T_{n1,n2}$ of an n1-th row and an n2-th column of the matrix $T(r_{out}, k_{in})$ represent a complex amplitude of the light observed at a position $r_{out}^{n1}$ when the plane wave having a wavenumber of $k_{in}^{n2}$ and an amplitude of 1 is input.

[Formula 11]

$$
\begin{pmatrix} u_{out}(\mathbf{r}_{out}^1) \\ \vdots \\ u_{out}(\mathbf{r}_{out}^N) \end{pmatrix} = \begin{pmatrix} T(\mathbf{r}_{out}^1, \mathbf{k}_{in}^1) & \cdots & T(\mathbf{r}_{out}^1, \mathbf{k}_{in}^N) \\ \vdots & \ddots & \vdots \\ T(\mathbf{r}_{out}^N, \mathbf{k}_{in}^1) & \cdots & T(\mathbf{r}_{out}^N, \mathbf{k}_{in}^N) \end{pmatrix} \begin{pmatrix} U_{in}(\mathbf{k}_{in}^1) \\ \vdots \\ U_{in}(\mathbf{k}_{in}^N) \end{pmatrix}
$$

$$
= \begin{pmatrix} T_{1,1} & \cdots & T_{1,N} \\ \vdots & \ddots & \vdots \\ T_{N,1} & \cdots & T_{N,N} \end{pmatrix} \begin{pmatrix} U_{in}(\mathbf{k}_{in}^1) \\ \vdots \\ U_{in}(\mathbf{k}_{in}^N) \end{pmatrix}
$$

$$(1\ 1)$$

FIG. B11 is a diagram illustrating input light $U_{out}(k_{out})$ and output light $u_{in}(r_{in})$ in the case in which the relationship between the light irradiation and the imaging is reversed. In this case, $U_{out}(k_{out})$ represents a complex amplitude of a wavenumber $k_{out}$ of the light with which the observation object is irradiated. $u_{in}(r_{in})$ represents a complex amplitude of a position $r_{in}$ of the light output from the observation object. The relationship between $U_{out}(k_{out})$ and $u_{in}(r_{in})$ is represented by the following Formula (12). An n-th element $U_{out}(k_{out}^n)$ of a column vector $U_{out}$ represents a complex amplitude of a plane wave of a wavenumber of $k_{out}^n$. An n-th element $u_{in}(r_{in}^n)$ of a column vector $u_{in}$ represents a complex amplitude of the light observed at a position $r_{in}^n$. A matrix $S(r_{in}, k_{out})$ of N rows and N columns represents a linear relationship between $U_{out}(k_{out})$ and $u_{in}(r_{in})$, and is a transmission matrix in the case in which the relationship between the light irradiation and the imaging is reversed.

[Formula 12]

$$
\begin{pmatrix} u_{in}(\mathbf{r}_{in}^1) \\ \vdots \\ u_{in}(\mathbf{r}_{in}^N) \end{pmatrix} = \begin{pmatrix} S(\mathbf{r}_{in}^1, \mathbf{k}_{out}^1) & \cdots & S(\mathbf{r}_{in}^1, \mathbf{k}_{out}^N) \\ \vdots & \ddots & \vdots \\ S(\mathbf{r}_{in}^N, \mathbf{k}_{out}^1) & \cdots & S(\mathbf{r}_{in}^N, \mathbf{k}_{out}^N) \end{pmatrix} \begin{pmatrix} U_{out}(\mathbf{k}_{out}^1) \\ \vdots \\ U_{out}(\mathbf{k}_{out}^N) \end{pmatrix}
$$

$$
= \begin{pmatrix} S_{1,1} & \cdots & S_{1,N} \\ \vdots & \ddots & \vdots \\ S_{N,1} & \cdots & S_{N,N} \end{pmatrix} \begin{pmatrix} U_{out}(\mathbf{k}_{out}^1) \\ \vdots \\ U_{out}(\mathbf{k}_{out}^N) \end{pmatrix}
$$

$$(1\ 2)$$

$U_{in}(k_{in})$ is represented by the Fourier transform of $u_{in}(r_{in})$ as shown in the following Formula (13). $U_{out}(k_{out})$ is represented by the Fourier transform of $u_{out}(r_{out})$ as shown in the following Formula (14). When Formulas (11) to (14) are used, the transmission matrix $S(r_{in}, k_{out})$ in the case in which the relationship between the light irradiation and the imaging is reversed is represented by the following Formula (15) by using a matrix representing the inverse Fourier transform and the transmission matrix $T(r_{out}, k_{in})$.

[Formula 13]

$$
\begin{pmatrix} U_{in}(\mathbf{k}_{in}^1) \\ \vdots \\ U_{in}(\mathbf{k}_{in}^N) \end{pmatrix} = \begin{pmatrix} F_{1,1} & \cdots & F_{1,N} \\ \vdots & \ddots & \vdots \\ F_{N,1} & \cdots & F_{N,N} \end{pmatrix} \begin{pmatrix} u_{in}(\mathbf{r}_{in}^1) \\ \vdots \\ u_{in}(\mathbf{r}_{in}^N) \end{pmatrix}
$$

$$(1\ 3)$$

[Formula 14]

$$\begin{pmatrix} U_{out}(\mathbf{k}_{out}^1) \\ \vdots \\ U_{out}(\mathbf{k}_{out}^N) \end{pmatrix} = \begin{pmatrix} F_{1,1} & \cdots & F_{1,N} \\ \vdots & \ddots & \vdots \\ F_{N,1} & \cdots & F_{N,N} \end{pmatrix} \begin{pmatrix} u_{out}(\mathbf{r}_{out}^1) \\ \vdots \\ u_{out}(\mathbf{r}_{out}^N) \end{pmatrix} \tag{14}$$

[Formula 15]

$$\begin{pmatrix} S_{1,1} & \cdots & S_{1,N} \\ \vdots & \ddots & \vdots \\ S_{N,1} & \cdots & S_{N,N} \end{pmatrix} = \begin{pmatrix} F_{1,1} & \cdots & F_{1,N} \\ \vdots & \ddots & \vdots \\ F_{N,1} & \cdots & F_{N,N} \end{pmatrix}^{-1} \begin{pmatrix} T_{1,1} & \cdots & T_{1,N} \\ \vdots & \ddots & \vdots \\ T_{N,1} & \cdots & T_{N,N} \end{pmatrix}^{-1} \begin{pmatrix} F_{1,1} & \cdots & F_{1,N} \\ \vdots & \ddots & \vdots \\ F_{N,1} & \cdots & F_{N,N} \end{pmatrix}^{-1} \tag{15}$$

In the phase conjugate operation step S64, first, the transmission matrix $T(r_{out}, k_{in})$ when the interference intensity image is imaged by the imaging unit is obtained based on the complex amplitude image. Next, based on the above transmission matrix $T(r_{out}, k_{in})$ and the above Formula (15), the transmission matrix $S(r_{in}, k_{out})$ in the case in which the relationship between the light irradiation and the imaging is reversed is obtained. Further, based on the above transmission matrix $S(r_{in}, k_{out})$, the complex amplitude image in the case in which the relationship between the light irradiation and the imaging is reversed is obtained.

The vector $U_{in}^n(k_{in})$ of the input light of the n-th light irradiation direction when the interference intensity image is imaged by the imaging unit for each of the plurality of light irradiation directions is represented by the following Formula (16), in which only the value of the n-th element is 1 and the values of the other elements are 0. For the above input light $U_{in}^n(k_{in})$, the output light $u_{out}^n(r_{out})$ is represented by the following Formula (17). The Formula (17) corresponds to the complex amplitude obtained for the n-th light irradiation direction.

[Formula 16]

$$U_{in}^n(\mathbf{k}_{in}) = \begin{pmatrix} U_{in}^n(\mathbf{k}_{in}^1) \\ \vdots \\ U_{in}^n(\mathbf{k}_{in}^N) \end{pmatrix} = \begin{pmatrix} 0 \\ \vdots \\ 0 \\ 1 \\ 0 \\ \vdots \\ 0 \end{pmatrix} \tag{16}$$

[Formula 17]

$$u_{out}^n(\mathbf{r}_{out}) = \begin{pmatrix} u_{out}^n(\mathbf{r}_{out}^1) \\ \vdots \\ u_{out}^n(\mathbf{r}_{out}^N) \end{pmatrix} \tag{17}$$

From the Formula (16) and the above Formula (11), the following Formula (18) is obtained. Further, the following Formula (19) is obtained by similarly obtaining for each of the plurality of light irradiation directions. In this way, the transmission matrix $T(r_{out}, k_{in})$ can be obtained. In addition, from the Formula (19) and the above Formula (15), the transmission matrix $S(r_{in}, k_{out})$ in the case in which the relationship between the light irradiation and the imaging is reversed can be obtained.

[Formula 18]

$$\begin{pmatrix} u_{out}^n(\mathbf{r}_{out}^1) \\ \vdots \\ u_{out}^n(\mathbf{r}_{out}^N) \end{pmatrix} = \begin{pmatrix} T_{1,1} & \cdots & T_{1,N} \\ \vdots & \ddots & \vdots \\ T_{N,1} & \cdots & T_{N,N} \end{pmatrix} \begin{pmatrix} U_{in}(\mathbf{k}_{in}^1) \\ \vdots \\ U_{in}(\mathbf{k}_{in}^N) \end{pmatrix} = \begin{pmatrix} T_{1,n} \\ \vdots \\ T_{N,n} \end{pmatrix} \tag{18}$$

[Formula 19]

$$\begin{pmatrix} T_{1,1} & \cdots & T_{1,N} \\ \vdots & \ddots & \vdots \\ T_{N,1} & \cdots & T_{N,N} \end{pmatrix} = \begin{pmatrix} u_{out}^1(\mathbf{r}_{out}^1) & \cdots & u_{out}^N(\mathbf{r}_{out}^1) \\ \vdots & \ddots & \vdots \\ u_{out}^1(\mathbf{r}_{out}^N) & \cdots & u_{out}^N(\mathbf{r}_{out}^N) \end{pmatrix} \qquad (19)$$

The input light $U_{out}^n(k_{out})$ of the n-th light irradiation direction out of the plurality of light irradiation directions in the case in which the relationship between the light irradiation and the imaging is reversed is represented by the following Formula (20), in which only the value of the n-th element is 1 and the values of the other elements are 0. From this Formula, the output light $u_{in}^n(r_{in})$ for the input light $U_{out}^n(k_{out})$ is represented by the following Formula (21). The Formula (21) represents the complex amplitude when the relationship between the light irradiation and the imaging is reversed. In this way, the complex amplitude image in the case in which the relationship between the light irradiation and the imaging is reversed can be obtained.

[Formula 20]

$$U_{out}^n(\mathbf{k}_{out}) = \begin{pmatrix} U_{out}^n(\mathbf{k}_{out}^1) \\ \vdots \\ U_{out}^n(\mathbf{k}_{out}^N) \end{pmatrix} = \begin{pmatrix} 0 \\ \vdots \\ 0 \\ 1 \\ 0 \\ \vdots \\ 0 \end{pmatrix} \qquad (20)$$

[Formula 21]

$$\begin{pmatrix} u_{in}^n(\mathbf{r}_{in}^1) \\ \vdots \\ u_{in}^n(\mathbf{r}_{in}^N) \end{pmatrix} = \begin{pmatrix} S_{1,1} & \cdots & S_{1,N} \\ \vdots & \ddots & \vdots \\ S_{N,1} & \cdots & S_{N,N} \end{pmatrix} \begin{pmatrix} U_{out}(\mathbf{k}_{out}^1) \\ \vdots \\ U_{out}(\mathbf{k}_{out}^N) \end{pmatrix} = \begin{pmatrix} S_{1,n} \\ \vdots \\ S_{N,n} \end{pmatrix} \qquad (21)$$

When the transmission matrix $S(r_{in}, k_{out})$ in the case in which the relationship between the light irradiation and the imaging is reversed is obtained, it is necessary to calculate the inverse matrix of the transmission matrix $T(r_{out}, k_{in})$ as shown in the above Formula (15). Therefore, the transmission matrix T needs to be a square matrix in which the number of row elements and the number of column elements are equal to each other. That is, a matrix dimension in a light irradiation side wavenumber space for the observation object in the interference intensity image acquisition step S61 and the number of pixels of the complex amplitude image need to be equal to each other.

In order to make them equal to each other, the matrix dimension in the light irradiation side wavenumber space for the observation object in the interference intensity image acquisition step S61 may be made equal to the number of pixels, or only a partial range of the image acquired by the imaging unit may be used in the subsequent processing steps. However, in general, the number of pixels of the image acquired by the imaging unit is, for example, $1024 \times 1024$, and thus, it is not easy to make the matrix dimension in the light irradiation side wavenumber space for the observation object equal to the number of pixels. Further, it is not preferable to use only the partial range of the image out of the image acquired by the imaging unit in the subsequent processing steps because this leads to a decrease in resolution.

Therefore, as illustrated in FIG. B12, in the phase conjugate operation step S64, it is preferable to divide the complex amplitude image into a plurality of partial images each having the same number of pixels as the matrix dimension in the light irradiation side wavenumber space for the observation object, perform the phase conjugate operation on each of the plurality of partial images, and then combine the plurality of partial images. In this case, any two or more partial images out of the plurality of partial images may have a common region.

Next, the refractive index distribution measuring method C will be described. FIG. C01 to C03 are diagrams showing respective configurations of observation apparatuses 1G to 1I that can be used when measuring the refractive index distribution by the refractive index distribution measuring method C. The observation apparatus 1G illustrated in FIG. C01, as compared with the configuration of the observation apparatus 1A illustrated in FIG. A01, has the common configuration for the optical system from the light source 11 to the imaging unit 43, and further, is different in that an analysis unit 70 is provided instead of the analysis unit 50. The observation apparatus 1H illustrated in FIG. C02, as compared with the configuration of the observation apparatus 1B illustrated in FIG. A02, has the common configuration for the optical system from the light source 11 to the imaging unit 43, and further, is different in that the analysis unit 70 is provided instead of the analysis unit 50. The observation apparatus 1I illustrated in FIG. C03, as compared with the configuration of the observation apparatus 1C illustrated in FIG. A03, has the common configuration for the optical system from the light source 11 to the imaging unit 43, and further, is different in that the analysis unit 70 is provided instead of the analysis unit 50.

The analysis unit 70 is electrically connected to the imaging unit 43, and inputs the interference intensity image output from the imaging unit 43. The analysis unit 70 calculates a three-dimensional refractive index distribution of the observation object S by processing the input interference intensity image. The analysis unit 70 may be a computer. The analysis unit 70 includes an interference intensity image acquisition unit 71, a first complex amplitude image generation unit 72, a second complex amplitude image generation unit 73, a two-dimensional phase image generation unit 74, a three-dimensional phase image generation unit 75, a refractive index distribution calculation unit 76, a third complex amplitude image generation unit 77, a display unit 78, and a storage unit 79.

The interference intensity image acquisition unit 71 irradiates the observation object S with the light along each of a plurality of light irradiation directions by changing the orientation of the reflection surface of the mirror 22. Further, the interference intensity image acquisition unit 71 acquires the interference intensity image at a reference position for each of the plurality of light irradiation directions from the imaging unit 43. The interference intensity image acquisition unit 71 includes a CPU, has an output port for outputting a control signal for changing the orientation of the reflection surface of the mirror 22, and has an input port for inputting the interference intensity image from the imaging unit 43. It is not necessary to move the objective lens 25 in an optical axis direction. The reference position is an image plane position having a conjugate relationship with respect to the imaging plane of the imaging unit 43.

The first complex amplitude image generation unit 72, the second complex amplitude image generation unit 73, the two-dimensional phase image generation unit 74, the three-dimensional phase image generation unit 75, the refractive index distribution calculation unit 76, and the third complex amplitude image generation unit 77 perform processing based on the interference intensity images, and include a processing device such as a CPU, a GPU, a DSP, or an FPGA. The display unit 78 displays an image to be processed, an image in the middle of the processing, an image after the processing, and the like, and includes, for example, a liquid crystal display. The storage unit 79 stores data of various images, and includes a hard disk drive, a flash memory, a RAM, a ROM, and the like. The first complex amplitude image generation unit 72, the second complex amplitude image generation unit 73, the two-dimensional phase image generation unit 74, the three-dimensional phase image generation unit 75, the refractive index distribution calculation unit 76, the third complex amplitude image generation unit 77, and the storage unit 79 may be constituted by a cloud computing.

The storage unit 79 also stores a program for causing the interference intensity image acquisition unit 71, the first complex amplitude image generation unit 72, the second complex amplitude image generation unit 73, the two-dimensional phase image generation unit 74, the three-dimensional phase image generation unit 75, the refractive index distribution calculation unit 76, and the third complex amplitude image generation unit 77 to execute respective steps of the processing. The program may be stored in the storage unit 79 at the time of manufacture or shipment of the observation apparatus 1G to 1I, may be acquired via a communication line after shipment and then stored in the storage unit 79, or may be recorded in a computer readable recording medium 2 and then stored in the storage unit 79. The recording medium 2 may be an arbitrary medium such as a flexible disk, a CD-ROM, a DVD-ROM, a BD-ROM, a USB memory, or the like.

The details of the processing step of each of the interference intensity image acquisition unit 71, the first complex amplitude image generation unit 72, the second complex amplitude image generation unit 73, the two-dimensional phase image generation unit 74, the three-dimensional phase image generation unit 75, the refractive index distribution calculation unit 76, and the third complex amplitude image generation unit 77 will be described later.

FIG. C04 and FIG. C05 are flowcharts of the refractive index distribution measuring method C. FIG. C05 illustrates a part of the flowchart illustrated in FIG. C04. The refractive index distribution measuring method C can be applied to each of the observation apparatus 1G to 1I. The observation method includes an interference intensity image acquisition step S71, a first complex amplitude image generation step S72, a second complex amplitude image generation step S73, a two-dimensional phase image generation step S74, a three-dimensional phase image generation step S75, a refractive index distribution calculation step S76, and a third complex amplitude image generation step S77.

The processing step of the interference intensity image acquisition step S71 is performed by the interference intensity image acquisition unit 71. The processing step of the first complex amplitude image generation step S72 is performed by the first complex amplitude image generation unit 72. The processing step of the second complex amplitude image generation step S73 is performed by the second complex amplitude image generation unit 73. The processing step of the two-dimensional phase image generation step S74 is performed by the two-dimensional phase image generation unit 74. The processing step of the three-dimensional phase image generation step S75 is performed by the three-dimensional phase image generation unit 75. The processing step of the refractive index distribution calculation step S76 is performed by the refractive index distribution calculation unit 76. The processing step of the third complex amplitude image generation step S77 is performed by the third complex amplitude image generation unit 77.

In the interference intensity image acquisition step S71, the interference intensity image acquisition unit 71 irradiates the observation object S with the light along each of the plurality of light irradiation directions by changing the orientation of the reflection surface of the mirror 22. Further, the interference intensity image acquisition unit 71 acquires the interference intensity image at the reference position for each of the plurality of light irradiation directions from the imaging unit 43.

In the first complex amplitude image generation step S72, the first complex amplitude image generation unit 72 generates, for each of the plurality of light irradiation directions, a complex amplitude image based on the interference intensity image acquired by the interference intensity image acquisition unit 71. In the case of the observation apparatus 1G (FIG. C01) or the observation apparatus 1I (FIG. C03), the first complex amplitude image generation unit 72 can generate the complex amplitude image based on one interference intensity image by a Fourier fringe analysis method. In the case of the observation apparatus 1H (FIG. C02), the first complex amplitude image generation unit 72 can generate the complex amplitude image based on three or more interference intensity images having different optical path differences (phase differences) between the object light and the reference light by a phase shift method. The complex amplitude image generated in the first complex amplitude image generation step S72 may be at the same reference position as the interference intensity image or may be at another position generated based on the complex amplitude image at the reference position.

In the second complex amplitude image generation step S73, the second complex amplitude image generation unit 73 generates, for each of the plurality of light irradiation directions, a complex amplitude image at each of a plurality of z direction positions between a first position and a second position based on the complex amplitude image at the first position with respect to a distance from the imaging unit 43 along a light propagation path.

In the two-dimensional phase image generation step S74, the two-dimensional phase image generation unit 74 generates, for each of the plurality of positions, a two-dimensional phase image based on the complex amplitude image of each of the plurality of light irradiation directions generated by the second complex amplitude image generation unit 73. The two-dimensional phase image generated in this step corresponds to a phase image centered on the focused z direction position.

In the three-dimensional phase image generation step S75, the three-dimensional phase image generation unit 75 generates a three-dimensional phase image between the first position and the second position based on the two-dimensional phase image at each of the plurality of positions generated by the two-dimensional phase image generation unit 74. The three-dimensional phase image generated in this step is an image in which the positions x and y in the two-dimensional phase image and the position z of the two-dimensional phase image are variables.

In the refractive index distribution calculation step S76, the refractive index distribution calculation unit 76 obtains a three-dimensional refractive index distribution of the observation object between the first position and the second position by deconvolution based on the three-dimensional phase image generated by the three-dimensional phase image generation unit 75.

The interference intensity image acquisition step S71, the first complex amplitude image generation step S72, the second complex amplitude image generation step S73, the two-dimensional phase image generation step S74, the three-dimensional phase image generation step S75, and the refractive index distribution calculation step S76 in the refractive index distribution measuring method C respectively perform substantially the same processing steps as the interference intensity image acquisition step S1, the first complex amplitude image generation step S2, the second complex amplitude image generation step S3, the two-dimensional phase image generation step S4, the three-dimensional phase image generation step S5, and the refractive index distribution calculation step S6 in the refractive index distribution measuring method A.

In the third complex amplitude image generation step S77, the third complex amplitude image generation unit 77 generates, for each of the plurality of light irradiation directions, a complex amplitude image at the second position based on the complex amplitude image at the first position used in the second complex amplitude image generation step S73 and the three-dimensional refractive index distribution of the observation object between the first position and the second position calculated in the refractive index distribution calculation step S76.

In the step S83 including the second complex amplitude image generation step S73, the two-dimensional phase image generation step S74, the three-dimensional phase image generation step S75, and the refractive index distribution calculation step S76, the three-dimensional refractive index distribution of the observation object between the first position and the second position is obtained based on the complex amplitude image at the first position with respect to the distance from the imaging unit 43 along the light propagation path. The processing steps of the step S83 and the third complex amplitude image generation step S77 are repeatedly performed. This will be described with reference to FIG. C04 to FIG. C07.

FIG. C06 is a diagram illustrating a relationship between a region including the observation object and first to J-th blocks. As illustrated in this diagram, the region including the observation object is divided into the first to J-th blocks in order based on the distance from the imaging unit along the light propagation path (z direction). In this diagram, it is set to J = 3. The j-th block in the first to J-th blocks is a region from $z = z_{j-1}$ to $z = z_j$. In each j-th block, a position (near end) of $z = z_{j-1}$ closest to the imaging unit is set as the first position, and a position (far end) of $z = z_j$ farthest from the imaging unit is set as the second position.

FIG. C07 is a diagram illustrating a processing procedure for the first to J-th blocks. As illustrated in this diagram, for each j-th block, in the step S83, the complex amplitude image and the two-dimensional phase image at each of the plurality of z direction positions from the first position to the second position are generated based on the complex amplitude image at the first position, the three-dimensional phase image between the first position and the second position is generated, and the three-dimensional refractive index distribution is obtained. For each j-th block, in the third complex amplitude image generation step S77, the complex amplitude image at the second position is generated based on the complex amplitude image at the first position and the three-dimensional refractive index distribution calculated in the step S83.

The complex amplitude image at the second position in the (j-1)-th block generated in the third complex amplitude image generation step S77 is used as the complex amplitude image at the first position in the next j-th block, and the processing steps of the step S83 and the third complex amplitude image generation step S77 are performed for the j-th block. When the three-dimensional refractive index distribution is obtained for each of the first to J-th blocks, the three-dimensional refractive index distribution of the entire observation object is obtained by combining these distributions. The three-dimensional refractive index distribution of each of the first to J blocks (for example, the refractive index distribution of the first block, the refractive index distribution of the second block, and the refractive index distribution of the third block in FIG. C07) can be a refractive index cross sectional data.

As illustrated in FIG. C04 and FIG. C05, in the step S81 after the first complex amplitude image generation step S72, it is set to j = 0, and in the subsequent step S82, the value of j is increased as j = 1, and the processing steps of the step S83 and the third complex amplitude image generation step S77 are performed for the first block. That is, for the first block closest to the imaging unit, based on the complex amplitude image generated in the first complex amplitude image generation step S72, a position of $z = z_0$ (near end) closest to the imaging unit is set as the first position, a position of $z = z_1$ (far end) farthest from the imaging unit is set as the second position, and the respective processing steps of the step S83 (the second complex amplitude image generation step S73, the two-dimensional phase image generation step S74, the three-dimensional phase image generation step S75, and the refractive index distribution calculation step S76) and the third complex amplitude image generation step S77 are sequentially performed. Thereafter, the process returns to the step S82.

For the j-th block (in this case, j is 2 or more and less than J), based on the complex amplitude image generated for the (j-1)-th block in the third complex amplitude image generation step S77, a position of $z = z_{j-1}$ (near end) closest to the imaging unit is set as the first position, a position of $z = z_j$ (far end) farthest from the imaging unit is set as the second position, and the respective processing steps of the step S83 (the second complex amplitude image generation step S73, the two-dimensional phase image generation step S74, the three-dimensional phase image generation step S75, and the refractive index distribution calculation step S76) and the third complex amplitude image generation step S77 are sequentially performed. Thereafter, the process returns to the step S82.

For the J-th block which is the last block farthest from the imaging unit, based on the complex amplitude image generated for the (J-1)-th block in the third complex amplitude image generation step S77, a position of $z = z_{J-1}$ (near end) closest to the imaging unit is set as the first position, a position of $z = z_J$ (far end) farthest from the imaging unit is set as the second position, and the processing step of the step S83 (the second complex amplitude image generation step S73, the two-dimensional phase image generation step S74, the three-dimensional phase image generation step S75, and the refractive index distribution calculation step S76) is performed.

For the J-th block, it is determined to be the last block in the step S84 after the step S83, and may be ended without proceeding to the third complex amplitude image generation step S77. In addition, for the J-th block, it may be determined to be the last block after the three-dimensional phase image generation step S75, and may be ended without proceeding to the refractive index distribution calculation step S76, and in this case, the three-dimensional phase image of the entire observation object is obtained.

In addition, the region including the observation object may be divided into the two blocks in order based on the distance from the imaging unit along the light propagation path (z direction), and in this case, the processing for the first block and the processing for the last J-th block described above may be performed. Further, the region including the observation object may not be divided into the plurality of blocks, and in this case, the respective processing steps of the step S83 (the second complex amplitude image generation step S73, the two-dimensional phase image generation step S74, the three-dimensional phase image generation step S75, and the refractive index distribution calculation step S76) and the third complex amplitude image generation step S77 may be sequentially performed only once.

Next, the details of the third complex amplitude image generation step S77 will be described. When acquiring the interference intensity image by irradiating the observation object with the light, in the j-th block, a light wavefront at the second position ($z = z_j$) propagates inside the j-th block to reach the first position ($z = z_{j-1}$) and further propagates to the imaging unit. Therefore, in the third complex amplitude image generation step S77, the light wavefront at the first position (z

= $z_{j-1}$) is reversely propagated inside the j-th block by numerical calculation in consideration of the refractive index distribution of the j-th block, thereby obtaining the light wavefront at the second position (z = $z_j$). That is, in the third complex amplitude image generation step S77, for each of the plurality of light irradiation directions, the complex amplitude image at the second position (z = $z_j$) of the j-th block is generated based on the complex amplitude image at the first position (z = $z_{j-1}$) of the j-th block and the refractive index distribution of the j-th block. In the above processing, a method of numerically calculating the propagation of the light wavefront in consideration of the refractive index distribution of the medium is used. A beam propagation method (BPM), a split-step non-paraxial (SSNP), and the like are known as the numerical calculation method of the inhomogeneous medium propagation described above. Hereinafter, the processing using the BPM in the third complex amplitude image generation step S77 will be described.

FIG. C08 is a diagram illustrating processing contents of the BPM. This diagram illustrates an arbitrary j-th block. As illustrated in this diagram, the j-th block is divided into M slices (7 slices in this diagram) (first to M-th slices) based on the distance from the imaging unit along the light propagation path (z direction). A thickness of each slice is about a wavelength.

The thickness of each slice may be constant. In this case, it is assumed that the thickness of each slice is a constant value of $\Delta z$. The m-th slice out of the first to M-th slices of the j-th block is from a position ($z_{j-1}$ + (m-1)$\Delta z$) to a position ($z_{j-1}$ + m$\Delta z$). In order from the first position (z = $z_{j-1}$) of the j-th block to the second position (z = $z_j$), a phase change according to the refractive index distribution is sequentially applied in each of the first to M-th slices, and the light wavefront is reversely propagated by $\Delta z$.

In addition, the thickness $\Delta z$ of each slice in the processing of the third complex amplitude image generation step S77 may be different from or may coincide with the position interval when generating the complex amplitude image of each of the plurality of z direction positions from the first position to the second position in the processing of the second complex amplitude image generation step S73.

The phase change o(x, y, z) applied to the light wavefront when reversely propagating the slice of the thickness $\Delta z$ at the position z is represented by the following Formula (22). In the Formula (22), $k_v$ is a wavenumber of the light in vacuum. $\delta n(x, y, z)$ is a difference between the refractive index distribution n(x, y, z) of the observation object at the position z and the refractive index $n_b$ of the background (medium), and is represented by the following Formula (23). Further, cos$\theta$ is represented by the following Formula (24).

[Formula 22]

$$o(x, y, z) = \exp\left(-ik_v \delta n(x, y, z) \frac{\Delta z}{\cos \theta}\right) \qquad (22)$$

[Formula 23]

$$\delta n(x, y, z) = n(x, y, z) - n_b \qquad (23)$$

[Formula 24]

$$\cos\theta = \frac{\sqrt{n_b^2 k_V^2 - k_x^2 - k_y^2}}{n_b k_v} \qquad (24)$$

Assuming that the complex amplitude of the light at the position (z = $z_{j-1}$ + (m-1)$\Delta z$) of the m-th slice is u(x, y, z), the complex amplitude u(x, y, z + $\Delta z$) of the light at the position (z + $\Delta z$) after the light reversely propagates inside the m-th slice is represented by the following Formula (25). In the Formula (25), P($k_x$, $k_y$ ; $\Delta z$) is represented by the following Formula (26). The Formula (25) indicates that the complex amplitude u(x, y, z + $\Delta z$) of the light at the position (z + $\Delta z$) after propagating the slice of the thickness $\Delta z$ is obtained by performing Fourier transform on a product of the complex amplitude u(x, y, z) of the light and the phase change o(x, y, z), and performing inverse Fourier transform on a product of a result of the above Fourier transform and P($k_x$, $k_y$ ; $\Delta z$). $P_{\Delta z}$ is a function for performing calculation of the light propagation of $\Delta z$.

[Formula 25]

$$u\left(x,y,z+\Delta z\right)=P_{\Delta z}\Big[o\left(x,y,z\right)\cdot u\left(x,y,z\right)\Big]$$
$$\equiv F^{-1}\Big[P\left(k_x,k_y;\Delta z\right)\cdot F\Big[o\left(x,y,z\right)\cdot u\left(x,y,z\right)\Big]\Big] \tag{25}$$

[Formula 26]

$$P\left(k_x,k_y;\Delta z\right)=\exp\left(-i\sqrt{n_b^2 k_v^2 - k_x^2 - k_y^2}\,\Delta z\right) \tag{26}$$

The propagation of the light wavefront in each slice of the j-th block is represented by the following Formulas (27) to (29). That is, when the complex amplitude of the light at the first position ($z = z_{j-1}$) of the j-th block is set to $u(x, y, z_{j-1})$, the complex amplitude $u(x, y, z_{j-1} + \Delta z)$ of the light after propagating the first slice of the j-th block is represented by the following Formula (27). When the complex amplitude of the light after propagating the (m-1)-th slice of the j-th block is set to $u(x, y, z_{j-1} + (m-1)\Delta z)$, the complex amplitude $u(x, y, z_{j-1} + m\Delta z)$ of the light after propagating the m-th slice of the j-th block is represented by the following Formula (28). When the complex amplitude of the light after propagating the (M-1)-th slice of the j-th block is set to $u(x, y, z_{j-1} + (M-1)\Delta z)$, the complex amplitude $u(x, y, z_j)$ of the light at the second position ($z = z_j$) after propagating the M-th slice of the j-th block is represented by the following Formula (29).

[Formula 27]

$$u\left(x,y,z_{j-1}+\Delta z\right)=P_{\Delta z}\Big[o\left(x,y,z_{j-1}\right)\cdot u\left(x,y,z_{j-1}\right)\Big] \tag{27}$$

[Formula 28]

$$u\left(x,y,z_{j-1}+m\Delta z\right)=P_{\Delta z}\Big[o\left(x,y,z_{j-1}+(m-1)\Delta z\right)\cdot u\left(x,y,z_{j-1}+(m-1)\Delta z\right)\Big] \tag{28}$$

[Formula 29]

$$u\left(x,y,z_j\right)=P_{\Delta z}\Big[o\left(x,y,z_{j-1}+(M-1)\Delta z\right)\cdot u\left(x,y,z_{j-1}+(M-1)\Delta z\right)\Big] \tag{29}$$

As described above, in the third complex amplitude image generation step S77, the light wavefront at the first position ($z = z_{j-1}$) is sequentially and reversely propagated inside the j-th block for each slice by the numerical calculation in consideration of the refractive index distribution of the j-th block, and thus, the light wavefront at the second position ($z = z_j$) can be obtained.

FIG. C09 is a flowchart of the third complex amplitude image generation step S77. In a step S41, the position z is initialized to the first position ($z = z_{j-1}$) of the j-th block. In a step S42, interaction between the complex amplitude $u(x, y, z)$ of the light at the position z and the phase change $o(x, y, z)$ is obtained. In a step S43, the wavefront of the light after the interaction is propagated by the distance $\Delta z$, and the complex amplitude $u(x, y, z + \Delta z)$ of the light at the position $z + \Delta z$ is obtained. In a step S44, z obtained by adding $\Delta z$ is set as new z. In a step S44, when it is determined that the position z has not yet reached the second position ($z = z_j$) of the j-th block, the process returns to the step S42 to repeat the steps S42 to S44. In the step S44, when it is determined that the position z reaches the second position ($z = z_j$) of the j-th block, the processing of the third complex amplitude image generation step S77 is ended. The complex amplitude of the light acquired at the end becomes the complex amplitude at the second position ($z = z_j$) of the j-th block.

Any of the refractive index distribution measuring methods A to C described above can realize three-dimensional refractive index tomography in which the influence of multiple scattered light is reduced even when the observation object is a multiple scattering object. Any of the refractive index distribution measuring methods A to C is suitable for measuring the refractive index distribution of a three-dimensional culture as an observation object.

In addition, self-interference may be used in the observation apparatus and the refractive index distribution measuring methods. For example, an observation apparatus 1J illustrated in FIG. C10 includes a light source 11, a lens 12, a lens 21, a mirror 22, a lens 23, a condenser lens 24, an objective lens 25, a mirror 44, a lens 42, an imaging unit 43, and an analysis unit 70. Compared with the configuration of the observation apparatus described above, the observation apparatus 1J is different in that the light output from the light source 11 is guided by the optical fiber 14, and then output from the light output end 18 without being split into two light beams. Further, the observation apparatus 1J is different in that the mirror 44 is provided instead of the beam splitter 41. The observation apparatus 1J does not include an interference optical system. The imaging unit 43 can image the interference intensity image at the reference position generated by self-interference of the light irradiating the observation object S along each of the plurality of light irradiation directions and passed through the observation object S. The analysis unit 70 can perform the same image processing as described above using the interference intensity image due to self-interference.

Further, the three-dimensional refractive index distribution of the observation object S from the first position to the second position may not be the refractive index distribution based on the three-dimensional phase image, and may be acquired separately by using a refractive index distribution acquisition apparatus capable of acquiring the refractive index distribution. In this case, the observation apparatus may include (1) an interference intensity image acquisition unit for acquiring, for each of a plurality of light irradiation directions, an interference intensity image at a reference position from an imaging unit for imaging the interference intensity image at the reference position of light irradiating an observation object along each of the plurality of light irradiation directions and passed through the observation object, (2) a first complex amplitude image generation unit for generating, for each of the plurality of light irradiation directions, a complex amplitude image based on the interference intensity image, (3) a refractive index distribution acquisition unit for acquiring a three-dimensional refractive index distribution of the observation object between a first position and a second position with respect to a distance from the imaging unit along a light propagation path, and (4) a second complex amplitude image generation unit for generating, for each of the plurality of light irradiation directions, a complex amplitude image at the second position based on a complex amplitude image at the first position and the three-dimensional refractive index distribution (corresponding to the third complex amplitude image generation unit provided in the observation apparatuses 1A to 1D).

Further, in this case, the refractive index distribution measuring method may include (1) an interference intensity image acquisition step of acquiring, for each of a plurality of light irradiation directions, an interference intensity image at a reference position from an imaging unit for imaging the interference intensity image at the reference position of light irradiating an observation object along each of the plurality of light irradiation directions and passed through the observation object, (2) a first complex amplitude image generation step of generating, for each of the plurality of light irradiation directions, a complex amplitude image based on the interference intensity image, (3) a refractive index distribution acquisition step of acquiring a three-dimensional refractive index distribution of the observation object between a first position and a second position with respect to a distance from the imaging unit along a light propagation path, and (4) a second complex amplitude image generation step of generating, for each of the plurality of light irradiation directions, a complex amplitude image at the second position based on a complex amplitude image at the first position and the three-dimensional refractive index distribution.

A first aspect of the present disclosure is a method for identifying a bile canaliculus region included in an observation object by using refractive index distribution data of the observation object containing a hepatocyte. In one embodiment, the bile canaliculus region is identified on the basis that the region in the observation object has the feature of a bile canaliculus. That is, in one embodiment, a region that is in the observation object and has the feature of the bile canaliculus is identified as the bile canaliculus region. In addition, in one embodiment, the bile canaliculus region may be identified by a machine learning model using a feature value corresponding to the region in the observation object having the feature of the bile canaliculus.

The feature of the bile canaliculus according to one embodiment includes one or more features selected from the group consisting of a feature that the region is an approximately circular or approximately cylindrical region existing between the hepatocytes, a feature that the refractive index of the region is lower than the refractive index of the hepatocyte, and a feature that the refractive index of the circumferential edge portion of the region is higher than the refractive index of the hepatocyte, and the feature of the bile canaliculus according to one embodiment includes the feature that the region is the approximately circular or approximately cylindrical region existing between the hepatocytes, the feature that the refractive index of the region is lower than the refractive index of the hepatocyte, and the feature that the refractive index of the circumferential edge portion of the region is higher than the refractive index or the hepatocyte. The refractive index of the region indicates a statistic value such as the average value, the median value, the minimum value, or the maximum value of the refractive index in the region. In addition, the refractive index of the hepatocyte indicates a statistic value such as the average value, the median value, the minimum value, or the maximum value of the refractive index in all or a part of the hepatocytes. The approximately circular region represents a region of which the circularity is a threshold value or more. The threshold value can be set in light of the type and the state of the observation object, the type and the content of the hepatocyte included in the observation object, and the like. The threshold value can be set so that, in refractive index distribution data of an observation object (a reference) different from the observation object (a sample) for which the bile canaliculus is identified, a region having the feature of another bile canaliculus is identified as the bile canaliculus region. For example, in a case where the sample is a cluster of hepatocytes, another cluster of hepatocytes can be used as the reference, and a two-dimensional liver culture can also be used. The threshold value, for example, may be 65%, may be 70%, may be 75%, may be 80%, or may be 85%. The approximately cylindrical region is a tubular region in which the cross section of the region perpendicular to the axis of a tubular flow or the cross section seen from a tomographic plane in refractive index tomography data is in an approximately circular shape. The axis of the tubular flow is an axis consisting of an axial line formed by the aggregation of cross-sectional centers (barycenters) of the tube. The circumferential edge portion of the region is a concept including, in the portion of the observation object not included in the region, a portion adjacent to the region (an adjacent portion) and a portion separated from the adjacent portion by a predetermined distance, and corresponds to a portion in the vicinity of the lumen, in a portion occupied by the hepatocytes forming the tube wall of the bile canaliculus by a tight junction.

In one embodiment, the feature of the bile canaliculus further includes a feature of comprising a region that is derived from a microvillus and has a refractive index higher than the refractive index of the entire region. In general, the region derived from the microvillus is in an approximately linear shape or curve shape (such as a curved shape and a spiral shape) in a case where the refractive index distribution data is three-dimensional data, and is in an approximately linear shape, curve shape, or dot shape in a case where the refractive index distribution data is two-dimensional data such as refractive index tomography data. In a case where the region derived from the microvillus is in the approximately linear shape or curve shape, the length thereof, for example, is approximately 1 to 2 $\mu$m, but may be elongated or shortened depending on the type and the state of the observation object, the type and the content of the hepatocyte contained in the observation object, and the like.

A second aspect of the present disclosure is a method for evaluating a bile canaliculus region, on the basis of a parameter of a bile canaliculus obtained from refractive index distribution data of an observation object containing a hepatocyte. In one embodiment, the bile canaliculus region is evaluated on the basis of the parameter of the bile canaliculus. That is, in one embodiment, a bile canaliculus region in the observation object, in which the parameter of the bile canaliculus has a more excellent value, is evaluated as a bile canaliculus with higher quality. In addition, in one embodiment, the bile canaliculus region may be evaluated by a machine learning model learned to set the bile canaliculus region in which the parameter has a more excellent value as the bile canaliculus with higher quality by using a feature value corresponding to the parameter of the bile canaliculus.

In one embodiment, the parameter of the bile canaliculus includes one or more parameters selected from the group consisting of the area or the boundary length of a cross section perpendicular to an axis of a tubular flow or a cross section seen from a tomographic plane, a refractive index, the median value of the refractive index, the number of microvilli, and the average length of the microvillus, and in one embodiment, the parameter of the bile canaliculus includes one or more parameters selected from the group consisting of the area of the cross section perpendicular to the axis of the tubular flow or the cross section seen from the tomographic plane, the refractive index, the number of microvilli, and the average length of the microvillus. In one embodiment, for the area of the cross section, since it is considered that the bile canaliculus is matured as the bile canaliculus is thickened, a bile canaliculus region with a large cross sectional area may be determined as a bile canaliculus with high quality. The refractive index is the refractive index of the bile canaliculus region, and in one embodiment, since it is considered that the refractive index increases as a space occupied by the microvillus and the amount of contents increases, a bile canaliculus region with a high refractive index may be evaluated as the bile canaliculus with high quality. In one embodiment, for the number of microvilli and the length of the microvillus, since it is considered that the secreted amount of a bile acid into the bile canaliculus and the excreted amount of a liver metabolite increases as the surface area of the microvillus increases, a bile canaliculus region with a large number of microvilli may be determined as the bile canaliculus with high quality, or a bile canaliculus region with a long average length of the microvillus may be determined as the bile canaliculus with high quality.

In one embodiment, the method for evaluating the bile canaliculus region may comprise a step of identifying the bile canaliculus region included in the observation object by using the refractive index distribution data of the observation object, before the evaluation step. That is, the method for evaluating the bile canaliculus region in one embodiment comprises a step of identifying the bile canaliculus region included in the observation object by using the refractive index distribution data of the observation object, and a step of evaluating the bile canaliculus region, on the basis of the parameter of the bile canaliculus in the identified bile canaliculus region. In one embodiment, the method for evaluating the bile canaliculus region comprises a step of identifying the bile canaliculus region included in the observation object by using the refractive index distribution data of the observation object, in which the bile canaliculus region is identified on the basis that the region in the observation object has the feature of the bile canaliculus, and the feature of the bile canaliculus includes a feature that the region is an approximately circular or approximately cylindrical region existing between the hepatocytes, a feature that the refractive index of the region is lower than the refractive index of the hepatocyte, and a feature that the refractive index of the circumferential edge portion of the region is higher than the refractive index of the hepatocyte, and a step of evaluating the bile canaliculus region, on the basis of the parameter of the bile canaliculus in the identified bile canaliculus region.

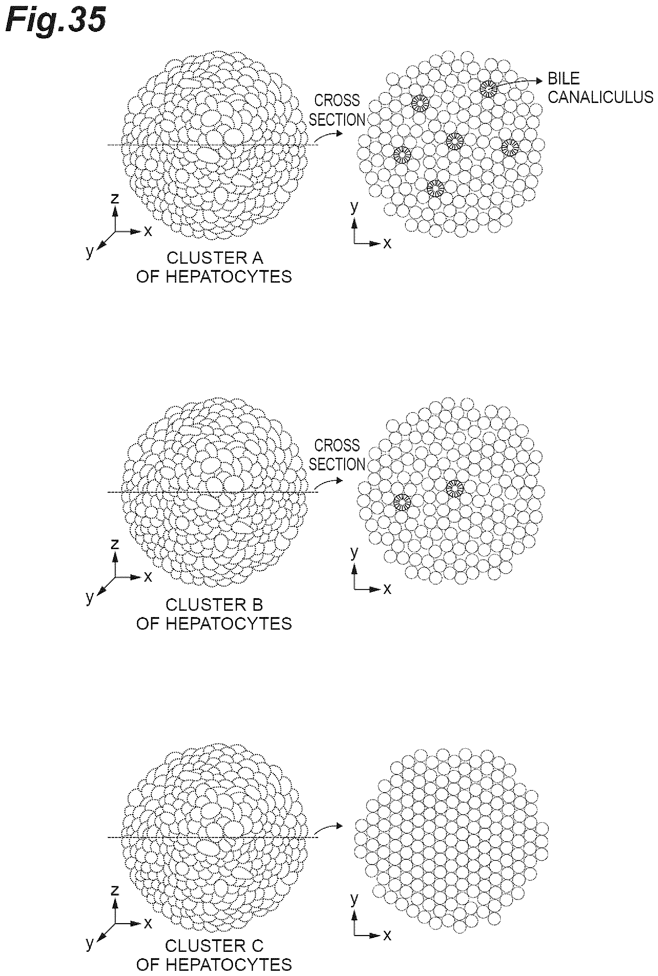

A third aspect of the present disclosure is a method for evaluating an observation object containing a hepatocyte, on the basis of the number of bile canaliculus regions identified by the method according to the first aspect of the present disclosure (the method for identifying the bile canaliculus region included in the observation object by using the refractive index distribution data of observation object the containing the hepatocyte) and/or the evaluation of the bile canaliculus region obtained by the method according to the second aspect of the present disclosure (the method for evaluating the bile canaliculus region, on the basis of the parameter of the bile canaliculus obtained from the refractive index distribution data of the observation object containing the hepatocyte). Since it is general to evaluate the quality of the observation object containing the hepatocyte by using the number of bile canaliculi as an index, and since it is obvious that an observation object , containing the hepatocyte, having a higher-quality bile canaliculus is with higher quality, it is possible to evaluate the observation object itself containing the hepatocyte by the method for identifying the bile canaliculus region and the method for evaluating the bile canaliculus region. For example, in the example illustrated in FIG. 35, a cluster A of hepatocytes with a large number of bile canaliculus regions can be evaluated as a cluster of hepatocytes with high quality.

The method for evaluating the observation object containing the hepatocyte according to one embodiment, for example, can be used in the following mode.

For example, since it is known that the formation of the bile canaliculus can be used as one of rough standards for the differentiation of the observation object containing the hepatocyte, by evaluating the number of bile canaliculus regions included in the observation object containing the hepatocyte, it is possible to evaluate the differentiation of the observation object containing the hepatocyte. That is, another aspect is a method for evaluating the differentiation of the observation object containing the hepatocyte by using the refractive index distribution data of the observation object containing the hepatocyte.

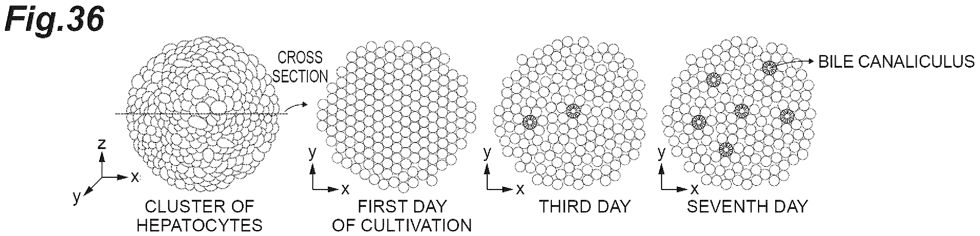

For example, the method for evaluating the observation object containing the hepatocyte according to one embodiment can be used for the evaluation of a hepatocyte cultivation method. As the hepatocyte cultivation method, in addition to general plane cultivation, various cultivation methods such as a sandwich cultivation method, a three-dimensional spheroid cultivation method, a hollow fiber bioreactor method, a micropattern co-cultivation method, a perfusion multiwell plate cultivation method, a microfluid liver biochip cultivation method, and a microfluid multiple organ device cultivation method have been developed, and the hepatocyte cultivation method can be evaluated by evaluating the number of bile canaliculus regions included in the observation object containing the hepatocyte that is cultivated by the methods described above. That is, a fourth aspect of the present disclosure is a method for evaluating a hepatocyte cultivation method comprising a step of cultivating a hepatocyte, and a step of evaluating an observation object obtained by the cultivation, using the method according to the third aspect of the present disclosure (the method for evaluating the observation object containing the hepatocyte, on the basis of the number of bile canaliculus regions identified by the method according to the first aspect of the present disclosure (the method for identifying the bile canaliculus region included in the observation object by using the refractive index distribution data) and/or the evaluation of the bile canaliculus region obtained by the method according to the second aspect of the present disclosure (the method for evaluating the bile canaliculus region by the parameter of the bile canaliculus obtained from the refractive index distribution data)). The hepatocyte cultivation method can also be evaluated, for example, as illustrated in FIG. 36, on the basis of the number of bile canaliculus regions according to the number of cultivation days of a cluster of hepatocytes, by taking an advantage that the bile canaliculus region can be non-invasively identified and evaluated.

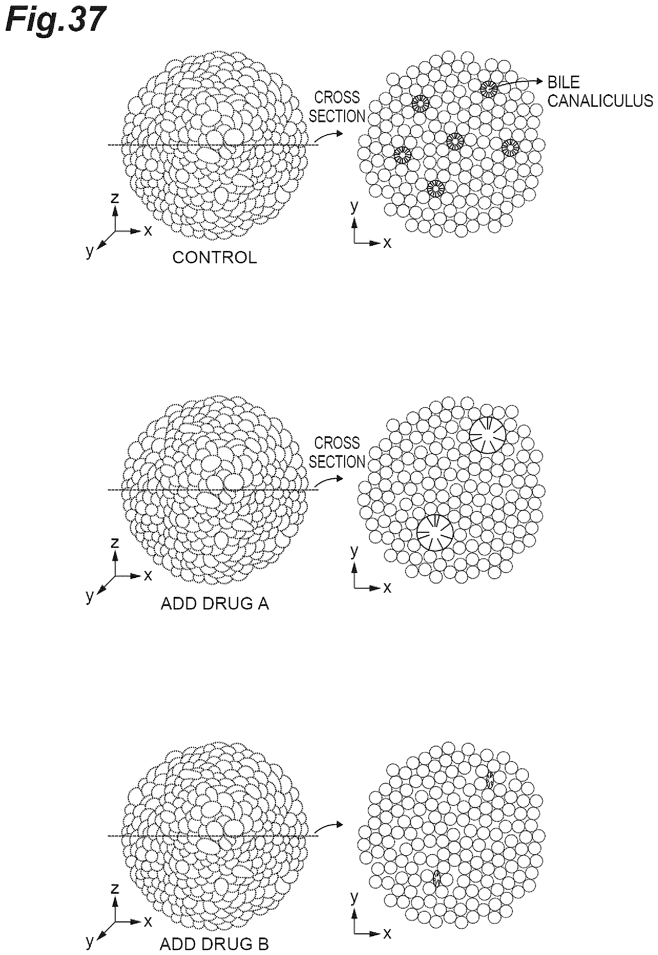

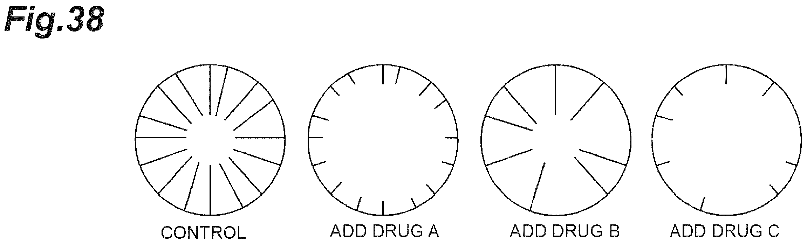

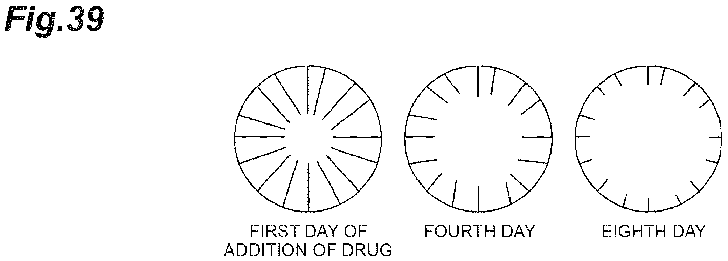

For example, the method for evaluating the observation object containing the hepatocyte according to one embodiment can be used for the screening of a drug. The bile canaliculus is not only a secretion destination of a bile acid synthesized in the hepatocyte but also an excretion route of liver metabolism that is a main excretion method of a drug. In addition, a drug-induced liver injury (DILI) is one of main reasons for the withdrawal of a pre-approved drug from the market and a restriction on use thereof, and the disapproval of a new drug due to a safety issue, and includes a hepatocellular disorder-type DILI, a bile stasis-type DILI, a combined DILI, and the like, and it is known that the morphological abnormality of the bile canaliculus is found, particularly in the bile stasis-type DILI. Therefore, by screening using the method for evaluating the observation object containing the hepatocyte according to one embodiment, for example, a drug causing an abnormal increase in the refractive index of the bile canaliculus region can be excluded from developmental candidates, as a drug that may cause the abnormal secretion or the abnormal excretion of a bile acid or a liver metabolite, and a drug causing the morphological abnormality of the bile canaliculus can be excluded from the developmental candidates, as a drug that may cause a bile stasis-type DILI. In a case where such events, which were only clarified during the clinical stage in the related art, can be clarified in vitro, it is possible to prevent in advance the failure of huge investments of money and time on a clinical trial by eliminating the withdrawal of the pre-approved drug from the market or the restriction on use thereof, and the disapproval of the new drug due to the safety issue. That is, a fifth aspect of the present disclosure is a drug screening method comprising a step of adding a drug to an observation object containing a hepatocyte, and a step of evaluating the observation object obtained by cultivation, using the method according to the third aspect of the present disclosure (the method for evaluating the observation object containing the hepatocyte, on the basis of the number of bile canaliculus regions identified by the method according to the first aspect of the present disclosure (the method for identifying the bile canaliculus region included in the observation object by using the refractive index distribution data) and/or the evaluation of the bile canaliculus region obtained by the method according to the second aspect of the present disclosure (the method for evaluating the bile canaliculus region by the parameter of the bile canaliculus obtained from the refractive index distribution data)). The screening of the drug, for example, can be performed by using the morphological abnormality of the bile canaliculus as an index, and in the example illustrated in FIG. 37, a drug A causing the expansion of the bile canaliculus and a drug B causing the contraction of the bile canaliculus in the cluster of hepatocytes can be excluded from the developmental candidates, as the drug that may cause the bile stasis-type DILI, on the basis of the area of the cross section of the bile canaliculus region. In addition, the screening of the drug, for example, can also be performed by using the morphological abnormality of the bile canaliculus focusing on a microvillus as an index, and in the example illustrated in FIG. 38, a drug A causing the shortening of the microvillus, a drug B causing the reduction of the microvillus, and a drug C causing the shortening and the reduction of the microvillus can be excluded from the developmental candidates, as the drug that may cause the bile stasis-type DILI. Such screening can also be performed on the basis of a chronological change at a plurality of time points, by taking an advantage that the bile canaliculus region can be non-invasively identified and evaluated, and in the example illustrated in FIG. 39, a drug causing the shortening of the microvillus according to the number of cultivation days after the drug is added can be excluded from the developmental candidates, as the drug that may cause the bile stasis-type DILI.

In addition, the method for identifying the bile canaliculus region according to one embodiment, for example, can be used in the following mode.

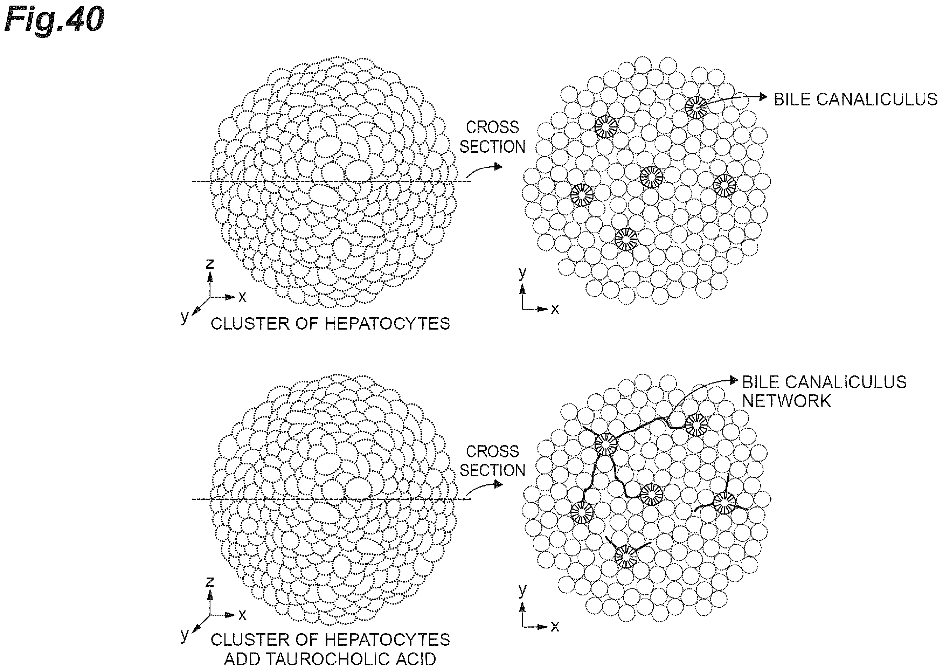

For example, the method for identifying the bile canaliculus region according to one embodiment can be used for evaluating the formation of a bile canaliculus network. It is known that after the formation of the bile canaliculus, the bile canaliculus further forms a three-dimensional network to secrete or excrete the liver metabolite, and by comprehensively identifying the bile canaliculus region included in the observation object containing the hepatocyte using the method for identifying the bile canaliculus region according to one embodiment, it is possible to evaluate the formation of the bile canaliculus network. Further, in one embodiment, as with the example illustrated in FIG. 40, the formation of the bile canaliculus network caused by adding a bile acid such as a taurocholic acid, which accelerates the formation of the network, may be evaluated. In addition, in one embodiment, the inhibition of the formation of the bile canaliculus network caused by adding a bile acid such as a lithocholic acid, which inhibits the formation of the network, may be evaluated. Such evaluation can also be performed on the basis of a chronological change at a plurality of time points, by taking an advantage that the bile canaliculus region can be noninvasively identified, and can also be performed by using the volume of the bile canaliculus region as an evaluation index.

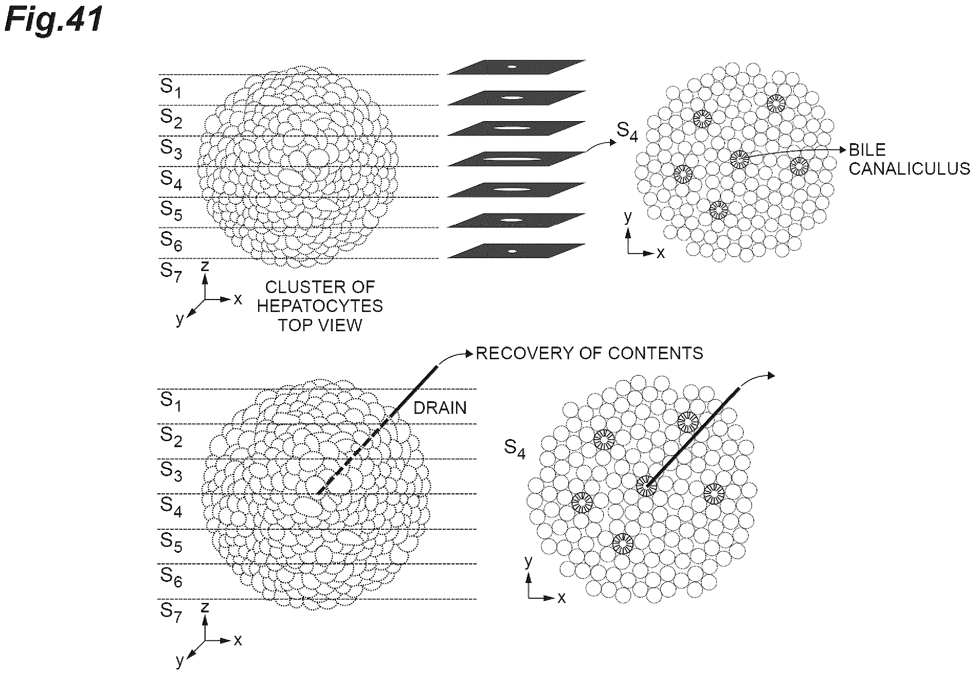

For example, the method for identifying the bile canaliculus region according to one embodiment can also be used as a guide for recovering contents from the bile canaliculus. The bile canaliculus is one of main routes for the liver metabolism, and in a case where the bile canaliculus region in the observation object containing the hepatocyte can be non-invasively identified, for example, as with the example illustrated in FIG. 41, a drain tube (a drain), a catheter, or the like can be inserted into the identified bile canaliculus, and the contents can be recovered. The recovered contents, for example, can be analyzed by mass analysis, liquid chromatography, gas chromatography, or the like.

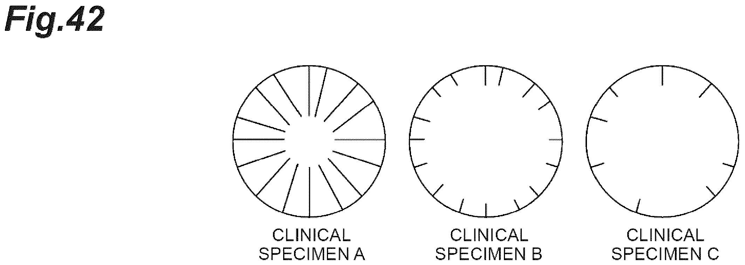

In addition, the method for evaluating the bile canaliculus region according to one embodiment can also be used as a method for diagnosing liver disease in a clinical specimen of a liver, a method for assisting the diagnosis of liver disease, or a method for collecting data for diagnosing liver disease. It is obvious that an abnormality in the bile canaliculus may occur in a liver that has developed liver disease, and for example, in a liver with Byler's disease, a lesion referred to as "Byler's Bile", in which the microvilli in the bile canaliculus are extremely reduced, is observed. In addition, in bile stasis derived from the liver disease, the expansion of the bile canaliculus, the reduction of the microvillus, and the formation of a bleb are observed. Although such findings have been disclosed by using an electron microscope, the method according to one embodiment for non-invasively evaluating the bile canaliculus region using the refractive index distribution data, it is possible to simply detect the abnormality in the bile canaliculus without having antagonism with other pathological stain and the like, such as HE stain, in the clinical specimen of the liver. In a case where the abnormality in the bile canaliculus can be detected on the basis of the refractive index distribution data, it is expected that more clinical specimens can be handled earlier. For example, in the example illustrated in FIG. 42, by comparing bile canaliculus regions in clinical specimens A to C, it is possible to assist diagnosis that the clinical specimen B with short microvilli and the clinical specimen C with short and few microvilli are highly likely to be the clinical specimen of the liver that has developed the liver disease.

Hereinafter, the present disclosure will be described in more detail by Examples and the like, but the present disclosure is not limited thereto.

**Examples**

[Example 1: Identification of Bile Canaliculus Region of Two-Dimensional Liver Culture Using Phase Contrast and Fluorescence Microscope]

A cell (HepG2) derived from a human liver cancer was seeded in a glass bottom dish (manufactured by MatTek), and cultivated in a DMEM culture medium containing 10% fetal bovine serum for 5 days under a condition of 5% $CO_2$, 37 Celsius degrees, and 100% humidity to prepare a two-dimensional liver culture. After the culture medium was removed, a Hanks' balanced salt solution (a final concentration of 2 $\mu$M) containing calcium chloride and magnesium chloride, of cholyl-lysyl-fluorescein (CLF, manufactured by Corning Incorporated), which is a fluorescence-marked bile acid known to be secreted to a bile canaliculus, was added, and was incubated for 45 minutes under a condition of 5% $CO_2$, 37°C, and 100% humidity.

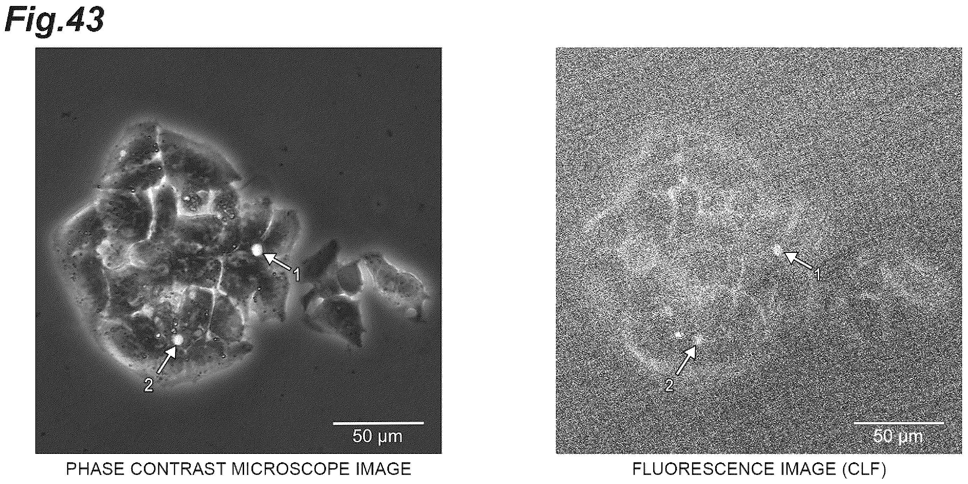

A phase contrast microscope image and a fluorescence image were imaged at the same field of view by using an inverted microscope DM IL LED (manufactured by Leica). An objective lens with a magnification of 20 times was used. The fluorescence image was acquired in a condition of an excitation wavelength of 470 nm and a fluorescence wavelength of 500 to 550 nm. Results are shown in FIG. 43.

From the phase contrast microscope image, it was observed that a lumen structure (in the drawing, arrows 1 and 2) exists encompassed by the cells, and from the fluorescence image, it was observed that CLF is accumulated in the lumen structure. From the above, the regions of the arrows 1 and 2 in the drawing were identified as a bile canaliculus region.

[Example 2: Evaluation of Bile Canaliculus Region of Two-Dimensional Liver Culture Using Optical Diffraction Tomography (ODT)]

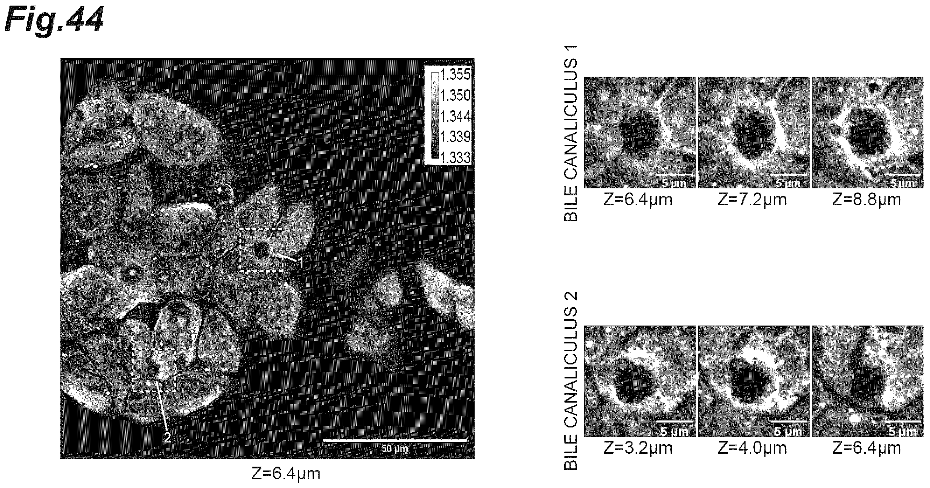

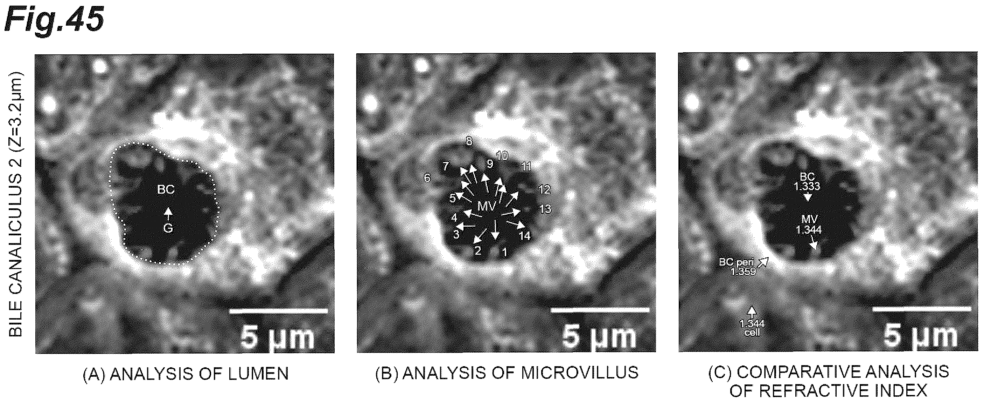

For the two-dimensional liver culture used in Example 1, refractive index distribution data was acquired by using the observation device 1A and ODT described in the refractive index distribution measurement method A2. An objective lens with a magnification of 60 times was used. Representative refractive index tomography data extracted from the obtained refractive index distribution data is shown in FIG. 44 and FIG. 45.

In FIG. 44, for the region identified as the bile canaliculus region in Example 1 (in the drawing, the arrows 1 and 2), a feature that the region is an approximately circular region (a circularity of 86%, hereinafter, a numerical value in parentheses in the paragraphs indicates a value in a bile canaliculus 2) existing between hepatocytes, a feature that the refractive index (1.336) of the region is lower than the refractive index (1.344) of the hepatocyte, and a feature that the refractive index (1.359) of the circumferential edge portion of the region is higher than the refractive index (1.344) of the hepatocyte were observed. In addition, in FIG. 45, for the region identified as the bile canaliculus region in Example 1 (in the drawing, the arrows 1 and 2), a feature of including a region that is derived from a microvillus (MV) and has a refractive index (1.344) higher than the refractive index (1.333) of the lumen of a bile canaliculus (BC) was further observed. In FIG. 45, G represents the barycenter of the bile canaliculus region.

[Example 3: Evaluation of Bile Canaliculus Region of Cluster of Hepatocytes Using Optical Diffraction Tomography (ODT)]

A cell (HepG2) derived from a human liver cancer was seeded in a cell cultivation container (manufactured by AGC Techno Glass, "EZSPHERE") including a compartment with low cellular adhesiveness at a density of $1.00 \times 10^5$ cells per 1 well, and was cultivated in a DMEM culture medium containing 10% fetal bovine serum for 3 days under a condition of 5% $CO_2$, 37°C, and 100% humidity. Dimethyl sulfoxide (manufactured by Sigma-Aldrich Corporation) was added thereto such that a final concentration was 0.05%, and cultivation for 1 day was further performed to prepare a cluster of hepatocytes.

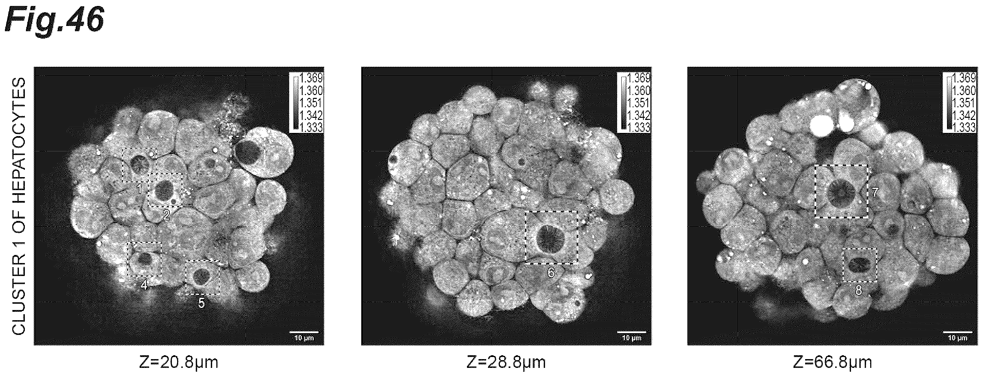

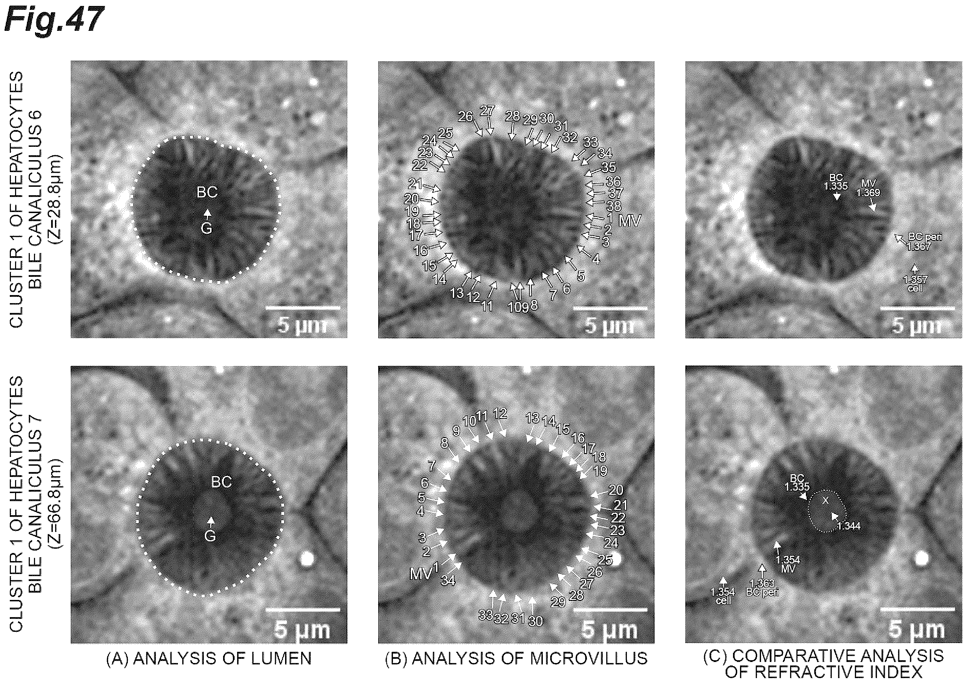

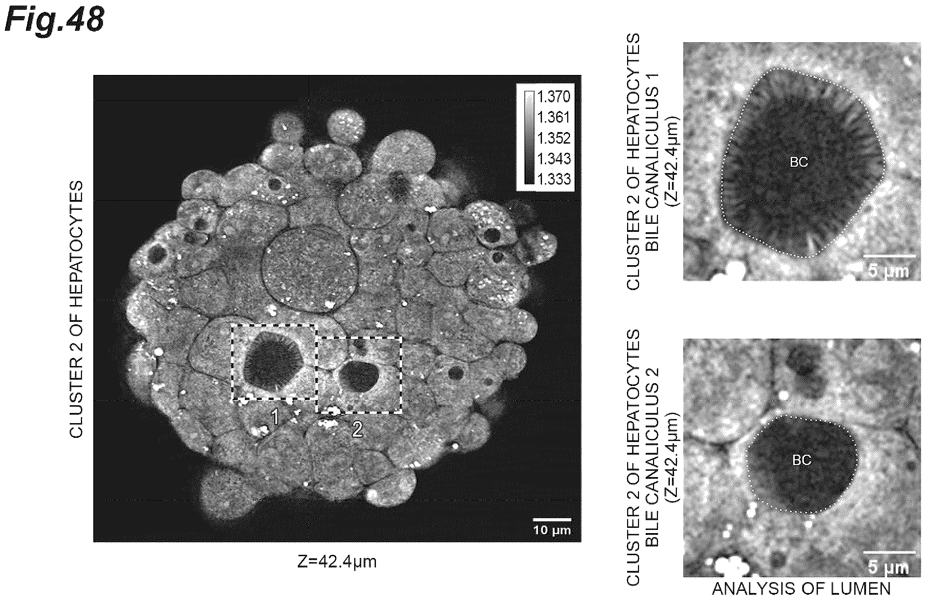

For the obtained cluster of hepatocytes (a cluster 1 of hepatocytes), refractive index distribution data was acquired by using the observation device 1A and ODT described in the refractive index distribution measurement method C. An objective lens with a magnification of 60 times was used. Representative refractive index tomography data extracted from the obtained refractive index distribution data is shown in FIG. 46 and FIG. 47. Parameters obtained from the refractive index distribution data acquired in Example 2 and the refractive index distribution data acquired for the cluster 1 of hepatocytes in Example 3 are shown in Tables 1 to 4. In addition, for another cluster of hepatocytes (a cluster 2 of hepatocytes) prepared by the same method, representative refractive index tomography data extracted from the refractive index distribution data acquired by the same method is shown in FIG. 48.

[Table 1]

| (A) Analysis of bile canalicular lumen (BC) | | Morphological information of BC | | |
|---|---|---|---|---|
| | | Area | Boundary length | Circularity |
| Two- dimensional liver culture | Bile canaliculus 2 | 26.2 $\mu m^2$ | 19.5 $\mu m$ | 0.86 |
| Cluster 1 of hepatocytes | Bile canaliculus 6 | 70.6 $\mu m^2$ | 30.8 $\mu m$ | 0.93 |
| Cluster 1 of hepatocytes | Bile canaliculus 7 | 73.5 $\mu m^2$ | 31.5 $\mu m$ | 0.93 |
| Structure X in lumen | | 5.2 $\mu m^2$ | 8.4 $\mu m$ | 0.93 |

[Table 2]

| (A) Analysis of bile canicular lumen (BC) | | Refractive index information of BC | | | | |
|---|---|---|---|---|---|---|
| | | Average | Standard deviation | Minimum value | Maximum value | Median value |
| Two-dimensional liver culture | Bile canaliculus 2 | 1.336 | 0.004 | 1.333 | 1.349 | 1.333 |
| Cluster 1 of hepatocytes | Bile canaliculus 6 | 1.344 | 0.005 | 1.333 | 1.359 | 1.344 |
| Cluster 1 of hepatocytes | Bile canaliculus 7 | 1.344 | 0.004 | 1.334 | 1.357 | 1.344 |

[Table 3]

| (B) Analysis of microvillus (MV) | | Morphological information of MV | |
|---|---|---|---|
| | | Number | Length (MV1) |
| Two-dimensional liver culture | Bile canaliculus 2 | 14 | 1.2 μm |
| Cluster 1 of hepatocytes | Bile canaliculus 6 | 38 | 2.1 μm |
| Cluster 1 of hepatocytes | Bile canaliculus 7 | 34 | 2.0 μm |

[Table 4]

| (C) Comparative analysis of refractive index | | Comparative information of refractive index | | | | |
|---|---|---|---|---|---|---|
| | | BC | MV | Circumferential edge portion | Hepatocyte | Structure |
| Two-dimensional liver culture | Bile canaliculus 2 | 1.333 | 1.344 | 1.359 | 1.344 | - |
| Cluster 1 of hepatocytes | Bile canaliculus 6 | 1.335 | 1.369 | 1.367 | 1.357 | - |
| Cluster 1 of hepatocytes | Bile canaliculus 7 | 1.335 | 1.354 | 1.363 | 1.354 | 1.344 |

In FIG. 46, it was observed that a structure (a bile canaliculus) having the same feature as the feature observed in the two-dimensional liver culture also exists in the cluster of hepatocytes. When observing any cross section, for example, it was observed that there are five bile canaliculi at Z = 20.8 μm, one bile canaliculus at Z = 28.8 μm, and two bile canaliculi at Z = 66.8 μm, which indicated that the bile canaliculus region can be identified by using the refractive index distribution data in the cluster of hepatocytes.

In FIG. 47, in addition to the existence of the bile canaliculus, it was possible to observe that the size of the lumen, the number of microvilli, the refractive index, and the like are different in each of the bile canaliculi, which indicated that the bile canaliculus region can be evaluated by using the refractive index distribution data in the cluster of hepatocytes. Further, for example, a structure X in the lumen inside the bile canaliculus region was observed in a bile canaliculus 7, which indicated that there is a possibility that the existence of features and parameters useful to the identification or the evaluation of the bile canaliculus region, the evaluation of the cluster of hepatocytes, the screening of the drug, or the like can be found by using the refractive index distribution data, in addition to the features and the parameters used in Examples.

In FIG. 48, as an evaluation example of the bile canaliculus region, for example, when comparing parameters of two bile canaliculus regions in the refractive index tomography data, since the bile canaliculus 1 in the drawing has a larger area of the cross section, a longer boundary length of the cross section, and a larger number of microvilli, a larger refractive index, and a larger median value of the refractive index than those of the bile canaliculus 2, the bile canaliculus 1 can be determined as a bile canaliculus with high quality.

**Reference Signs List**

1A to 1J: observation device, 2: recording medium, 11: light source, 12: lens, 13: light incident end, 14: optical fiber, 15: fiber coupler, 16, 17: optical fiber, 18, 19: light exiting end, 21: lens, 22: mirror, 23: lens, 24: condenser lens, 25: objective lens, 31: lens, 32: mirror, 33: driving unit, 34: lens, 41: beam splitter, 42: lens, 43: imaging unit, 44: mirror, 50:

analysis unit, 51: interference intensity image acquisition unit, 52: first complex amplitude image creation unit, 53: second complex amplitude image creation unit, 54: two-dimensional phase image creation unit, 55: three-dimensional phase image creation unit, 56: refractive index distribution calculation unit, 57: display unit, 58: storage unit, 60: analysis unit, 61: interference intensity image acquisition unit, 62: first complex amplitude image creation unit, 63: second complex amplitude image creation unit, 64: phase conjugation arithmetic unit, 65: two-dimensional phase image creation unit, 66: three-dimensional phase image creation unit, 67: refractive index distribution calculation unit, 68: display unit, 69: storage unit, 70: analysis unit, 71: interference intensity image acquisition unit, 72: first complex amplitude image creation unit, 73: second complex amplitude image creation unit, 74: two-dimensional phase image creation unit, 75: three-dimensional phase image creation unit, 76: refractive index distribution calculation unit, 77: third complex amplitude image creation unit, 78: display unit, 79: storage unit.

**Claims**

1. A method for identifying a bile canaliculus region included in an observation object by using refractive index distribution data of the observation object containing a hepatocyte.

2. The method according to claim 1,
   wherein the bile canaliculus region is identified on the basis that the region in the observation object has a feature of a bile canaliculus, and the feature of the bile canaliculus includes a feature that the region is an approximately circular or approximately cylindrical region existing between hepatocytes, a feature that a refractive index of the region is lower than a refractive index of hepatocyte, and a feature that a refractive index of a circumferential edge portion of the region is higher than the refractive index of the hepatocyte.

3. The method according to claim 2,
   wherein the feature of the bile canaliculus further includes a feature of comprising a region that is derived from a microvillus and has a refractive index higher than a refractive index of the entire region.

4. The method according to claim 3,
   wherein the refractive index distribution data is refractive index tomography data in a predetermined direction.

5. A method for evaluating a bile canaliculus region, on the basis of a parameter of a bile canaliculus obtained from refractive index distribution data of an observation object containing a hepatocyte,
   wherein the parameter of the bile canaliculus includes at least one parameter of an area of a cross section perpendicular to an axis of a tubular flow or a cross section seen from a tomographic plane, a refractive index, the number of microvilli, and an average length of microvilli.

6. The method according to any one of claims 1 to 5,
   wherein the observation object containing the hepatocyte is a cluster of hepatocytes.

7. The method according to claim 5,
   wherein the method comprises a step of identifying the bile canaliculus region included in the observation object by the method according to any one of claims 1 to 4, and the bile canaliculus region is evaluated on the basis of the parameter of the bile canaliculus in the identified bile canaliculus region.

8. A method for evaluating an observation object containing a hepatocyte, on the basis of the number of bile canaliculus regions identified by the method according to any one of claims 1 to 4 and/or the evaluation of the bile canaliculus region obtained by the method according to claim 5.

9. A method for evaluating a hepatocyte cultivation method, comprising:

   a step of cultivating a hepatocyte; and
   a step of evaluating an observation object obtained by the cultivation, using the method according to claim 8.

10. A drug screening method, comprising:

    a step of adding a drug to an observation object containing a hepatocyte; and
    a step of evaluating the observation object to which the drug is added, using the method according to claim 8.

**11.** The method according to claim 8,
wherein the observation object containing the hepatocyte is a cluster of hepatocytes.

**12.** A device for identifying a bile canaliculus region, comprising:

a data acquisition unit acquiring refractive index distribution data of an observation object containing a hepatocyte; and
an identification unit identifying the bile canaliculus region included in the observation object by the method according to any one of claims 1 to 4.

**13.** A device for evaluating a bile canaliculus region, comprising:

a data acquisition unit acquiring refractive index distribution data of an observation object containing a hepatocyte;
an identification unit identifying the bile canaliculus region included in the observation object by the method according to any one of claims 1 to 4; and
an evaluation unit evaluating the bile canaliculus region by the method according to claim 5.

**14.** A program for causing a computer to execute:

a data acquisition step of acquiring refractive index distribution data of an observation object containing a hepatocyte; and
an identification step of identifying a bile canaliculus region included in the observation object by the method according to any one of claims 1 to 4.

**15.** A program for causing a computer to execute:

a data acquisition step of acquiring refractive index distribution data of an observation object containing a hepatocyte;
an identification step of identifying a bile canaliculus region included in the observation object by the method according to any one of claims 1 to 4; and
an evaluation step of evaluating the bile canaliculus region by the method according to claim 5.

Fig.1

**Fig.2**

# Fig.3

ANALYSIS UNIT — 50

INTERFERENCE INTENSITY IMAGE ACQUISITION UNIT — 51

FIRST COMPLEX AMPLITUDE IMAGE GENERATION UNIT — 52

SECOND COMPLEX AMPLITUDE IMAGE GENERATION UNIT — 53

TWO-DIMENSIONAL PHASE IMAGE GENERATION UNIT — 54

THREE-DIMENSIONAL PHASE IMAGE GENERATION UNIT — 55

REFRACTIVE INDEX DISTRIBUTION CALCULATION UNIT — 56

DISPLAY UNIT — 57

STORAGE UNIT — 58

RECORDING MEDIUM — 2

1C

EP 4 542 203 A1

# Fig.4

INTERFERENCE INTENSITY IMAGE ACQUISITION — S1

INTERFERENCE INTENSITY IMAGE FOR
EACH LIGHT IRRADIATION DIRECTION

FIRST COMPLEX AMPLITUDE
IMAGE GENERATION — S2

COMPLEX AMPLITUDE IMAGE FOR
EACH LIGHT IRRADIATION DIRECTION

SECOND COMPLEX AMPLITUDE
IMAGE GENERATION — S3

COMPLEX AMPLITUDE IMAGE FOR
EACH POSITION AND EACH
LIGHT IRRADIATION DIRECTION

TWO-DIMENSIONAL PHASE IMAGE GENERATION — S4

TWO-DIMENSIONAL PHASE IMAGE
FOR EACH POSITION

THREE-DIMENSIONAL PHASE IMAGE GENERATION — S5

THREE-DIMENSIONAL
PHASE IMAGE

REFRACTIVE INDEX DISTRIBUTION CALCULATION — S6

THREE-DIMENSIONAL REFRACTIVE
INDEX DISTRIBUTION

# Fig.5

(a)

(b)

(c)

Fig.6

Fig.7

(a)

(b)

EP 4 542 203 A1

Fig.8

EP 4 542 203 A1

# Fig.9

COMPLEX AMPLITUDE IMAGE FOR
EACH LIGHT IRRADIATION DIRECTION

PHASE CORRECTION AND SUMMATION OF
COMPLEX AMPLITUDE IMAGES ～S11

COMPLEX AMPLITUDE SUMMATION
IMAGE FOR EACH POSITION

PHASE IMAGE GENERATION ～S12

TWO-DIMENSIONAL PHASE IMAGE
FOR EACH POSITION

# Fig.10

```
┌─────────────────────────────────┐
│   COMPLEX AMPLITUDE IMAGE FOR    │
│       EACH POSITION AND EACH     │
│    LIGHT IRRADIATION DIRECTION   │
└─────────────────────────────────┘
                 │
┌─────────────────────────────────────┐
│  COMPLEX DIFFERENTIAL INTERFERENCE   │  ~S21
│         IMAGE GENERATION             │
└─────────────────────────────────────┘
                 │
┌─────────────────────────────────────┐
│  COMPLEX DIFFERENTIAL INTERFERENCE   │
│      IMAGE FOR EACH POSITION AND     │
│    EACH LIGHT IRRADIATION DIRECTION  │
└─────────────────────────────────────┘
                 │
┌─────────────────────────────────────┐
│  PHASE DIFFERENTIAL IMAGE GENERATION │  ~S22
└─────────────────────────────────────┘
                 │
┌─────────────────────────────────────┐
│      PHASE DIFFERENTIAL IMAGE        │
│         FOR EACH POSITION            │
└─────────────────────────────────────┘
                 │
┌─────────────────────────────────────┐
│        PHASE IMAGE GENERATION        │  ~S23
└─────────────────────────────────────┘
                 │
┌─────────────────────────────────────┐
│    TWO-DIMENSIONAL PHASE IMAGE       │
│         FOR EACH POSITION            │
└─────────────────────────────────────┘
```

Fig.11

# Fig.12

```
┌─────────────────────────────────────┐
│    COMPLEX AMPLITUDE IMAGE FOR       │
│       EACH POSITION AND EACH         │
│     LIGHT IRRADIATION DIRECTION      │
└─────────────────────────────────────┘
                   │
┌─────────────────────────────────────┐
│ DIVIDING INTO BATCHES AND PHASE CORRECTION │ ～S31
│ AND SUMMATION OF COMPLEX AMPLITUDE IMAGES  │
│           FOR EACH BATCH              │
└─────────────────────────────────────┘
                   │
┌─────────────────────────────────────┐
│    COMPLEX AMPLITUDE SUMMATION       │
│     IMAGE FOR EACH POSITION          │
│          AND EACH BATCH              │
└─────────────────────────────────────┘
                   │
┌─────────────────────────────────────┐
│   COMPLEX DIFFERENTIAL INTERFERENCE  │ ～S32
│          IMAGE GENERATION            │
└─────────────────────────────────────┘
                   │
┌─────────────────────────────────────┐
│      COMPLEX DIFFERENTIAL            │
│     INTERFERENCE IMAGE FOR           │
│   EACH POSITION AND EACH BATCH       │
└─────────────────────────────────────┘
                   │
┌─────────────────────────────────────┐
│  PHASE DIFFERENTIAL IMAGE GENERATION │ ～S33
└─────────────────────────────────────┘
                   │
┌─────────────────────────────────────┐
│     PHASE DIFFERENTIAL IMAGE         │
│        FOR EACH POSITION             │
└─────────────────────────────────────┘
                   │
┌─────────────────────────────────────┐
│       PHASE IMAGE GENERATION         │ ～S34
└─────────────────────────────────────┘
                   │
┌─────────────────────────────────────┐
│   TWO-DIMENSIONAL PHASE IMAGE        │
│        FOR EACH POSITION             │
└─────────────────────────────────────┘
```

**Fig.13**

ANALYSIS UNIT — 60

INTERFERENCE INTENSITY IMAGE ACQUISITION UNIT — 61

FIRST COMPLEX AMPLITUDE IMAGE GENERATION UNIT — 62

SECOND COMPLEX AMPLITUDE IMAGE GENERATION UNIT — 63

PHASE CONJUGATE OPERATION UNIT — 64

TWO-DIMENSIONAL PHASE IMAGE GENERATION UNIT — 65

THREE-DIMENSIONAL PHASE IMAGE GENERATION UNIT — 66

REFRACTIVE INDEX DISTRIBUTION CALCULATION UNIT — 67

DISPLAY UNIT — 68

STORAGE UNIT — 69

RECORDING MEDIUM — 2

1D

# Fig.14

ANALYSIS UNIT — 60

INTERFERENCE INTENSITY IMAGE ACQUISITION UNIT — 61

FIRST COMPLEX AMPLITUDE IMAGE GENERATION UNIT — 62

SECOND COMPLEX AMPLITUDE IMAGE GENERATION UNIT — 63

PHASE CONJUGATE OPERATION UNIT — 64

TWO-DIMENSIONAL PHASE IMAGE GENERATION UNIT — 65

THREE-DIMENSIONAL PHASE IMAGE GENERATION UNIT — 66

REFRACTIVE INDEX DISTRIBUTION CALCULATION UNIT — 67

DISPLAY UNIT — 68

STORAGE UNIT — 69

RECORDING MEDIUM — 2

EP 4 542 203 A1

*Fig.15*

ANALYSIS UNIT

INTERFERENCE INTENSITY IMAGE ACQUISITION UNIT

FIRST COMPLEX AMPLITUDE IMAGE GENERATION UNIT

SECOND COMPLEX AMPLITUDE IMAGE GENERATION UNIT

PHASE CONJUGATE OPERATION UNIT

TWO-DIMENSIONAL PHASE IMAGE GENERATION UNIT

THREE-DIMENSIONAL PHASE IMAGE GENERATION UNIT

REFRACTIVE INDEX DISTRIBUTION CALCULATION UNIT

DISPLAY UNIT

STORAGE UNIT

RECORDING MEDIUM

## Fig.16

INTERFERENCE INTENSITY IMAGE ACQUISITION — S61

NTERFERENCE INTENSITY IMAGE FOR
EACH LIGHT IRRADIATION DIRECTION

FIRST COMPLEX AMPLITUDE IMAGE GENERATION — S62

COMPLEX AMPLITUDE IMAGE FOR
EACH LIGHT IRRADIATION DIRECTION

SECOND COMPLEX AMPLITUDE IMAGE GENERATION — S63

COMPLEX AMPLITUDE IMAGE FOR
EACH POSITION AND EACH
LIGHT IRRADIATION DIRECTION

PHASE CONJUGATE OPERATION — S64

COMPLEX AMPLITUDE IMAGE FOR
EACH POSITION AND EACH
LIGHT IRRADIATION DIRECTION

TWO-DIMENSIONAL PHASE IMAGE GENERATION — S65

TWO-DIMENSIONAL PHASE IMAGE
FOR EACH POSITION

THREE-DIMENSIONAL PHASE IMAGE GENERATION — S66

THREE -DIMENSIONAL PHASE IMAGE

REFRACTIVE INDEX DISTRIBUTION CALCULATION — S67

THREE -DIMENSIONAL REFRACTIVE
INDEX DISTRIBUTION

50

**Fig.17**

SECOND COMPLEX AMPLITUDE
IMAGE GENERATION STEP S63

TWO-DIMENSIONAL PHASE
IMAGE GENERATION STEP S65

$z = z_1$

$z = z_2$

$z = z_3$

COMPLEX
AMPLITUDE IMAGE

$z = z_0$

COMPLEX
AMPLITUDE IMAGE

COMPLEX DIFFERENTIAL
INTERFERENCE IMAGE

# Fig.18

SECOND COMPLEX AMPLITUDE
IMAGE GENERATION STEP S63

PHASE
CONJUGATE
OPERATION
STEP S64

TWO-DIMENSIONAL PHASE
IMAGE GENERATION STEP S65

$z = z_0$

COMPLEX
AMPLITUDE IMAGE

$z = z_1$

$z = z_2$

$z = z_3$

COMPLEX
AMPLITUDE IMAGE

$z = z_1$

$z = z_2$

$z = z_3$

COMPLEX
AMPLITUDE IMAGE

COMPLEX DIFFERENTIAL
INTERFERENCE IMAGE

*Fig.19*

**Fig.20**

EP 4 542 203 A1

## Fig.21

THREE-DIMENSIONAL
PHASE IMAGE
GENERATION STEP S66

PHASE IMAGE GENERATED FROM
COMPLEX AMPLITUDE IMAGE AFTER
PHASE CONJUGATE OPERATION
IN TWO-DIMENSIONAL PHASE
IMAGE GENERATION STEP S65

REFRACTIVE INDEX
DISTRIBUTION
CALCULATION
STEP S67

THREE -DIMENSIONAL
PHASE IMAGE

THREE -DIMENSIONAL
REFRACTIVE INDEX
DISTRIBUTION

PHASE IMAGE GENERATED FROM
COMPLEX AMPLITUDE IMAGE BEFORE
PHASE CONJUGATE OPERATION
IN TWO-DIMENSIONAL PHASE
IMAGE GENERATION STEP S65

# Fig.22

LIGHT
IRRADIATION $U_{in}(k_{in})$

OBSERVATION
OBJECT $T(r_{out}, k_{in})$

$u_{out}(r_{out})$

IMAGING

IMAGING UNIT

**Fig.23**

IMAGING UNIT

IMAGING

$u_{in} (r_{in})$

OBSERVATION OBJECT

$S (r_{in}, k_{out})$

LIGHT IRRADIATION

$U_{out} (k_{out})$

Fig.24

**Fig.25**

EP 4 542 203 A1

*Fig.26*

ANALYSIS UNIT 70

INTERFERENCE INTENSITY IMAGE ACQUISITION UNIT 71

FIRST COMPLEX AMPLITUDE IMAGE GENERATION UNIT 72

SECOND COMPLEX AMPLITUDE IMAGE GENERATION UNIT 73

TWO-DIMENSIONAL PHASE IMAGE GENERATION UNIT 74

THREE-DIMENSIONAL PHASE IMAGE GENERATION UNIT 75

REFRACTIVE INDEX DISTRIBUTION CALCULATION UNIT 76

THIRD COMPLEX AMPLITUDE IMAGE GENERATION UNIT 77

DISPLAY UNIT 78

STORAGE UNIT 79

RECORDING MEDIUM 2

1H

# Fig.27

## Fig.28

INTERFERENCE INTENSITY IMAGE ACQUISITION — S71

INTERFERENCE INTENSITY IMAGE FOR
EACH LIGHT IRRADIATION DIRECTION

FIRST COMPLEX AMPLITUDE IMAGE GENERATION — S72

COMPLEX AMPLITUDE IMAGE FOR
EACH LIGHT IRRADIATION DIRECTION

j = 0 — S81

j ← j + 1 — S82

— S83

SECOND COMPLEX AMPLITUDE IMAGE GENERATION — S73

TWO-DIMENSIONAL PHASE IMAGE GENERATION — S74

THREE-DIMENSIONAL PHASE IMAGE GENERATION — S75

REFRACTIVE INDEX DISTRIBUTION CALCULATION — S76

THREE -DIMENSIONAL REFRACTIVE
INDEX DISTRIBUTION

S84

j < J — false — END

true

THIRD COMPLEX AMPLITUDE IMAGE GENERATION — S77

COMPLEX AMPLITUDE IMAGE FOR
EACH LIGHT IRRADIATION DIRECTION

## Fig.29

```
┌─────────────────────────────────┐
│ COMPLEX AMPLITUDE IMAGE FOR     │
│ EACH LIGHT IRRADIATION DIRECTION│
└─────────────────────────────────┘
                 │
┌────────────────────────────────────────────────┐ ~S83
│  ┌──────────────────────────────────────────┐  │
│  │ SECOND COMPLEX AMPLITUDE IMAGE GENERATION │  │ S73
│  └──────────────────────────────────────────┘  │
│            ┌──────────────────────────┐         │
│            │ COMPLEX AMPLITUDE IMAGE FOR│       │
│            │ EACH POSITION AND EACH    │        │
│            │ LIGHT IRRADIATION DIRECTION│       │
│            └──────────────────────────┘         │
│  ┌──────────────────────────────────────────┐  │
│  │ TWO-DIMENSIONAL PHASE IMAGE GENERATION    │  │ S74
│  └──────────────────────────────────────────┘  │
│            ┌──────────────────────────┐         │
│            │ TWO-DIMENSIONAL PHASE     │        │
│            │ IMAGE FOR EACH POSITION   │        │
│            └──────────────────────────┘         │
│  ┌──────────────────────────────────────────┐  │
│  │ THREE-DIMENSIONAL PHASE IMAGE GENERATION  │  │ S75
│  └──────────────────────────────────────────┘  │
│            ┌──────────────────────────┐         │
│            │ THREE-DIMENSIONAL PHASE IMAGE│     │
│            └──────────────────────────┘         │
│  ┌──────────────────────────────────────────┐  │
│  │ REFRACTIVE INDEX DISTRIBUTION CALCULATION │  │ S76
│  └──────────────────────────────────────────┘  │
└────────────────────────────────────────────────┘
                 │
┌─────────────────────────────────┐
│ THREE -DIMENSIONAL              │
│ REFRACTIVE INDEX DISTRIBUTION   │
└─────────────────────────────────┘
```

Fig.30

## Fig.31

COMPLEX AMPLITUDE IMAGE IN THIRD BLOCK

THIRD COMPLEX AMPLITUDE IMAGE GENERATION STEP S77

COMPLEX AMPLITUDE IMAGE IN SECOND BLOCK

THIRD COMPLEX AMPLITUDE IMAGE GENERATION STEP S77

COMPLEX AMPLITUDE IMAGE IN FIRST BLOCK

STEP S83

$z = z_3$
$z = z_2$

REFRACTIVE INDEX DISTRIBUTION OF THIRD BLOCK

STEP S83

$z = z_2$
$z = z_1$

REFRACTIVE INDEX DISTRIBUTION OF SECOND BLOCK

STEP S83

$z = z_1$
$z = z_0$

z
y
x

REFRACTIVE INDEX DISTRIBUTION OF FIRST BLOCK

$z = z_3$
$z = z_2$
$z = z_1$
$z = z_0$

z
y
x

REFRACTIVE INDEX DISTRIBUTION OF OBSERVATION OBJECT

# Fig.32

$z = z_{j-1} + 7\Delta z = z_j$

$z = z_{j-1} + 6\Delta z$

$z = z_{j-1} + 5\Delta z$

$z = z_{j-1} + 4\Delta z$

$z = z_{j-1} + 3\Delta z$

$z = z_{j-1} + 2\Delta z$

$z = z_{j-1} + \Delta z$

$z = z_{j-1}$

j-TH BLOCK

## Fig.33

```
┌─────────────────────────────────────┐
│   COMPLEX AMPLITUDE IMAGE            │
│   AT FIRST POSITION z_{j-1}          │
└─────────────────────────────────────┘
          z ← z_{j-1}                      ~S41

   INTERACTION BETWEEN THE COMPLEX
   AMPLITUDE AT POSITION z AND REFRACTIVE   ~S42
   INDEX DISTRIBUTION AT POSITION z

   PROPAGATION OF DISTANCE Δz              ~S43

   COMPLEX AMPLITUDE IMAGE
   AT POSITION z+Δz

          z ← z+Δz                         ~S44

                                            S45
   true ──◇   z < z_j   ◇
               false
   COMPLEX AMPLITUDE IMAGE
   AT SECOND POSITION z_j
```

# Fig.34

22

18

14

23

13

12

24

11

S

70

ANALYSIS
UNIT

25

44

43

42

EP 4 542 203 A1

# Fig.35

CLUSTER A
OF HEPATOCYTES

BILE CANALICULUS

CROSS SECTION

CLUSTER B
OF HEPATOCYTES

CLUSTER C
OF HEPATOCYTES

## Fig.36

CLUSTER OF HEPATOCYTES — CROSS SECTION

FIRST DAY OF CULTIVATION

THIRD DAY

SEVENTH DAY — BILE CANALICULUS

Fig.37

CONTROL

CROSS SECTION

BILE CANALICULUS

ADD DRUG A

CROSS SECTION

ADD DRUG B

Fig.38

CONTROL

ADD DRUG A

ADD DRUG B

ADD DRUG C

EP 4 542 203 A1

# Fig.39

FIRST DAY OF
ADDITION OF DRUG

FOURTH DAY

EIGHTH DAY

Fig.40

CLUSTER OF HEPATOCYTES

CROSS SECTION

BILE CANALICULUS

CLUSTER OF HEPATOCYTES
ADD TAUROCHOLIC ACID

CROSS SECTION

BILE CANALICULUS NETWORK

Fig.41

EP 4 542 203 A1

Fig.42

*Fig.43*

FLUORESCENCE IMAGE (CLF)

PHASE CONTRAST MICROSCOPE IMAGE

Fig.44

Z=6.4μm

BILE CANALICULUS 1

Z=6.4μm    Z=7.2μm    Z=8.8μm

BILE CANALICULUS 2

Z=3.2μm    Z=4.0μm    Z=6.4μm

# Fig.45

(A) ANALYSIS OF LUMEN

(B) ANALYSIS OF MICROVILLUS

(C) COMPARATIVE ANALYSIS OF REFRACTIVE INDEX

Fig.46

Fig.47

Fig.48

CLUSTER 2 OF HEPATOCYTES
BILE CANALICULUS 1
(Z=42.4μm)

CLUSTER 2 OF HEPATOCYTES
BILE CANALICULUS 2
(Z=42.4μm)

ANALYSIS OF LUMEN

CLUSTER 2 OF HEPATOCYTES

Z=42.4μm

## INTERNATIONAL SEARCH REPORT

| International application No. |
|---|
| **PCT/JP2023/007107** |

### A. CLASSIFICATION OF SUBJECT MATTER

*G01N 21/41*(2006.01)i; *C12M 1/00*(2006.01)i; *C12M 3/00*(2006.01)i; *C12Q 1/02*(2006.01)i; *G01N 21/17*(2006.01)i; *G01N 33/48*(2006.01)i; *G01N 33/483*(2006.01)i
FI:   G01N21/41 Z; C12M1/00 Z; C12M3/00 Z; C12Q1/02; G01N21/17 610; G01N33/48 Z; G01N33/483 C

According to International Patent Classification (IPC) or to both national classification and IPC

### B. FIELDS SEARCHED

Minimum documentation searched (classification system followed by classification symbols)

G01N21/00-21/01; G01N21/17-21/74; G01N33/48-33/98; C12M1/00-3/10; C12Q1/00-3/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

Optica; ACS PUBLICATIONS; SPIE Digital Library; Oxford Journals; APS Journals

### C. DOCUMENTS CONSIDERED TO BE RELEVANT

| Category* | Citation of document, with indication, where appropriate, of the relevant passages | Relevant to claim No. |
|---|---|---|
| A | KR 10-1855366 B1 (TOMOCUBE, INC.) 09 May 2018 (2018-05-09)<br>paragraphs [0038]-[0099], fig. 1-6 | 1-15 |
| A | WO 2022/054305 A1 (HAMAMATSU PHOTONICS K.K.) 17 March 2022 (2022-03-17)<br>paragraphs [0002]-[0097], fig. 1-32 | 1-15 |
| A | 石丸伊知郎. 生きたままの細胞内3次元成分分布時系列計測 単一細胞分光トモグラフィ. BIO INDUSTRY. vol. 25, no. 2, 2008, pp. 55-66, non-official translation (ISHIMARU, Ichirou. 3D-Time Series Compositional Distribution Measurement of Single Living Cells: Spectroscopy-Tomography of Single Cells.)<br>in particular, "2. 3D Refraction Factor Distribution Measurement of Unlabeled Free-Floating Single Cells" | 1-15 |
| A | WO 2011/024592 A1 (THE UNIVERSITY OF TOKYO) 03 March 2011 (2011-03-03)<br>paragraphs [0001]-[0065], fig. 1-21 | 1-15 |
| A | JP 2020-28305 A (NAT AGRICULTURE & FOOD RES ORGANIZATION) 27 February 2020 (2020-02-27)<br>paragraphs [0001]-[0085], fig. 1-11B | 1-15 |

☐ Further documents are listed in the continuation of Box C.      ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **23 March 2023** | **04 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/007107**

| Patent document cited in search report | | | Publication date (day/month/year) | Patent family member(s) | | | Publication date (day/month/year) |
|---|---|---|---|---|---|---|---|
| KR | 10-1855366 | B1 | 09 May 2018 | (Family: none) | | | |
| WO | 2022/054305 | A1 | 17 March 2022 | (Family: none) | | | |
| WO | 2011/024592 | A1 | 03 March 2011 | US | 2012/0183989 | A1 | |
| | | | | paragraphs [0001]-[0194], fig. 1-21 | | | |
| | | | | EP | 2471908 | A1 | |
| JP | 2020-28305 | A | 27 February 2020 | US | 2018/0120298 | A1 | |
| | | | | paragraphs [0001]-[0172], fig. 1-11B | | | |
| | | | | WO | 2016/158417 | A1 | |
| | | | | EP | 3275994 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011024592 A
- JP 2020028305 A

- JP 6644786 B

**Non-patent literature cited in the description**

- **WATKINS**. Drug safety sciences and the bottleneck in drug development.. *Clin. Pharmacol. Ther.*, 2011, vol. 89, 788-790
- **GISSEN et al.** Structural and functional hepatocyte polarity and liver disease. *Journal of Hepatology*, 2015, vol. 63

- **SUNGSAM KANG et al.** Imaging deep within a scattering medium using collective accumulation of single-scattered waves. *NATURE PHOTONICS*, 2015, vol. 9, 253-258